# EUROPEAN PATENT APPLICATION

(11) **EP 1 437 351 A1**
(43) Date of publication of application: **14.07.2004**
(21) Application number: 02775363.1
(22) Date of filing: 17.10.2002
(51) Int. Cl.: C07D 401/06, C07D 401/14, C07D 405/14, A61K 31/4709, A61K 31/4725, A61K 31/496, A61K 31/5377, A61K 31/541, A61K 31/551, A61P 3/10, A61P 43/00

(54) **AMINE DERIVATIVE**

(30) Priority: 19.10.2001 JP 2001322897
(71) Applicant: Takeda Chemical Industries, Ltd., Osaka-shi, Osaka 541-0045 (JP)
(72) Inventor: ABE, Hidenori, Ikeda-shi, Osaka 563-0029 (JP); KASAI, Shizuo, Kawanishi-shi, Hyogo 666-0115 (JP); TAKEKAWA, Shiro, Nishinomiya-shi, Hyogo 662-0976 (JP); WATANABE, Masanori, Suita-shi, Osaka 565-0862 (JP)
(74) Representative: Rickard, Timothy Mark Adrian
(86) International application number: PCT/JP2002/010800
(87) International publication number: WO 2003/042204

(57) **Abstract**

The present invention provide a compound of the formula: 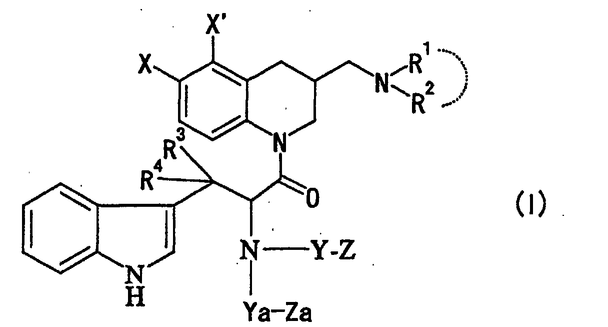 wherein X and X' are the same or different and each represents a hydrogen atom, etc.; R¹ and R² are the same or different and each represents a hydrogen atom, etc.; R³ represents a hydrocarbon group optionally having substituents, etc.; R⁴ represents a hydrogen atom, etc.; Y and Ya are the same or different and each represents a bond or a spacer having a main chain of 1 to 8 atoms; Z and Za are the same or different and each represents a hydrogen atom, etc.; or a salt thereof or a prodrug thereof, having a somatostatin receptor binding inhibitory activity and is useful for preventing and/or treating diseases associated with somatostatin.

## Description

### Technical Field

The present invention relates to novel amine derivatives. In further detail, the present invention relates to a compound having a somatostatin receptor binding inhibitory activity, which is useful for preventing or treating diseases associated with somatostatin.

### Background Art

In 1973, somatostatin was found to be a growth hormone-inhibiting factor (somatotropin release inhibiting factor; SRIF).

Heretofore, somatostatin receptors have been found to include five subtypes, which were respectively named SSTR1, SSTR2, SSTR3, SSTR4 and SSTR5 (e.g., Endocrinology, vol. 136, pp. 3695-3697, 1995; Trends in Pharmacological Sciences, pp. 87-94, Vol. 18, 1997; Life Science, Vol. 57, pp. 1249-1265, 1995).

Somatostatin is known to inhibit production and/or secretion of various hormones, growth factors and physiologically active substances in living organisms. The hormones inhibited by somatostatin include growth hormone (GH), thyroid-stimulating hormones (TSH), prolactin, insulin, glucagon and the like. Therefore, somatostatin has various functions in endocrine systems, exocrine systems and nerve systems, and the development of drugs targeting somatostatin are ongoing (e.g., Endocrinology, vol. 136, pp. 3695-3697, 1995; Trends in Pharmacological Sciences, pp. 87-94, vol. 18, 1997).

The diseases caused by somatostatin include life-style related diseases such as diabetes; central nervous system diseases, immune system diseases, hormone-dependent tumors and the like. Attempts have been made to develop somatostatin itself or somatostatin analogues as pharmaceutical products. For instance, octreotide known as a somatostatin receptor agonist has been marketed as a drug for treating hormone-dependent tumors.

As somatostatin receptor antagonists or agonists, the following compounds are known.

### 1) A compound represented by the formula:

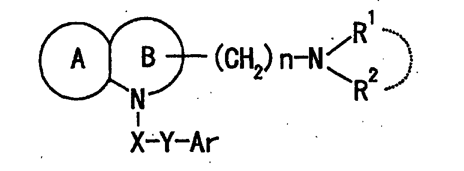

wherein Ar represents an aromatic group optionally having substituents; X represents a methylene, S, SO, SO₂ or CO; Y represents a spacer having a main chain of 2 to 5 atoms; n represent an integer of 1 to 5;
i) R¹ and R² each represent a hydrogen atom or a lower alkyl optionally having substituents,
ii) R¹ and R², together with the adjacent nitrogen atom, form a nitrogen-containing heterocyclic ring optionally having substituents, or
iii) R¹ or R² is linked with a constituting atom on ring B together with -(CH₂)ₙ-N= to form a spiro ring optionally having substituents;
ring A represents an aromatic ring optionally having substituents; ring B represents a 4- to 7-membered nitrogen-containing non-aromatic ring which may be further substituted by alkyl or acyl, provided that, when the ring A has a group represented by the formula -NHCOR¹¹ (R¹¹ represents alkyl group, alkoxy-alkyl group, alkylthio-alkyl group, cycloalkyl group, cycloalkyl-alkyl group, aryl group, aryl-alkyl group or a group represented by the formula -NHR¹² (R¹² represents alkyl group, cycloalkyl group, cycloalkylalkyl group, aryl group or arylalkyl group)) as a substituent, X represents S, SO, SO₂ or CO; or a salt thereof (WO99/52875).

### 2) A compound of the formula:

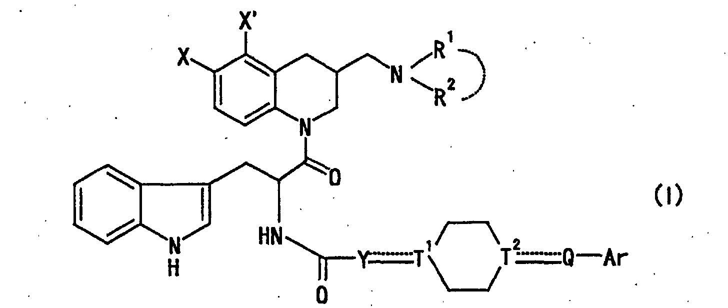

wherein X and X' are the same or different and each represents a hydrogen atom, a fluorine atom, a chlorine atom or an amino optionally having substituents, and at least one of X and X' represents a fluorine atom, a chlorine atom or an amino optionally having substituents;
R¹ and R² represent a hydrogen atom or C₁₋₆ alkyl optionally having substituents, or R¹ and R², together with the adjacent nitrogen atom, form a nitrogen-containing heterocyclic ring optionally having substituents;
Y and Q are the same or different, and each represents a bond or a spacer having a main chain of 1 to 6 atoms;
... represents a single bond or a double bond;
T¹ and T² are the same or different, and each represents C(R⁹) (R⁹ represents a hydrogen atom, a hydroxy or C₁₋₆ alkyl) or N when each of the adjacent ... is a single bond, and C when the adjacent ... is a double bond; and
Ar represents an aromatic group optionally having substituents, a C₃₋₉ cycloalkyl group optionally having substituents, a 3 to 9-membered saturated heterocyclic group optionally having substituents, a hydrogen atom or a halogen atom (WO01/25228).

### Disclosure of the Invention

There is a demand for the development of a compound having an excellent somatostatin receptor binding inhibitory activity and the like, as well as superior properties as a pharmaceutical product, such as oral absorbability, pharmacokinetics and the like.

The present inventors have studied various aspects of a compound having somatostatin receptor binding inhibitory activity and first synthesized a compound represented by the formula: 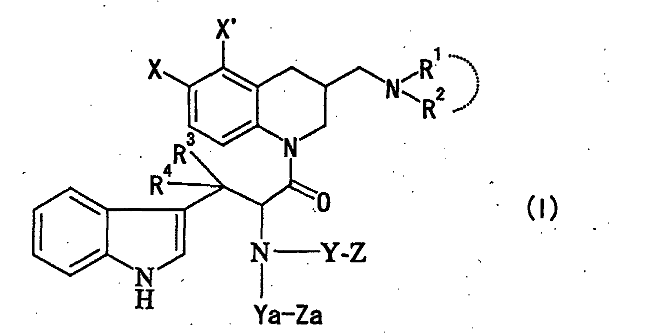 wherein
- X and X': are the same or different and each represents a hydrogen atom, a halogen atom or an amino optionally having substituents;
- R¹ and R²: are the same or different and each represents a hydrogen atom or a C₁₋₆ alkyl optionally having substituents, or R¹ and R² form, together with the adjacent nitrogen atom, a nitrogen-containing heterocyclic ring optionally having substituents;
- R³: represents a hydrocarbon group optionally having substituents or a heterocyclic group optionally having substituents;
- R⁴: represents a hydrogen atom, a hydrocarbon group optionally having substituents or a heterocyclic group optionally having substituents; or
- R³ and R⁴: may form, together with the adjacent carbon atom, a ring optionally having substituents;
- Y and Ya: are the same or different and each represents a bond or a spacer having a main chain of 1 to 8 atoms; and
- Z and Za: are the same or different, and each represents a hydrogen atom, a halogen atom or a cyclic group optionally having substituents;
or a salt thereof [hereinafter sometimes to be abbreviated as compound (I)], which is structurally characterized by having specific substituents R³ and R⁴ at the 3-position of the group: 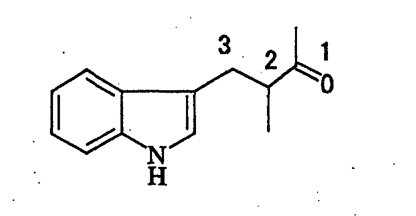 first found that this compound (I) unexpectedly has a superior somatostatin receptor binding inhibitory activity, has superior property as a pharmaceutical product as shown in low toxicity and the like, and is fully satisfactory as a pharmaceutical agent, and completed the present invention based on these findings.

Accordingly, the present invention relates to:
1) a compound (I) or a prodrug thereof;
2) the compound (I) wherein X is a halogen atom, and X' is a hydrogen atom;
3) the compound (I) wherein R¹ and R² are the same or different and each is a C₁₋₆ alkyl;
4) the compound (I) wherein R³ is a C₁₋₆ alkyl;
5) the compound (I) wherein R⁴ is a hydrogen atom;
6) the compound (I) wherein the spacers having a main chain of 1 to 8 atoms represented by Y and Ya are each a divalent group comprising 1 to 5 groups selected from -O-, -S-, -CO-, -SO-, - SO₂-, -NR⁵- (R⁵ is a hydrogen atom, a C₁₋₆ alkyl optionally having substituents, a C₁₋₆ alkyl-carbonyl optionally having substituents or a C₁₋₆ alkylsulfonyl optionally having substituents) and a divalent C₁₋₆ non-cyclic hydrocarbon group optionally having substituents;
7) the compound (I) wherein Y is -CO-;
8) the compound (I) wherein Y is -CH₂-;
9) the compound (I) wherein Z is a cyclic group optionally having substituents;
10) the compound (I) according to the above 9) wherein the cyclic group optionally having substituents is a 4- to 10-membered monocyclic non-aromatic heterocyclic group which may have 1 to 3 substituents selected from an oxo, an optionally halogenated C₁₋₆ alkyl, a C₆₋₁₄ aryloxy optionally having substituents, a C₇₋₁₉ aralkyl optionally having substituents, a carbamoyl, an optionally halogenated C₁₋₆ alkyl-carbonyl, an optionally halogenated C₁₋₆ alkylsulfonyl, a C₆₋₁₄ aryl-carbonyl optionally having substituents, a C₆₋₁₄ aryl-sulfonyl optionally having substituents, a C₇₋₁₉ aralkyloxy-carbonyl optionally having substituents, a heterocyclic carbonyl optionally having substituents, a C₆₋₁₄ aryl optionally having substituents, an aromatic heterocyclic group optionally having substituents, a 5- to 7-membered non-aromatic heterocyclic group optionally having substituents and a C₇₋₁₉ aralkyloxy optionally having substituents;
11) the compound (I) wherein Ya is a bond and Za is a hydrogen atom;
12) the compound (I) which is
   N-[(1R,2S)-1-[((3R)-6-chloro-3-[(dimethylamino)methyl]-3,4-dihydro-1(2H)-quinolinyl)carbonyl]-2-(1H-indol-3-yl)propyl]-1-[(1-methyl-1H-indol-2-yl)carbonyl]-4-piperidinecarboxamide,
   N-[(1R,2S)-1-[((3R)-6-chloro-3-[(dimethylamino)methyl]-3,4-dihydro-1(2H)-quinolinyl)carbonyl]-2-(1H-indol-3-yl)propyl]-4-phenoxy-1-piperidinecarboxamide,
   (-)-N-[1-[((3R)-6-chloro-3-[(dimethylamino)methyl]-3,4-dihydro-1(2H)-quinolinyl)carbonyl]-2-(1H-indol-3-yl)propyl]-4-(4-hydroxyphenoxy)-1-piperidinecarboxamide,
   N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-4-(4-fluorophenoxy)-1-piperidinecarboxamide,
   4-benzoyl-N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-1-piperazinecarboxamide, or a salt thereof;
13) a pharmaceutical composition comprising the compound (I) or a prodrug thereof;
14) the composition according to the above 13), which is a somatostatin receptor binding inhibitor;
15) the composition according to the above 14), which is a somatostatin subtype 2 receptor binding inhibitor;
16) the composition according to the above 13), which is a somatostatin receptor agonist;
17) the composition according to the above 16), which is a somatostatin subtype 2 receptor agonist;
18) the composition according to the above 13), which is an agent for preventing or treating diabetes or diabetic complications;
19) use of the compound (I) or a prodrug thereof for manufacturing a somatostatin receptor binding inhibitor;
20) a method for inhibiting binding of a somatostatin receptor in a mammal, which comprises administering an effective amount of the compound (I) or a prodrug thereof to the mammal;
21) use of the compound (I) or a prodrug thereof for manufacturing an agent for preventing or treating diabetes or diabetic complications;
22) a method for preventing or treating diabetes or diabetic complications in a mammal, which comprises administering an effective amount of the compound (I) or a prodrug thereof to the mammal; and the like.

In the formula (I), X and X' are the same or different and each represents a hydrogen atom, a halogen atom or an amino optionally having substituents.

As used herein, the "halogen atom" includes fluorine, chlorine, bromine, iodine and the like. Of these, fluorine and chlorine are preferable, and chlorine is particularly preferable.

The substituent of the "amino optionally having substituents" includes an optionally halogenated C₁₋₆ alkyl, formyl, an optionally halogenated C₁₋₆ alkyl-carbonyl, an C₁₋₆ alkoxy-carbonyl, an optionally halogenated C₁₋₆ alkylsulfonyl and the like.

The "optionally halogenated C₁₋₆ alkyl" includes, for example, a C₁₋₆ alkyl (e.g., methyl, ethyl, propyl, isopropyl, butyl, isobutyl, sec-butyl, tert-butyl, pentyl, hexyl, etc.) which may have 1 to 5, preferably 1 to 3 halogen atoms (e.g., fluorine, chlorine, bromine, iodine, etc.). Concrete examples are methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl and the like.

The "optionally halogenated C₁₋₆ alkyl-carbonyl" includes, for example, a C₁₋₆ alkyl-carbonyl (e.g., acetyl, propanoyl, butanoyl, pentanoyl, hexanoyl, etc.) which may have 1 to 5, preferably 1 to 3 halogen atoms (e.g., fluorine, chlorine, bromine, iodine, etc.) and the like. Concrete examples are acetyl, monochloroacetyl, trifluoroacetyl, trichloroacetyl, propanoyl, butanoyl, pentanoyl, hexanoyl and the like.

The "C₁₋₆ alkoxy-carbonyl" includes, for example, methoxycarbonyl, ethoxycarbonyl, propoxycarbonyl, tert-butoxycarbonyl and the like.

The "optionally halogenated C₁₋₆ alkylsulfonyl" includes, for example, a C₁₋₆ alkylsulfonyl (e.g., methylsulfonyl, ethylsulfonyl, propylsulfonyl, isopropylsulfonyl, butylsulfonyl, sec-butylsulfonyl, tert-butylsulfonyl, etc.) which may have 1 to 5, preferably 1 to 3, halogen atoms (e.g., fluorine, chlorine, bromine, iodine, etc.) and the like. Concrete examples are methylsulfonyl, difluoromethylsulfonyl, trifluoromethylsulfonyl, ethylsulfonyl, propylsulfonyl, isopropylsulfonyl, butylsulfonyl, 4,4,4-trifluorobutylsulfonyl, pentylsulfonyl, hexylsulfonyl and the like.

In the formula (I), X and X' are preferably the same or different and each is a hydrogen atom or a halogen atom. Moreover, it is preferable that X be a halogen atom and X' be a hydrogen atom, and it is particularly preferable that X be a chlorine atom and X' be a hydrogen atom.

In the formula (I), the "C₁₋₆ alkyl" of the "C₁₋₆ alkyl optionally having substituents" includes, for example, methyl, ethyl, propyl, isopropyl, butyl, isobutyl, sec-butyl, tert-butyl, pentyl, hexyl and the like. Of these, preferred are methyl, ethyl, propyl and the like, and especially preferred is methyl.

The "substituent" of the above "C₁₋₆ alkyl optionally having substituents" includes, for example, halogen atoms (e.g., fluorine, chlorine, bromine, iodine, etc.), a C₁₋₃ alkylenedioxy (e.g., methylenedioxy, ethylenedioxy, etc.), nitro, cyano, an optionally halogenated C₃₋₆ cycloalkyl, an optionally halogenated C₁₋₆ alkoxy, an optionally halogenated C₁₋₆ alkylthio, hydroxy, amino, a mono-C₁₋₆ alkylamino (e.g., methylamino, ethylamino, propylamino, isopropylamino, butylamino, etc.), a di-C₁₋₆ alkylamino (e.g., dimethylamino, diethylamino, dipropylamino, dibutylamino, ethylmethylamino, etc.), formyl, carboxy, carbamoyl, thiocarbamoyl, an optionally halogenated C₁₋₆ alkyl-carbonyl, a C₁₋₆ alkoxy-carbonyl, a mono-C₁₋₆ alkyl-carbamoyl (e.g., methylcarbamoyl, ethylcarbamoyl, etc.), a di-C₁₋₆ alkyl-carbamoyl (e.g., dimethylcarbamoyl, diethylcarbamoyl, ethylmethylcarbamoyl, etc.), an optionally halogenated C₁₋₆ alkylsulfonyl, formylamino, an optionally halogenated C₁₋₆ alkyl-carboxamide, a C₁₋₆ alkoxy-carboxamide (e.g., methoxycarboxamide, ethoxycarboxamide, propoxycarboxamide, butoxycarboxamide, etc.), a C₁₋₆ alkylsulfonylamino (e.g., methylsulfonylamino, ethylsulfonylamino, etc.), a C₁₋₆ alkyl-carbonyloxy (e.g., acetoxy, propanoyloxy, etc.), a C₁₋₆ alkoxy-carbonyloxy (e.g., methoxycarbonyloxy, ethoxycarbonyloxy, propoxycarbonyloxy, butoxycarbonyloxy, etc.), a mono-C₁₋₆ alkyl-carbamoyloxy (e.g., methylcarbamoyloxy, ethylcarbamoyloxy, etc.), a di-C₁₋₆ alkyl-carbamoyloxy (e.g., dimethylcarbamoyloxy, diethylcarbamoyloxy, etc.), an aromatic group optionally having substituents, a 5-to 10-membered non-aromatic heterocyclic group optionally having substituents and the like. The number of the substituents is, for example, 1 to 3. When the number of the substituents is 2 or more, these substituents may be the same or different.

The above-mentioned "optionally halogenated C₃₋₆ cycloalkyl" includes, for example, a C₃₋₆ cycloalkyl (e.g., cyclopropyl, cyclobutyl, cyclopentyl, cyclohexyl, etc.) which may have 1 to 5, preferably 1 to 3, halogen atoms (e.g., fluorine, chlorine, bromine, iodine, etc.) and the like. Concrete examples are cyclopropyl, cyclobutyl, cyclopentyl, cyclohexyl, 4,4-dichlorocyclohexyl, 2,2,3,3-tetrafluorocyclopentyl, 4-chlorocyclohexyl and the like.

The above-mentioned "optionally halogenated C₁₋₆ alkoxy" includes, for example, a C₁₋₆ alkoxy (e.g., methoxy, ethoxy, propoxy, butoxy, pentyloxy, etc.) which may have 1 to 5, preferably 1 to 3, halogen atoms (e.g., fluorine, chlorine, bromine, iodine, etc.) and the like. Concrete examples are methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy and the like.

The above-mentioned "optionally halogenated C₁₋₆ alkylthio" includes, for example, a C₁₋₆ alkylthio (e.g., methylthio, ethylthio, propylthio, isopropylthio, butylthio, sec-butylthio, tert-butylthio, etc.) which may have 1 to 5, preferably 1 to 3, halogen atoms (e.g., fluorine, chlorine, bromine, iodine. etc.) and the like. Concrete examples are methylthio, difluoromethylthio, trifluoromethylthio, ethylthio, propylthio, isopropylthio, butylthio, 4,4,4-trifluorobutylthio, pentylthio, hexylthio and the like.

As the above-mentioned "optionally halogenated C₁₋₆ alkyl-carbonyl", "C₁₋₆ alkoxy-carbonyl" and "optionally halogenated C₁₋₆ alkylsulfonyl", those respectively exemplified respectively for the "substituent" in the "amino optionally having substituents" represented by X can be mentioned.

The above-mentioned "optionally halogenated C₁₋₆ alkyl-carboxamide" includes, for example, a C₁₋₆ alkyl-carboxamide (e.g., acetamide, propanamide, butanamide, etc.) which may have 1 to 5, preferably 1 to 3, halogen atoms (e.g., fluorine, chlorine, bromine, iodine, etc.) and the like. Concrete examples are acetamide, trifluoroacetamide, propanamide, butanamide and the like.

The "aromatic group" of the "aromatic group optionally having substituents" includes, for example, a monocyclic aromatic group, a fused aromatic group, an aromatic ring assembly group and the like.

The monocyclic aromatic group includes, for example, phenyl and a 5- or 6-membered aromatic heterocyclic group.

The "5- or 6-membered aromatic heterocyclic group" includes, for example, a 5- or 6-membered aromatic heterocyclic group containing, in addition to carbon atoms, 1 to 4, preferably 1 to 3, heteroatoms selected from nitrogen, sulfur and oxygen atoms and the like. Concrete examples are thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, 1,2,4-oxadiazolyl, 1,3,4-oxadiazolyl, 1,2,4-thiadiazolyl, 1,3,4-thiadiazolyl, furazanyl, tetrazolyl and the like.

The "monocyclic aromatic group" is preferably phenyl; 2-or 3-thienyl; 2- or 3-furyl; 2-, 3- or 4-pyridyl; 2-, 4- or 5-thiazolyl; 2-, 4- or 5-oxazolyl; 3- or 4-pyrazolyl; 2-pyrazinyl; 2-, 4- or 5-pyrimidinyl; 1-, 2- or 3-pyrrolyl; 1-, 2- or 4-imidazolyl; 3- or 4-pyridazinyl; 3-isothiazolyl; 3-isoxazolyl; 1,2,4-oxadiazol-5-yl; 1,2,4-oxadiazol-3-yl; and the like.

The "fused aromatic group" includes, for example, a fused polycyclic aromatic hydrocarbon group, a fused polycyclic aromatic heterocyclic group and the like.

Said "fused polycyclic aromatic hydrocarbon group" includes, for example, a C₉₋₁₄ fused polycyclic (bi- or tri-cyclic) aromatic hydrocarbon group (e.g., naphthyl, indenyl, fluorenyl, anthracenyl, etc.) and the like.

Said "fused polycyclic aromatic heterocyclic group" includes, for example, a 9- to 14-membered, preferably 9- or 10-membered, fused polycyclic (preferably bi- to tetra-cyclic, more preferably bi- or tri-cyclic) aromatic heterocyclic group containing, in addition to carbon atoms, 1 to 4 heteroatoms selected from nitrogen, sulfur and oxygen atoms and the like. The "fused polycyclic aromatic heterocyclic group" includes, for example, benzofuranyl, benzothiophenyl, benzimidazolyl, benzoxazolyl, benzothiazolyl, benzisothiazolyl, naphtho[2,3-b]thienyl, isoquinolinyl, quinolinyl, indolyl, quinoxalinyl, phenanthridinyl, phenothiazinyl, phenoxazinyl, phthalazinyl, naphthyridinyl, quinazolinyl, cinnolinyl, carbazolyl, β-carbolinyl, acridinyl, phenadinyl, phthalimido and the like.

The "fused aromatic group" is preferably 1-naphthyl; 2-naphthyl; 2-, 3-, 4-, 5- or 8-quinolyl; 1-, 3-, 4-, 5-, 6-, 7-or 8-isoquinolyl; 1-, 2-, 3-, 4-, 5-, 6- or 7-indolyl; 1-, 2-, 4- or 5-isoindolyl; 1-, 5- or 6-phthalazinyl; 2-, 3- or 5-quinoxalinyl; 2-, 3-, 4-, 5- or 6-benzothienyl; 2-, 3-, 4-, 5-or 6-benzofuranyl; 2-, 4-, 5- or 6-benzothiazolyl; 1-, 2-, 4-, 5- or 6-benzimidazolyl and the like.

The "aromatic ring assembly group" includes, for example, a group derived by removing an optional hydrogen atom from aromatic ring assemblies in which two or more, preferably two or three, aromatic rings are directly connected with each other by single bond(s) and the number of such direct ring junctions is one less than the number of the rings involved.

The above-mentioned aromatic ring assemblies include, for example, aromatic ring assemblies formed by two or three (preferably two) groups selected from a C₆₋₁₄ monocyclic or fused polycyclic aromatic hydrocarbon (e.g., benzene ring, naphthalene ring, etc.) and a 5- to 10-membered (preferably 5-or 6-membered) aromatic heterocyclic ring.

Preferred examples of the aromatic ring assemblies include one composed of two or three aromatic rings selected from benzene, naphthalene, pyridine, pyrimidine, thiophene, furan, thiazole, isothiazole, oxazole, 1,2,4-oxadiazole, 1,3,4-oxadiazole, 1,2,4-thiadiazole, 1,3,4-thiadiazole, quinoline, isoquinoline, indole, benzothiophene, benzoxazole, benzothiazole and benzofuran.

Concrete examples of the "aromatic ring assembly group" are 2-, 3- or 4-biphenylyl; 3-(1-naphthyl)-1,2,4-axadiazol-5-yl; 3-(2-naphthyl)-1,2,4-oxadiazol-5-yl; 3-(2-benzofuranyl)-1,2,4-oxadiazol-5-yl; 3-phenyl-1,2,4-oxadiazol-5-yl; 3-(2-benzoxazolyl)-1,2,4-oxadiazol-5-yl; 3-(3-indolyl)-1,2,4-oxadiazol-5-yl; 3-(2-indolyl)-1,2,4-oxadiazol-5-yl; 4-phenylthiazol-2-yl; 4-(2-benzofuranyl)thiazol-2-yl; 4-phenyl-1,3-oxazol-5-yl; 5-phenyl-isothiazol-4-yl; 5-phenyloxazol-2-yl; 4-(2-thienyl)phenyl; 4-(3-thienyl)phenyl; 3-(3-pyridyl)phenyl; 4-(3-pyridyl)phenyl; 6-phenyl-3-pyridyl; 5-phenyl-1,3,4-oxadiazol-2-yl; 4-(2-naphthyl)phenyl; 4-(2-benzofuranyl)phenyl; 4,4'-terphenyl; and the like.

The "5- to 10-membered non-aromatic heterocyclic group" of the "5- to 10-membered non-aromatic heterocyclic group optionally having substituents" includes, for example, a 5- to 10-membered non-aromatic heterocyclic group containing, in addition to carbon atoms, 1 to 4 heteroatoms selected from nitrogen, sulfur and oxygen atoms and the like. Preferable examples of the non-aromatic heterocyclic group include 1-, 2-or 3-pyrrolidinyl; 1-, 2-, 4- or 5-imidazolidinyl; 2- or 4-imidazolinyl; 2-, 3- or 4-pyrazolidinyl; piperidinyl; 2-, 3-or 4-piperidinyl; 1- or 2-piperazinyl; morpholino; thiomorpholino and the like.

The "substituent" of the "aromatic group optionally having substituents" and "5- to 10-membered non-aromatic heterocyclic group optionally having substituents" includes, for example, oxo, halogen atoms (e.g., fluorine, chlorine, bromine, iodine, etc.), a C₁₋₃ alkylenedioxy (e.g., methylenedioxy, ethylenedioxy, etc.), nitro, cyano, an optionally halogenated C₁₋₆ alkyl, a C₆₋₁₄ aryloxy-C₁₋₆ alkyl (e.g., phenoxymethyl, etc.), a C₁₋₆ alkyl-C₆₋₁₄ aryl-C₂₋₆ alkenyl (e.g., methylphenylethenyl, etc.), an optionally halogenated C₃₋₆ cycloalkyl, a C₇₋₁₉ aralkyl optionally having substituents, an optionally halogenated C₁₋₆ alkoxy, an optionally halogenated C₁₋₆ alkylthio, hydroxy, a C₆₋₁₄ aryloxy optionally having substituents, a C₇₋₁₉ aralkyloxy optionally having substituents, amino, a mono-C₁₋₆ alkylamino (e.g., methylamino, ethylamino, propylamino, isopropylamino, butylamino, etc.), a di-C₁₋₆ alkylamino (e.g., dimethylamino, diethylamino, dipropylamino, dibutylamino, ethylmethylamino, etc.), a 5- to 7-membered non-aromatic heterocyclic group optionally having substituents, an acyl, an acylamino, an acyloxy and the like.

The "aromatic group" and "5- to 10-membered non-aromatic heterocyclic group" may have 1 to 5, preferably 1 to 3, of the above-mentioned substituents at substitutable positions. When the number of the substituents is two or more, those substituents may be the same or different.

The "optionally halogenated C₁₋₆ alkyl" is exemplified by those mentioned as the "substituent" of the "amino optionally having substituents" represented by X.

The "optionally halogenated C₃₋₆ cycloalkyl", "optionally halogenated C₁₋₆ alkoxy" and "optionally halogenated C₁₋₆ alkylthio" are exemplified by those mentioned as the "substituent" of the "C₁₋₆ alkyl optionally having substituents" represented by R¹.

The "C₇₋₁₉ aralkyl" of the "C₇₋₁₉ aralkyl optionally having substituents" includes, for example, benzyl, phenethyl, diphenylmethyl, triphenylmethyl, 1-naphthylmethyl, 2-naphthylmethyl, 2,2-diphenylethyl, 3-phenylpropyl, 4-phenylbutyl, 5-phenylpentyl and the like. Of these, benzyl, and the like are preferable.

The "C₆₋₁₄ aryloxy" of the "C₆₋₁₄ aryloxy optionally having substituents" mentioned above includes, for example, phenyloxy, 1-naphthyloxy, 2-naphthyloxy and the like.

The "C₇₋₁₉ aralkyloxy" of the "C₇₋₁₉ aralkyloxy optionally having substituents" mentioned above includes, for example, benzyloxy, phenethyloxy, diphenylmethyloxy, triphenylmethyloxy, 1-naphthylmethyloxy, 2-naphthylmethyloxy, 2,2-diphenylethyloxy, 3-phenylpropyloxy, 4-phenylbutyloxy, 5-phenylpentyloxy, etc.

The "5- to 7-membered non-aromatic heterocyclic group" of the "5- to 7-membered non-aromatic heterocyclic group optionally having substituents" includes, for example, a 5- to 7-membered non-aromatic heterocyclic ring containing, in addition to carbon atoms, 1 to 4 heteroatoms selected from nitrogen, sulfur and oxygen atoms. Preferable examples of the non-aromatic heterocyclic ring include 1-, 2- or 3-pyrrolidinyl; 1-, 2- 4- or 5-imidazolidinyl; 2- or 4-imidazolinyl; 2-, 3- or 4-pyrazolidinyl; piperidinyl; 2-, 3-or 4-piperidinyl; 1- or 2-piperazinyl; morpholino; thiomorpholino and the like.

The "substituent" of the "C₇₋₁₉ aralkyl optionally having substituents", "C₆₋₁₄ aryloxy optionally having substituents", "C₇₋₁₉ aralkyloxy optionally having substituents" and "5- to 7-membered non-aromatic heterocyclic group optionally having substituents" includes, for example, halogen atoms (e.g., fluorine, chlorine, bromine, iodine, etc.), a C₁₋₃ alkylenedioxy (e.g., methylenedioxy, ethylenedioxy, etc.), nitro, cyano, an optionally halogenated C₁₋₆ alkyl, an optionally halogenated C₃₋₆ cycloalkyl, an optionally halogenated C₁₋₆ alkoxy, an optionally halogenated C₁₋₆ alkylthio, hydroxy, amino, a mono-C₁₋₆ alkylamino (e.g., methylamino, ethylamino, propylamino, isopropylamino, butylamino, etc.), a di-C₁₋₆ alkylamino (e.g., dimethylamino, diethylamino, dipropylamino, dibutylamino, ethylmethylamino, etc.), formyl, carboxy, carbamoyl, thiocarbamoyl, an optionally halogenated C₁₋₆ alkyl-carbonyl, a C₁₋₆ alkoxy-carbonyl, a mono-C₁₋₆ alkyl-carbamoyl (e.g., methylcarbamoyl, ethylcarbamoyl, etc.), a di-C₁₋₆ alkyl-carbamoyl (e.g., dimethylcarbamoyl, diethylcarbamoyl, ethylmethylcarbamoyl, etc.), an optionally halogenated C₁₋₆ alkylsulfonyl, sulfamoyl, formylamino, an optionally halogenated C₁₋₆ alkyl-carboxamide, a C₁₋₆ alkoxy-carboxamide (e.g., methoxycarboxamide, ethoxycarboxamide, propoxycarboxamide, butoxycarboxamide, etc.), a C₁₋₆ alkylsulfonylamino (e.g., methylsulfonylamino, ethylsulfonylamino, etc.), a C₁₋₆ alkyl-carbonyloxy (e.g., acetoxy, propanoyloxy, etc.), a C₁₋₆ alkoxy-carbonyloxy (e.g., methoxycarbonyloxy, ethoxycarbonyloxy, propoxycarbonyloxy, butoxycarbonyloxy, etc.), a mono-C₁₋₆ alkyl-carbamoyloxy (e.g., methylcarbamoyloxy, ethylcarbamoyloxy, etc.), a di-C₁₋₆ alkyl-carbamoyloxy (e.g., dimethylcarbamoyloxy, diethylcarbamoyloxy, etc.), a 5- or 6-membered aromatic heterocyclic group (e.g., tetrazolyl) and the like. The number of the substituents is, for example, 1 to 3. When the number of the substituents is 2 or more, these substituents may be the same or different.

Here, the "optionally halogenated C₁₋₆ alkyl", "optionally halogenated C₁₋₆ alkyl-carbonyl", "C₁₋₆ alkoxy-carbonyl" and "optionally halogenated C₁₋₆ alkylsulfonyl" are exemplified by those mentioned as the "substituent" of the "amino optionally having substituents" represented by X.

As the "optionally halogenated C₃₋₆ cycloalkyl", "optionally halogenated C₁₋₆ alkoxy", "optionally halogenated C₁₋₆ alkylthio" and "optionally halogenated C₁₋₆ alkyl-carboxamide", those respectively exemplified as the "substituent" of the "C₁₋₆ alkyl optionally having substituents" represented by R¹ are used.

The aforementioned "acyl" includes, for example, an acyl represented by the formula: -CO-R⁶, -CO-OR⁶, -CO-NR⁶R⁷, -CS-NR⁶R⁷, -SO₂-R^{6a}, -SO-R^{6a} or -SO₂-NR⁶R⁷, wherein R⁶ represents (i) a hydrogen atom, (ii) a hydrocarbon group optionally having substituents or (iii) a heterocyclic group optionally having substituents; R^{6a} represents (i) a hydrocarbon group optionally having substituents or (ii) a heterocyclic group optionally having substituents; R⁷ represents a hydrogen atom or a C₁₋₆ alkyl; R⁶ and R⁷, together with the adjacent nitrogen atom, may form a nitrogen-containing heterocyclic ring optionally having substituents; and the like.

The "hydrocarbon group" of the "hydrocarbon group optionally having substituents" represented by R⁶ or R^{6a} includes, for example, an alkyl, an alkenyl, an alkynyl, a cycloalkyl, an aryl, an aralkyl and the like. Of these, the following hydrocarbon group having 1 to 19 carbon atoms, etc. are preferable:
a) a C₁₋₆ alkyl (e.g., methyl, ethyl, propyl, isopropyl, butyl, isobutyl, sec-butyl, tert-butyl, pentyl, hexyl, etc.);
b) a C₂₋₆ alkenyl (e.g., vinyl, allyl, isopropenyl, 2-butenyl, etc.);
c) a C₂₋₆ alkynyl (e.g., ethynyl, propargyl, 2-butynyl, etc.);
d) a C₃₋₈ cycloalkyl which may be condensed with benzene ring (e.g., cyclopropyl, cyclobutyl, cyclopentyl, cyclohexyl, dihydroindenyl, etc.);
e) a C₆₋₁₄ aryl (e.g., phenyl, 1-naphthyl, 2-naphthyl, 2-indenyl, 2-anthryl, etc.), preferably phenyl;
f) a C₇₋₁₉ aralkyl (e.g., benzyl, phenethyl, diphenylmethyl, triphenylmethyl, 1-naphthylmethyl, 2-naphthylmethyl, 2,2-diphenylethyl, 3-phenylpropyl, 4-phenylbutyl, 5-phenylpentyl, etc.), preferably benzyl.

The "substituent" of the "hydrocarbon group optionally having substituents" includes, for example, halogen atoms (e.g., fluorine, chlorine, bromine, iodine, etc.), a C₁₋₃ alkylenedioxy (e.g., methylenedioxy, ethylenedioxy, etc.), nitro, cyano, an optionally halogenated C₁₋₆ alkoxy, an optionally halogenated C₁₋₆ alkylthio, hydroxy, amino, a mono-C₁₋₆ alkylamino (e.g., methylamino, ethylamino, propylamino, isopropylamino, butylamino, etc.), a di-C₁₋₆ alkylamino (e.g., dimethylamino, diethylamino, dipropylamino, dibutylamino, ethylmethylamino, etc.), a 5- to 7-membered non-aromatic heterocyclic group optionally having substituents, formyl, carboxy, carbamoyl, thiocarbamoyl, an optionally halogenated C₁₋₆ alkyl-carbonyl, a C₁₋₆ alkoxy-carbonyl, a C₆₋₁₄ aryl-carbonyl (e.g., benzoyl, 1-naphthoyl, 2-naphthoyl, etc.), a heterocyclic carbonyl optionally having substituents, a C₆₋₁₄ aryloxy-carbonyl (e.g., phenyloxycarbonyl, 1-naphthyloxycarbonyl, 2-naphthyloxycarbonyl), a C₇₋₁₉ aralkyloxy-carbonyl (e.g., benzyloxycarbonyl, phenethyloxycarbonyl, diphenylmethyloxycarbonyl, triphenylmethyloxycarbonyl, 1-naphthylmethyloxycarbonyl, 2-naphthylmethyloxycarbonyl, 2,2-diphenylethyloxycarbonyl, 3-phenylpropyloxycarbonyl, 4-phenylbutyloxycarbonyl, 5-phenylpentyloxycarbonyl, etc.), a mono-C₁₋₆ alkyl-carbamoyl (e.g., methylcarbamoyl, ethylcarbamoyl, etc.), a di-C₁₋₆ alkyl-carbamoyl (e.g., dimethylcarbamoyl, diethylcarbamoyl, ethylmethylcarbamoyl, etc.), a C₆₋₁₄ aryl-carbamoyl (e.g., phenylcarbamoyl, etc.), a heterocyclic carbamoyl optionally having substituents, an optionally halogenated C₁₋₆ alkylsulfonyl, a C₆₋₁₄ arylsulfonyl (e.g., phenylsulfonyl, 1-.naphthylsulfonyl, 2-naphthylsulfonyl, etc.), formylamino, an optionally halogenated C₁₋₆ alkyl-carboxamide, a C₆₋₁₄ aryl-carboxamide (e.g., phenylcarboxamide, naphthylcarboxamide, etc.), a C₁₋₆ alkoxy-carboxamide (e.g., methoxycarboxamide, ethoxycarboxamide, propoxycarboxamide, butoxycarboxamide, etc.), a C₁₋₆ alkylsulfonylamino (e.g., methylsulfonylamino, ethylsulfonylamino, etc.), a C₁₋₆ alkyl-carbonyloxy (e.g., acetoxy, propanoyloxy, etc.), a C₆₋₁₄ aryl-carbonyloxy (e.g., benzoyloxy, 1-naphthoyloxy, 2-naphthoyloxy, etc.), a C₁₋₆ alkoxy-carbonyloxy (e.g., methoxycarbonyloxy, ethoxycarbonyloxy, propoxycarbonyloxy, butoxycarbonyloxy), a mono-C₁₋₆ alkyl-carbamoyloxy (e.g., methylcarbamoyloxy, ethylcarbamoyloxy, etc.), a di-C₁₋₆ alkyl-carbamoyloxy (e.g., dimethylcarbamoyloxy, diethylcarbamoyloxy, etc.), a C₆₋₁₄ aryl-carbamoyloxy (e.g., phenylcarbamoyloxy, naphthylcarbamoyloxy, etc.), a 5- or 6-membered heterocyclic carbonyloxy (e.g., nicotinoyloxy, etc.), a C₆₋₁₄ aryloxy (e.g., phenoxy, naphthoxy, etc.) and the like. The number of the substituents is, for example, 1 to 5, preferably 1 to 3. When the number of the substituents is 2 or more, these substituents may be the same or different.

As used herein, as the "optionally halogenated C₁₋₆ alkoxy", "optionally halogenated C₁₋₆ alkylthio" and "optionally halogenated C₁₋₆ alkyl-carboxamide", those respectively exemplified as the "substituent" of the "C₁₋₆ alkyl optionally having substituents" represented by R¹ are used.

As the "optionally halogenated C₁₋₆ alkyl-carbonyl", "C₁₋₆ alkoxy-carbonyl" and "optionally halogenated C₁₋₆ alkylsulfonyl", those exemplified as the "substituent" of the "amino optionally having substituents" represented by X are used.

As the "5- to 7-membered non-aromatic heterocyclic group optionally having substituents", those exemplified as the "substituent" of the aforementioned "aromatic group optionally having substituents" are used.

The "heterocyclic carbonyl" of the "heterocyclic carbonyl optionally having substituents" includes, for example, nicotinoyl, isonicotinoyl, 2-thenoyl, 3-thenoyl, 2-furoyl, 3-furoyl, morpholinocarbonyl, piperidinocarbonyl, pyrrolidin-1-ylcarbonyl, indolylcarbonyl and the like.

The "heterocyclic carbamoyl" of the "heterocyclic carbamoyl optionally having substituents" includes, for example, morpholinocarbamonyl, piperidinocarbamoyl, 2-pyridylcarbamoyl, 3-pyridylcarbamoyl, 4-pyridylcarbamoyl, 2-thienylcarbamoyl, 3-thienylcarbamoyl, indolylcarbamoyl and the like.

As the "substituent" of the "heterocyclic carbonyl optionally having substituents" and "heterocyclic carbamoyl optionally having substituents", those exemplified as the "substituent" of the aforementioned "C₇₋₁₉ aralkyl optionally having substituents" are used. The number of the substituents is, for example, 1 to 3. When the number of the substituents is 2 or more, these substituents may be the same or different.

The "heterocyclic group" of the "heterocyclic group optionally having substituents" represented by R⁶ or R^{6a} includes, for example, a 4- to 14-membered mono-, di- or tri-cyclic heterocyclic group containing, in addition to carbon atoms, 1 to 4 heteroatoms selected from nitrogen, sulfur and oxygen atoms, preferably (i) an aromatic heterocyclic group, (ii) a non-aromatic heterocyclic group, or (iii) a 7- to 10-membered bridged heterocyclic group and the like.

As used herein, the "aromatic heterocyclic group" includes, for example, a 4- to 14-membered (preferably 4- to 10-membered) aromatic heterocyclic group containing, in addition to carbon atoms, 1 to 4 heteroatoms selected from nitrogen, sulfur and oxygen atoms and the like. Preferable examples of the "aromatic heterocyclic group" include thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, 1,2,4-oxadiazolyl, 1,3,4-oxadiazolyl, 1,2,4-thiadiazolyl, 1,3,4-thiadiazolyl, furazanyl, benzothiophenyl, benzofuranyl, benzimidazolyl, benzoxazolyl, benzothiazolyl, benzisothiazole, naphtho[2,3-b]thiophenyl, phenoxathiinyl, indolyl, isoindolyl, 1H-indazolyl, purinyl, 4H-quinolidinyl, isoquinolinyl, quinolinyl, phthalazinyl, naphthyridinyl, quinoxalinyl, quinazolinyl, cinnolinyl, carbazolyl, β-carbolinyl, phenanthridinyl, acridinyl, phenazinphenothiazinyl, phenoxazinyl, phthalimide and the like.

The "non-aromatic heterocyclic group" includes, for example, a 4- to 14-membered (preferably 4- to 10-membered) non-aromatic heterocyclic group containing, in addition to carbon atoms, 1 to 4 heteroatoms selected from nitrogen, sulfur and oxygen atoms and the like. Preferable examples of the "non-aromatic heterocyclic group" include a monocyclic non-aromatic heterocyclic group such as azetidinyl, tetrahydrothiophenyl, tetrahydrofuranyl, pyrrolinyl, pyrrolidinyl, imidazolinyl, imidazolidinyl, oxazolinyl, oxazolidinyl, pyrazolinyl, pyrazolidinyl, thiazolinyl, thiazolidinyl, tetrahydrothiazolyl, tetrahydroisothiazolyl, tetrahydrooxazolyl, tetrahydroisoxazolyl, piperidinyl, piperazinyl, tetrahydropyridinyl, dihydropyridinyl, tetrahydropyrimidinyl, tetrahydropyridazinyl, hexamethyleneiminyl, morpholinyl, thiomorpholinyl, diazepanyl, etc.;
a fused polycyclic (preferably bi or tri-cyclic) non-aromatic heterocyclic group such as dihydrobenzofuranyl, dihydrobenzimidazolyl, dihydrobenzoxazolyl, dihydrobenzothiazolyl, dihydrobenzisothiazolyl, dihydronaphtho[2,3-b]thiophenyl, tetrahydroisoquinolinyl, tetrahydroquinolinyl, indolinyl, isoindolinyl, tetrahydroquinoxalinyl, tetrahydrophenanthridinyl, hexahydrophenothiazinyl, hexahydrophenoxazinyl, tetrahydrophthalazinyl, tetrahydronaphthyridinyl, tetrahydroquinazolinyl, tetrahydrocinnolinyl, tetrahydrocarbazolyl, tetrahydro-β-carbolinyl, tetrahydroacridinyl, tetrahydrophenazinyl, tetrahydrothioxanthenyl, octahydroisoquinolinyl, and the like.

Preferable examples of the "7- to 10-membered bridged heterocyclic group" include, for example, quinuclidyl, 7-azabicyclo[2.2.1]heptanyl and the like.

The "heterocyclic group" is preferably a 4- to 10-membered monocyclic or bicyclic heterocyclic group containing, in addition to carbon atoms, 1 to 4 heteroatoms selected from nitrogen, sulfur and oxygen atoms.

The "substituent" of the "heterocyclic group optionally having substituents" is exemplified by those mentioned as the "substituent" of the "C₇₋₁₉ aralkyl optionally having substituents". The number of the substituents is, for example, 1 to 3. When the number of the substituents is 2 or more, these substituents may be the same or different.

The "C₁₋₆ alkyl" represented by R⁷ includes, for example, methyl, ethyl, propyl, isopropyl, butyl, isobutyl, sec-butyl, tert-butyl, pentyl, hexyl and the like.

The "nitrogen-containing heterocyclic ring" of the "nitrogen-containing heterocyclic ring optionally having substituents" formed by R⁶ and R⁷ together with the adjacent nitrogen atom includes, for example, a 3- to 8-membered nitrogen-containing heterocyclic ring containing, in addition to carbon atoms, at least one nitrogen atom and optionally further containing 1 to 3 heteroatoms selected from nitrogen, sulfur and oxygen atoms. Concrete examples are aziridine, azetidine, morpholine, thiomorpholine, piperidine, piperazine, pyrrolidine, hexamethyleneimine, heptamethyleneimine, hexahydropyrimidine, 1,4-diazepane; unsaturated cyclic amines thereof (e.g., 1,2,5,6-tetrahydropyridine, etc.), and the like. Of these, preferred are morpholine, piperidine, piperazine, pyrrolidine and the like.

The "substituent" of the "nitrogen-containing heterocyclic ring optionally having substituents" includes, for example, oxo, an optionally halogenated C₁₋₆ alkyl, an optionally halogenated C₁₋₆ alkyl-carbonyl, an optionally halogenated C₁₋₆ alkylsulfonyl, a C₆₋₁₄ aryl optionally having substituents, a C₇₋₁₉ aralkyl optionally having substituents, a C₆₋₁₄ aryl-carbonyl optionally having substituents, a 5- to 10-membered aromatic heterocyclic group optionally having substituents, etc. can be mentioned. The number of the substituents is, for example, 1 to 3. When the number of the substituents is 2 or more, these substituents may be the same or different.

As the aforementioned "optionally halogenated C₁₋₆ alkyl", "optionally halogenated C₁₋₆ alkyl-carbonyl" and "optionally halogenated C₁₋₆ alkylsulfonyl", those respectively exemplified as the "substituent" of the "amino optionally having substituents" represented by X can be mentioned.

The "C₆₋₁₄ aryl" of the "C₆₋₁₄ aryl optionally having substituents" includes, for example, phenyl, 1-naphthyl, 2-naphthyl, 2-indenyl, 2-anthryl and the like. Of these, phenyl and the like are preferable.

As the "C₇₋₁₉ aralkyl optionally having substituents", those exemplified as the "substituent" of the aforementioned "aromatic group optionally having substituents" can be mentioned.

The "C₆₋₁₄ aryl-carbonyl" of the "C₆₋₁₄ aryl-carbonyl optionally having substituents" includes, for example, benzoyl, 1-naphthoyl, 2-naphthoyl and the like.

The "5- to 10-membered aromatic heterocyclic group" of the "5- to 10-membered aromatic heterocyclic group optionally having substituents" includes, for example, a 5- to 10-membered monocyclic or bi-cyclic aromatic heterocyclic group containing, in addition to carbon atoms, 1 or 2 kinds of, preferably 1 to 4, heteroatoms selected from nitrogen, sulfur and oxygen atoms. Concrete examples thereof include 2- or 3-thienyl; 2-, 3- or 4-pyridyl; 2- or 3-furyl; 2-, 4- or 5-thiazolyl; 2-, 4- or 5-oxazolyl; 1-, 3- or 4-pyrazolyl; 2-pyrazinyl; 2-, 4- or 5-pyrimidinyl; 1-, 2- or 3-pyrrolyl; 1-, 2- or 4-imidazolyl; 3- or 4-pyridazinyl; 3-isothiazolyl; 3-isoxazolyl; 1,2,4-oxadiazol-5-yl; 1,2,4-oxadiazol-3-yl; 2-, 3-, 4-, 5- or 8-quinolyl; 1-, 3-, 4-, 5-, 6-, 7- or 8-isoquinolyl; 1-, 2-, 3-, 4-, 5-, 6- or 7-indolyl; 1-, 2-, 4- or 5-isoindblyl; 1-, 5- or 6-phthalazinyl; 2-, 3- or 5-quinoxalinyl; 2-, 3-, 4-, 5- or 6-benzofuranyl; 2-, 4-, 5- or 6-benzothiazolyl; 1-, 2-, 4-, 5- or 6-benzimidazolyl and the like.

For the "substituent" of the aforementioned "C₆₋₁₄ aryl optionally having substituents", "C₆₋₁₄ aryl-carbonyl optionally having substituents" and "5- to 10-membered aromatic heterocyclic group optionally having substituents", those exemplified as the "substituent" of the aforementioned "C₇₋₁₉ aralkyl optionally having substituents" can be mentioned. The number of the substituents is, for example, 1 to 3. When the number of the substituents is 2 or more, these substituents may be the same or different.

The "acyl" is preferably formyl, carboxy, carbamoyl, an optionally halogenated C₁₋₆ alkyl-carbonyl (e.g., acetyl, etc.), a C₁₋₆ alkoxy-carbonyl (e.g., methoxycarbonyl, ethoxycarbonyl, propoxycarbonyl, tert-butoxycarbonyl, etc.), a C₆₋₁₄ aryl-carbonyl (e.g., benzoyl, 1-naphthoyl, 2-naphthoyl, etc.) optionally having substituents, a C₆₋₁₄ aryloxy-carbonyl (e.g., phenoxycarbonyl, etc.) optionally having substituents, a C₇₋₁₉ aralkyloxy-carbonyl (e.g., benzyloxycarbonyl, phenethyloxycarbonyl, etc.) optionally having substituents, a 5- or 6-membered heterocyclic carbonyl (e.g., nicotinoyl, isonicotinoyl, 2-thenoyl, 3-thenoyl, 2-furoyl, 3-furoyl, morpholinocarbonyl, piperidinocarbonyl, pyrrolidin-1-ylcarbonyl, etc.) optionally having substituents, a mono-C₁₋₆ alkyl-carbamoyl (e.g., methylcarbamoyl, ethylcarbamoyl, etc.), a di-C₁₋₆ alkyl-carbamoyl (e.g., dimethylcarbamoyl, diethylcarbamoyl, ethylmethylcarbamoyl, etc.), a C₆₋₁₄ aryl-carbamoyl (e.g., phenylcarbamoyl, 1-naphthylcarbamoyl, 2-naphthylcarbamoyl, etc.) optionally having substituents, a 5-to 6-membered heterocyclic carbamoyl (e.g., 2-pyridylcarbamoyl, 3-pyridylcarbamoyl, 4-pyridylcarbamoyl, 2-thienylcarbamoyl, 3-thienylcarbamoyl, etc.) optionally having substituents, an optionally halogenated C₁₋₆ alkylsulfonyl (e.g., methylsulfonyl, etc.), a C₆₋₁₄ arylsulfonyl optionally having substituents, sulfamoyl and the like. Of these, preferred are an optionally halogenated C₁₋₆ alkyl-carbonyl, a C₁₋₆ alkoxy-carbonyl, a C₆₋₁₄ aryl-carbonyl optionally having substituents, a C₆₋₁₄ arylsulfonyl (e.g., benzenesulfonyl, 1-naphthalenesulfonyl, 2-naphthalenesulfonyl, etc.) optionally having substituents and the like.

For the "substituent" of the "C₆₋₁₄ aryl-carbonyl optionally having substituents", "C₆₋₁₄ aryloxy-carbonyl optionally having substituents", "C₇₋₁₉ aralkyloxy-carbonyl optionally having substituents", "5- to 6-membered heterocyclic carbonyl optionally having substituents", "C₆₋₁₄ aryl-carbamoyl optionally having substituents", "5- to 6-membered heterocyclic carbamoyl optionally having substituents" and "C₆₋₁₄ arylsulfonyl optionally having substituents", those exemplified as the "substituent" of the aforementioned "C₇₋₁₉ aralkyl optionally having substituents" can be used. The number of the substituents is, for example, 1 to 3. When the number of the substituents is 2 or more, these substituents may be the same or different.

The aforementioned "acylamino" includes, for example, an amino which is mono- or di-substituted by the aforementioned "acyl" and preferably an acylamino represented by the formula: - NR⁸-COR⁹, -NR⁸-COOR^{9a}, -NR⁸-SO₂R^{9a} or -NR⁸-CONR⁹R^{9b}, wherein R⁸ represents a hydrogen atom or C₁₋₆ alkyl; R⁹ has the same meanings as the above R⁶; R^{9a} has the same meanings as the above R^{6a}; R^{9b} has the same meanings as the above R⁷; and the like.

The "C₁₋₆ alkyl" represented by R⁸ is exemplified by those mentioned as the aforementioned "C₁₋₆ alkyl" represented by R⁷.

The "acylamino" is preferably formylamino, an optionally halogenated C₁₋₆ alkyl-carboxamide (e.g., acetylamino), a C₆₋₁₄ aryl-carboxamide (e.g., phenylcarboxamide, naphthylcarboxamide, etc.) optionally having substituents, an optionally halogenated C₁₋₆ alkoxy-carboxamide (e.g., methoxycarboxamide, ethoxycarboxamide, propoxycarboxamide, butoxycarboxamide, etc.), an optionally halogenated C₁₋₆ alkylsulfonylamino (e.g., methylsulfonylamino, ethylsulfonylamino, etc.) and the like.

As the "substituent" of the "C₆₋₁₄ aryl-carboxamide optionally having substituents", those exemplified as the "substituent" of the aforementioned "C₇₋₁₉ aralkyl optionally having substituents" can be used. The number of the substituents is, for example, 1 to 3. When the number of the substituents is 2 or more, these substituents may be the same or different.

The aforementioned "acyloxy" includes, for example, oxy substituted by one "acyl" mentioned above and preferably an acyloxy represented by the formula: -O-COR¹⁰, -O-COOR¹⁰ or -O-CONHR¹⁰, wherein R¹⁰ has the same meanings as the aforementioned R⁶; and the like.

The "acyloxy" is preferably a C₁₋₆ alkyl-carbonyloxy (e.g., acetoxy, propanoyloxy, isobutanoyloxy, pivaloyloxy, etc.), a C₆₋₁₄ aryl-carbonyloxy (e.g., benzoyloxy, 1-naphthoyloxy, 2-naphthoyloxy, etc.) optionally having substituents, an optionally halogenated C₁₋₆ alkoxy-carbonyloxy (e.g., methoxycarbonyloxy, trifluoromethoxycarbonyloxy, ethoxycarbonyloxy, propoxycarbonyloxy, butoxycarbonyloxy, etc.), a mono-C₁₋₆ alkyl-carbamoyloxy (e.g., methylcarbamoyloxy, ethylcarbamoyloxy, etc.), a di-C₁₋₆ alkyl-carbamoyloxy (e.g., dimethylcarbamoyloxy, diethylcarbamoyloxy, etc.), a C₆₋₁₄ aryl-carbamoyloxy (e.g., phenylcarbamoyloxy, naphthylcarbamoyloxy, etc.) optionally having substituents, nicotinoyloxy and the like.

As the "substituent" of the "C₆₋₁₄ aryl-carbonyloxy optionally having substituents" and "C₆₋₁₄ aryl-carbamoyloxy optionally having substituents", those exemplified as the "substituent" of the aforementioned "C₇₋₁₉ aralkyl optionally having substituents" can be used. The number of the substituents is, for example, 1 to 3. When the number of the substituents is 2 or more, these substituents may be the same or different.

The "nitrogen-containing heterocyclic ring" of the "nitrogen-containing heterocyclic ring optionally having substituents" formed by R¹ and R² together with the adjacent nitrogen atom is exemplified by the "nitrogen-containing heterocyclic ring optionally having substituents" formed by the aforementioned R⁶ and R⁷ together with the adjacent nitrogen atom.

R¹ and R² are the same or different and each is preferably C₁₋₆ alkyl, and more preferably methyl.

In the formula (I), the "hydrocarbon group optionally having substituents" and the "heterocyclic group optionally having substituents" represented by R³ and R⁴ are respectively exemplified by those mentioned for R⁶.

The "ring" of the "ring optionally having substituents" formed by R³ and R⁴ together with the adjacent carbon atom includes, for example, a C₃₋₆ cycloalkane, a 5- to 10-membered non-aromatic heterocyclic ring and the like.

The C₃₋₆ cycloalkane here includes, for example, cyclopropane, cyclobutane, cyclopentane, cyclohexane and the like.

The 5- to 10-membered non-aromatic heterocyclic ring includes, for example, a 5- to 10-membered non-aromatic heterocyclic ring containing, in addition to carbon atoms, 1 to 4 heteroatoms selected from nitrogen, sulfur and oxygen atoms and the like. Preferable examples of the non-aromatic heterocyclic ring include pyrrolidine; imidazolidine; imidazoline; pyrazolidine; piperidine; piperazine; morpholine; thiomorpholine and the like.

The "substituent" of the "ring optionally having substituents" is exemplified by those mentioned as the "substituent" of the "nitrogen-containing heterocyclic ring optionally having substituents" formed by R⁶ and R⁷ together with the adjacent nitrogen atom. The number of the substituents is, for example, 1 to 3. When the number of the substituents is 2 or more, these substituents may be the same or different.

R³ is preferably a C₁₋₆ alkyl, more preferably methyl.

R⁴ is preferably a hydrogen atom.

The "spacer having a main chain of 1 to 8 atoms" represented by Y or Ya in the formula (I) means a spacer in which 1 to 8 atoms of a main chain are combined in a straight-chain form. The "number of atoms of a main chain" is counted such that the number of atoms of the main chain is minimum.

The "spacer having a main chain of 1 to 8 atoms" includes, for example, a divalent group comprising 1 to 5 groups selected from -O-, -S-, -CO-, -SO-, -SO₂-, -NR⁵- (R⁵ is a hydrogen atom, a C₁₋₆ alkyl optionally having substituents, a C₁₋₆ alkyl-carbonyl optionally having substituents or a C₁₋₆ alkylsulfonyl optionally having substituents) and a divalent C₁₋₆ non-cyclic hydrocarbon group optionally having substituents and the like.

As used herein, as the "C₁₋₆ alkyl optionally having substituents", those exemplified for R¹ can be mentioned. The "C₁₋₆ alkyl optionally having substituents" is preferably a C₁₋₆ alkyl which may have 1 to 3 substituents selected from a halogen atom and an optionally halogenated C₁₋₆ alkoxy.

The "C₁₋₆ alkyl-carbonyl" of the "C₁₋₆ alkyl-carbonyl optionally having substituents" includes, for example, acetyl, propanoyl, butanoyl, pentanoyl, hexanoyl and the like.

The "C₁₋₆ alkylsulfonyl" of the "C₁₋₆ alkylsulfonyl optionally having substituents" includes, for example, methylsulfonyl, ethylsulfonyl, propylsulfonyl, isopropylsulfonyl, butylsulfonyl, sec-butylsulfonyl, tert-butylsulfonyl and the like.

R⁵ is preferably a hydrogen atom or a C₁₋₆ alkyl which may have 1 to 3 substituents selected from a halogen atom and an optionally halogenated C₁₋₆ alkoxy.

The "divalent C₁₋₆ non-cyclic hydrocarbon group" of the "divalent C₁₋₆ non-cyclic hydrocarbon group optionally having substituents" includes, for example,
(1) a C₁₋₆ alkylene (e.g., -CH₂-, -(CH₂)₂-, -(CH₂)₃-, - (CH₂)₄-, -(CH₂)₅-, -(CH₂)₆-, -CH (CH₃)-, -C (CH₃)₂-, -CH(CH₃)CH₂-, -C(CH₃)₂CH₂-, -CH(CH₂CH₃)CH₂-, -(CH(CH₃))₂-, - (CH₂)₂C(CH₃)₂-, -CH₂C (CH₃)₂CH₂-, -CH (CH₂CH₃)(CH₂)₂-, -(CH₂)₃C(CH₃)₂-, -(CH₂)₃CH(CH₃)CH₂-, etc.);
(2) a C₂₋₆ alkenylene (e.g., -CH=CH-, -CH₂-CH=CH-, - C(CH₃)₂-CH=CH-, -CH₂-CH=CH-CH₂-, -CH₂-CH₂-CH=CH-, - CH=CH-CH=CH-, -CH=CH-CH₂-CH₂-CH₂-, etc.);
(3) a C₂₋₆ alkynylene (e.g., -C≡C-, -CH₂-C≡C-, - CH₂-C≡C-CH₂-CH₂-, etc.), and the like.

The substituent of the "C₁₋₆ alkyl-carbonyl optionally having substituents", "C₁₋₆ alkylsulfonyl optionally having substituents" and "divalent C₁₋₆ non-cyclic hydrocarbon group optionally having substituents" includes, for example, a halogen atom, a C₁₋₃ alkylenedioxy, nitro, cyano, an optionally halogenated C₃₋₆ cycloalkyl, an optionally halogenated C₁₋₆ alkoxy, an optionally halogenated C₁₋₆ alkylthio, hydroxy, amino, a mono-C₁₋₆ alkylamino, a di-C₁₋₆ alkylamino, formyl, carboxy, carbamoyl, thiocarbamoyl, an optionally halogenated C₁₋₆ alkyl-carbonyl, a C₁₋₆ alkoxy-carbonyl, a mono-C₁₋₆ alkyl-carbamoyl, a di-C₁₋₆ alkyl-carbamoyl, an optionally halogenated C₁₋₆ alkylsulfonyl, formylamino, an optionally halogenated C₁₋₆ alkyl-carboxamide, a C₁₋₆ alkoxy-carboxamide, a C₁₋₆ alkylsulfonylamino, a C₁₋₆ alkyl-carbonyloxy, a C₁₋₆ alkoxy-carbonyloxy, a mono-C₁₋₆ alkyl-carbamoyloxy, a di-C₁₋₆ alkyl-carbamoyloxy, etc. These substituents are exemplified by those mentioned as the "substituent" of the above "C₁₋₆ alkyl optionally having substituents" represented by R¹. Of these, preferred are a halogen atom, hydroxy, cyano, an optionally halogenated C₁₋₆ alkoxy and the like. The number of the substituents is, for example, 1 to 3. When the number of the substituents is 2 or more, these substituents may be the same or different.

Preferable examples of the "spacer having a main chain of 1 to 8 atoms" include,
(1) a C₁₋₆ alkylene which may have 1 to 3 substituents selected from a halogen atom, hydroxy and cyano (e.g., -CH₂-, -CF₂-, -CCl₂-, -(CH₂)₂-, -(CH₂)₃-, -(CH₂)₄-, -(CH₂)₅-, -CH(CH₃)-, -CH(CN)-, -C(CH₃)₂-, -CH(CF₃)-, -CH(CH₃)CH₂-, -C(CH₃)₂CH₂-, -(CH(CH₃))₂-, -CH(CH₂CH₃)CH₂-, -(CF₂)₂-, -(CH₂)₂C(CH₃)₂-, -CH₂C(CH₃)₂CH₂-, -CH(CH₂CH₃)(CH₂)₂-, -(CH₂)₃C(CH₃)₂-, -(CH₂)₃CH(CH₃)CH₂-, etc.);
(2) a C₂₋₆ alkenylene which may have 1 to 3 substituents selected from a halogen atom, hydroxy and cyano (e.g., -CH=CH-, -CH₂-CH=CH-, -CH₂-CF=CH-, -C(CH₃)₂-CH=CH-, -CH₂-CH=CH-CH₂-, -CH₂-CH₂-CH=CH-, -CH=CH-CH=CH-, -CH=CH-CH₂-CH₂-CH₂- etc.);
(3) a C₂₋₆ alkynylene which may have 1 to 3 substituents selected from a halogen atom, hydroxy and cyano (e.g., -C≡C-, -CH₂-C≡C-, -CH₂-C≡C-CH₂-CH₂- etc.);
(4) a spacer having a main chain of 1 to 8 atoms represented by the following formula:
   -alka-O-alkb-, -alka-S-alkb-, -alka-CO-alkb-, -alka-SO-alkb-, -alka-SO₂-alkb- or -alka-NR⁵-alkb-;
(5) a spacer having a main chain of 2 to 8 atoms represented by the following formula:
   -alkc-CO-alkd-NR⁵-alke-, -alkc-NR⁵-alkd-CO-alke-, -alkc-SO₂-alkd-NR⁵-alke-, -alkc-NR⁵-alkd-SO₂-alke-, -alkc-CO-alkd-O-alke-, -alkc-O-alkd-CO-alke-, -alkc-CO-alkd-S-alke- or -alkc-S-alkd-CO-alke-;
(6) a spacer having a main chain of 3 to 8 atoms represented by the following formula:
   -alkf-NR⁵ CO-alkg-NR^{5a}-alkh-, -alkf-CONR⁵-alkg-NR^{5a}-alkh-, -alkf-CONR⁵-alkg-O-alkh-, -alkf-CONR⁵-alkg-S-alkh-, -alkf-NR⁵CO-alkg-O-alkh-, -alkf-NR⁵CO-alkg-S-alkh-, -alkf-SO₂N⁵-alkg-O-alkh-, -alkf-SO₂NR⁵-alkg-S-alkh-, -alkf-NR⁵SO₂-alkg-O-alkh-, -alkf-NR⁵SO₂-alkg-S-alkh-, -alkf-CO-alkg-CONR⁵-alkh- or -alkf-CO-alkg-NR⁵CO-alkh-
(R⁵ has the same meanings as above; R^{5a} has the same meanings as R⁵; alka, alkb, alkc, alkd, alke, alkf, alkg and alkh are the same or different and each represents a C₁₋₆ alkylene which may have 1 to 3 substituents selected from a halogen atom, hydroxy and cyano or a bond), and the like.

Y is preferably a bond; a C₁₋₆ alkylene which may have 1 to 3 substituents selected from a halogen atom, hydroxy and cyano; -alka-O-alkb-, -alka-S-alkb-, -alka-CO-alkb-, -alka-NR⁵-alkb -, -alkc-CO-alkd-NR⁵-alke-, -alkc-NR⁵-alkd-CO-alke -, -alkc-CO-alkd-O-alke-, -alkc-CO-alkd-S-alke-, -alkf-CONR⁵-alkg-NR^{5a}-alkh-, -alkf-CONR⁵-alkg-O-alkh-, -alkf-CO-alkg-NR⁵CO-alkh- (each symbol is as defined above); and the like. Y is more preferably a C₁₋₆ alkylene, -alka-CO-alkb- and the like. Of these, preferred is -CH₂-, -CO- and the like.

Ya is preferably a bond, a C₁₋₆ alkylene and the like. Of these, preferred is a bond and the like.

In the formula (I), Z and Za are the same or different and each represents a hydrogen atom, a halogen atom or a cyclic group optionally having substituents.

As used herein, the "halogen atom" includes fluorine, chlorine, bromine, iodine and the like.

The "cyclic group" of the "cyclic group optionally having substituents" includes, for example, an aromatic group, a non-aromatic cyclic hydrocarbon group, a non-aromatic heterocyclic group and the like.

As used herein, the "aromatic group" is exemplified by the "aromatic group" of the "aromatic group optionally having substituents" mentioned as the "substituent" of the "C₁₋₆ alkyl optionally having substituents" represented by R¹.

The "aromatic group" is preferably phenyl, a 5 or 6-membered aromatic heterocyclic group (preferably furyl, pyrrolyl, imidazolyl, pyridyl, etc.) and the like.

The "non-aromatic cyclic hydrocarbon group" includes, for example, a C₃₋₈ cycloalkyl which may be condensed with benzene ring, a C₃₋₈ cycloalkenyl which may be condensed with benzene ring, and the like.

As used herein, the C₃₋₈ cycloalkyl includes, for example, cyclopropyl, cyclobutyl, cyclopentyl, cyclohexyl, cycloheptyl, cyclooctyl and the like.

The C₃₋₈ cycloalkenyl includes, for example, cyclopropenyl, cyclobutenyl, cyclopentenyl, cyclohexenyl, cycloheptenyl, cyclooctenyl and the like.

The "non-aromatic cyclic hydrocarbon group" is preferably a C₃₋₈ cycloalkyl (preferably cyclopropyl, cyclohexyl, dihydroindenyl) which may be condensed with benzene ring and the like.

The "non-aromatic heterocyclic group" is exemplified by the "non-aromatic heterocyclic group" mentioned as the "heterocyclic group" represented by the above R⁶.

The "non-aromatic heterocyclic group" is preferably a 4-to 10-membered monocyclic non-aromatic heterocyclic group such as azetidinyl, pyrrolidinyl, piperidinyl, piperazinyl, morpholinyl, thiomorpholinyl, diazepanyl, tetrahydrofuranyl and the like; or a 4- to 10-membered bicyclic non-aromatic heterocyclic group such as tetrahydroisoquinolinyl, octahydroisoquinolinyl and the like; and the like.

The "cyclic group" is preferably a non-aromatic heterocyclic group, and more preferably a 4- to 10-membered monocyclic non-aromatic heterocyclic group. Of these, preferred are piperidinyl (preferably 1-piperidinyl), piperazinyl (preferably 1-piperazinyl) and the like.

The "substituent" of the "cyclic group optionally having substituents" includes, for example, oxo, a halogen atom, a C₁₋₃ alkylenedioxy, nitro, cyano, a hydrocarbon group optionally having substituents (provided when the "cyclic group" is an aromatic group, it is not a C₆₋₁₄ aryl), an optionally halogenated C₁₋₆ alkoxy, an optionally halogenated C₁₋₆ alkylthio, hydroxy, a C₆₋₁₄ aryloxy optionally having substituents, a C₇₋₁₉ aralkyloxy optionally having substituents, amino, a mono-C₁₋₆ alkylamino, a di-C₁₋₆ alkylamino, a 5- to 7-membered non-aromatic heterocyclic group optionally having substituents, an acyl, an acylamino, an acyloxy and the like.

As used herein, the "hydrocarbon group optionally having substituents" is exemplified by one mentioned as the above R⁶. For other substituents, those exemplified by the "substituent" of the aforementioned "aromatic group optionally having substituents" can be used.

When the "cyclic group" is a non-aromatic cyclic hydrocarbon group or a non-aromatic heterocyclic group, as the substituent thereof, an "aromatic heterocyclic group optionally having substituents", a "C₆₋₁₄ aryl optionally having substituents" and the like can be also mentioned. As the "aromatic heterocyclic group optionally having substituents", the "heterocyclic group optionally having substituents" exemplified for R⁶ wherein the heterocyclic group is an aromatic heterocyclic group can be mentioned. The "C₆₋₁₄ aryl optionally having substituents" is exemplified by those mentioned as the "substituent" of the "nitrogen-containing heterocyclic ring optionally having substituents" formed by R⁶ and R⁷.

The number of the "substituent" of the "cyclic group optionally having substituents" is, for example, 1 to 3. When the number of the substituents is 2 or more, these substituents may be the same or different.

When the "cyclic group" is 1-piperidinyl or 1-piperazinyl, it preferably has a "substituent" at the 4-position.

The "substituent" of the "cyclic group optionally having substituents" is preferably oxo, a halogen atom (e.g., fluorine, chlorine, bromine, iodine, etc.), an optionally halogenated C₁₋₆ alkyl, an optionally halogenated C₁₋₆ alkoxy, hydroxy, an optionally halogenated C₁₋₆ alkylthio, a C₆₋₁₄ aryloxy optionally having substituents, a C₇₋₁₉ aralkyl optionally having substituents, amino, a mono-C₁₋₆ alkylamino (e.g., methylamino, ethylamino, propylamino, isopropylamino, butylamino, etc.), a di-C₁₋₆ alkylamino (e.g., dimethylamino, diethylamino, dipropylamino, dibutylamino, ethylmethylamino, etc.), an acyl, an acylamino, a 5- to 7-membered non-aromatic heterocyclic ring-C₁₋₆ alkyl, a C₇₋₁₉ aralkyloxy optionally having substituents and the like.

When the "cyclic group" is a non-aromatic cyclic hydrocarbon group or non-aromatic heterocyclic group, as the substituent thereof, a C₆₋₁₄ aryl optionally having substituents, an aromatic heterocyclic group optionally having substituents, a 5- to 7-membered non-aromatic heterocyclic group optionally having substituents and the like are also preferable.

Z is preferably a cyclic group optionally having substituents, and as specific examples thereof, the following groups can be mentioned.
1) phenyl which may have 1 to 3 substituents selected from a halogen atom, an optionally halogenated C₁₋₆ alkyl, hydroxy, an optionally halogenated C₁₋₆ alkoxy, an optionally halogenated C₁₋₆ alkylthio, amino, a mono-C₁₋₆ alkylamino, a di-C₁₋₆ alkylamino, an optionally halogenated C₁₋₆ alkyl-carboxamide, carbamoyl, an optionally halogenated C₁₋₆ alkylsulfonyl and the like;
2) a 5 or 6-membered aromatic heterocyclic group (preferably furyl, pyrrolyl, imidazolyl, pyridyl, etc.) which may have 1 to 3 optionally halogenated C₁₋₆ alkyl;
3) a C₃₋₈ cycloalkyl (preferably cyclopropyl, cyclohexyl, dihydroindenyl, etc.) which may be condensed with benzene ring, which may be substituted by 1 to 3 optionally halogenated C₁₋₆ alkyl;
4) a 4- to 10-membered monocyclic non-aromatic heterocyclic group (preferably azetidinyl, pyrrolidinyl, piperidinyl, piperazinyl, morpholinyl, thiomorpholinyl, diazepanyl, tetrahydrofuranyl, etc.) or a 4- to 10-membered bicyclic non-aromatic heterocyclic group (preferably tetrahydroisoquinolinyl, octahydroisoquinolinyl, etc.), each of which may have 1 to 3 substituents selected from oxo, an optionally halogenated C₁₋₆ alkyl, a C₆₋₁₄ aryloxy optionally having substituents [preferably hydroxy, a halogen atom, cyano, an optionally halogenated C₁₋₆ alkyl, an optionally halogenated C₁₋₆ alkoxy, an optionally halogenated C₁₋₆ alkylthio, an optionally halogenated C₁₋₆ alkylsulfonyl, sulfamoyl, a 5 or 6-membered aromatic heterocyclic group (preferably tetrazolyl, etc.), etc.], a C₇₋₁₉ aralkyl optionally having substituents, carbamoyl, an optionally halogenated C₁₋₆ alkyl-carbonyl, an optionally halogenated C₁₋₆ alkylsulfonyl, a C₆₋₁₄ aryl-carbonyl optionally having substituents (preferably a halogen atom, etc.), a C₆₋₁₄ aryl-sulfonyl optionally having substituents (preferably an optionally halogenated C₁₋₆ alkyl, etc.), a C₇₋₁₉ aralkyloxy-carbonyl optionally having substituents, a heterocyclic carbonyl (preferably, indolylcarbonyl, etc.) optionally having substituents (preferably an optionally halogenated C₁₋₆ alkyl, etc.), a 5- to 7-membered non-aromatic heterocyclic ring-C₁₋₆ alkyl (preferably pyrrolidinylmethyl, etc.), a C₆₋₁₄ aryl optionally having substituents (preferably a halogen atom, an optionally halogenated C₁₋₆ alkyl, hydroxy, an optionally halogenated C₁₋₆ alkoxy, an optionally halogenated C₁₋₆ alkyl-carbonyl, etc.), an aromatic heterocyclic group (preferably pyridyl, pyrimidinyl, etc.) optionally having substituents, a 5- to 7-membered non-aromatic heterocyclic group (preferably pyrrolidinyl, piperidinyl, piperazinyl, etc.) optionally having substituents, a C₇₋₁₉ aralkyloxy optionally having substituents, and the like.

Z is more preferably 1-piperidinyl or 1-piperazinyl, each of which has substituents at 4-position, which are selected from a C₆₋₁₄ aryloxy optionally having substituents [preferably hydroxy, a halogen atom, cyano, an optionally halogenated C₁₋₆ alkyl, an optionally halogenated C₁₋₆ alkoxy, an optionally halogenated C₁₋₆ alkylthio, an optionally halogenated C₁₋₆ alkylsulfonyl, sulfamoyl, a 5 or 6-membered aromatic heterocyclic group (preferably tetrazolyl, etc.), etc.], a C₇₋₁₉ aralkyl optionally having substituents, a C₆₋₁₄ aryl-carbonyl optionally having substituents (preferably a halogen atom, etc.), a C₆₋₁₄ aryl-sulfonyl optionally having substituents (preferably an optionally halogenated C₁₋₆ alkyl, etc.), a C₇₋₁₉ aralkyloxy-carbonyl optionally having substituents, a heterocyclic carbonyl (preferably indolylcarbonyl, etc.) optionally having substituents (preferably an optionally halogenated C₁₋₆ alkyl, etc.), a 5- to 7-membered non-aromatic heterocyclic ring-C₁₋₆ alkyl (preferably pyrrolidinylmethyl, etc.), a C₆₋₁₄ aryl optionally having substituents (preferably a halogen atom, an optionally halogenated C₁₋₆ alkyl, hydroxy, an optionally halogenated C₁₋₆ alkoxy, an optionally halogenated C₁₋₆ alkyl-carbonyl, etc.), an aromatic heterocyclic group (preferably pyridyl, pyrimidinyl, etc.) optionally having substituents, a 5- to 7-membered non-aromatic heterocyclic group (preferably pyrrolidinyl, piperidinyl, piperazinyl, etc.) optionally having substituents and a C₇₋₁₉ aralkyloxy optionally having substituents.

Za is preferably a hydrogen atom or phenyl, and more preferably a hydrogen atom.

Preferable examples of compound (I) include the following compounds.
1) A compound wherein
   X is a halogen atom (preferably a chlorine atom), X' is a hydrogen atom;
   R¹ and R² are the same or different and each is a C₁₋₆ alkyl (preferably methyl);
   R³ is a C₁₋₆ alkyl (preferably methyl);
   R⁴ is a hydrogen atom;
   Y is a C₁₋₆ alkylene (preferably -CH₂-) or -alka-CO-alkb-(each symbol is as defined above) (preferably -CO-);
   Z is a cyclic group optionally having substituents [preferably a 4- to 10-membered monocyclic non-aromatic heterocyclic group (preferably azetidinyl, pyrrolidinyl, piperidinyl, piperazinyl, morpholinyl, thiomorpholinyl, diazepanyl, tetrahydrofuranyl, etc.) which may have 1 to 3 substituents selected from oxo, an optionally halogenated C₁₋₆ alkyl, a C₆₋₁₄ aryloxy which may have substituents [preferably hydroxy, a halogen atom, cyano, an optionally halogenated C₁₋₆ alkyl, an optionally halogenated C₁₋₆ alkoxy, an optionally halogenated C₁₋₆ alkylthio, an optionally halogenated C₁₋₆ alkylsulfonyl, sulfamoyl, a 5 or 6-membered aromatic heterocyclic group (preferably tetrazolyl, etc.), etc.], a C₇₋₁₉ aralkyl optionally having substituents, carbamoyl, an optionally halogenated C₁₋₆ alkyl-carbonyl, an optionally halogenated C₁₋₆ alkylsulfonyl, a C₆₋₁₄ aryl-carbonyl optionally having substituents (preferably a halogen atom, etc.), a C₆₋₁₄ aryl-sulfonyl optionally having substituents (preferably an optionally halogenated C₁₋₆ alkyl, etc.), a C₇₋₁₉ aralkyloxy-carbonyl optionally having substituents, a heterocyclic carbonyl (preferably indolylcarbonyl, etc.) optionally having substituents (preferably an optionally halogenated C₁₋₆ alkyl, etc.), a C₆₋₁₄ aryl optionally having substituents (preferably a halogen atom, an optionally halogenated C₁₋₆ alkyl, hydroxy, an optionally halogenated C₁₋₆ alkoxy, an optionally halogenated C₁₋₆ alkyl-carbonyl, etc.), an aromatic heterocyclic group (preferably pyridyl, pyrimidinyl, etc.) optionally having substituents, a 5- to 7-membered non-aromatic heterocyclic group (preferably pyrrolidinyl, piperidinyl, piperazinyl, etc.) optionally having substituents, etc.];
   Ya is a bond; and
   Za is a hydrogen atom.
2) A compound wherein
   X is a halogen atom (preferably a chlorine atom), X' is a hydrogen atom;
   R¹ and R² are the same or different and each is a C₁₋₆ alkyl (preferably methyl);
   R³ is a C₁₋₆ alkyl (preferably methyl);
   R⁴ is a hydrogen atom;
   Y is a C₁₋₆ alkylene (preferably -CH₂-), -alka-CO-alkb- or - alkc-CO-alkd-NR⁵-alke- (each symbol is as defined above; R⁵ is preferably a hydrogen atom; or a C₁₋₆ alkyl which may have 1 to 3 substituents selected from a halogen atom and an optionally halogenated C₁₋₆ alkoxy);
   Z is a cyclic group optionally having substituents [preferably (1) phenyl, (2) a 5 or 6-membered aromatic heterocyclic group (preferably furyl, pyrrolyl, imidazolyl, pyridyl, etc.), (3) a C₃₋₈ cycloalkyl (preferably cyclopropyl, cyclohexyl, dihydroindenyl, etc.) which may be condensed with benzene ring, (4) a 4- to 10-membered monocyclic non-aromatic heterocyclic group (preferably azetidinyl, pyrrolidinyl, piperidinyl, piperazinyl, morpholinyl, thiomorpholinyl, diazepanyl, tetrahydrofuranyl, etc.) or (5) a 4- to 10-membered bicyclic non-aromatic heterocyclic group (preferably tetrahydroisoquinolinyl, octahydroisoquinolinyl, etc.), each of which may have 1 to 3 substituents selected from hydroxy, a halogen atom, oxo, an optionally halogenated C₁₋₆ alkyl, an optionally halogenated C₁₋₆ alkoxy, an optionally halogenated C₁₋₆ alkylthio, amino, a mono-C₁₋₆ alkylamino, a di-C₁₋₆ alkylamino, an optionally halogenated C₁₋₆ alkyl-carboxamide, a C₆₋₁₄ aryloxy optionally having substituents [preferably hydroxy, a halogen atom, cyano, an optionally halogenated C₁₋₆ alkyl, an optionally halogenated C₁₋₆ alkoxy, an optionally halogenated C₁₋₆ alkylthio, an optionally halogenated C₁₋₆ alkylsulfonyl, sulfamoyl, a 5 or 6-membered aromatic heterocyclic group (preferably tetrazolyl, etc.), etc.], a C₇₋₁₉ aralkyl optionally having substituents, carbamoyl, an optionally halogenated C₁₋₆ alkyl-carbonyl, an optionally halogenated C₁₋₆ alkylsulfonyl, a C₆₋₁₄ aryl-carbonyl optionally having substituents (preferably halogen atom, etc.), a C₆₋₁₄ aryl-sulfonyl optionally having substituents (preferably an optionally halogenated C₁₋₆ alkyl, etc.), a C₇₋₁₉ aralkyloxy-carbonyl optionally having substituents, a heterocyclic carbonyl (preferably indolylcarbonyl, etc.) optionally having substituents (preferably an optionally halogenated C₁₋₆ alkyl, etc.), a 5- to 7-membered non-aromatic heterocyclic ring-C₁₋₆ alkyl (preferably pyrrolidinylmethyl, etc.), a C₆₋₁₄ aryl optionally having substituents (preferably a halogen atom, an optionally halogenated C₁₋₆ alkyl, hydroxy, an optionally halogenated C₁₋₆ alkoxy, an optionally halogenated C₁₋₆ alkyl-carbonyl, etc.), an aromatic heterocyclic group (preferably pyridyl, pyrimidinyl, etc.) optionally having substituents, a 5- to 7-membered non-aromatic heterocyclic group (preferably pyrrolidinyl, piperidinyl, piperazinyl, etc.) optionally having substituents, a C₇₋₁₉ aralkyloxy optionally having substituents, etc.];
   Ya is a bond or a C₁₋₆ alkylene (preferably a bond); and
   Za is a hydrogen atom or phenyl (preferably a hydrogen atom).
3) A compound wherein
   X is a halogen atom (preferably a chlorine atom), X' is a hydrogen atom;
   R¹ and R² are the same or different and each is a C₁₋₆ alkyl (preferably methyl);
   R³ is a C₁₋₆ alkyl (preferably methyl) ;
   R⁴ is a hydrogen atom;
   Y is a bond, a C₁₋₆ alkylene, -alka-CO-alkb-, - alkc-CO-alkd-NR⁵-alke-, -alkc-CO-alkd-O-alke-, -alkc-CO-alkd-S-alke-, -alkf-CONR⁵-alkg-O-alkh-, - alkf-CONR⁵-alkg-NR^{5a}-alkh-, -alkf-CO-alkg-NR⁵CO-alkh- (each symbol is as defined above; R⁵ is preferably a hydrogen atom; or a C₁₋₆ alkyl,which may have 1 to 3 substituents selected from a halogen atom and an optionally halogenated C₁₋₆ alkoxy);
   Z is a hydrogen atom;
   Ya is a bond or a C₁₋₆ alkylene; and
   Za is a hydrogen atom.
4) A compound wherein
   X is a halogen atom (preferably a chlorine atom), X' is a hydrogen atom;
   R¹ and R² are the same or different and each is a C₁₋₆ alkyl (preferably methyl);
   R³ is a C₁₋₆ alkyl (preferably methyl) ;
   R⁴ is a hydrogen atom;
   Y is -alka-CO-alkb- (each symbol is as defined above) (preferably -CO-);
   Z is 1-piperidinyl or 1-piperazinyl, each of which has substituents at the 4-position, which are selected from a C₆₋₁₄ aryloxy optionally having substituents [preferably hydroxy, a halogen atom, cyano, an optionally halogenated C₁₋₆ alkyl, an optionally halogenated C₁₋₆ alkoxy, an optionally halogenated C₁₋₆ alkylthio, an optionally halogenated C₁₋₆ alkylsulfonyl, sulfamoyl, a 5 or 6-membered aromatic heterocyclic group (preferably tetrazolyl, etc.), etc.], a C₇₋₁₉ aralkyl optionally having substituents, a C₆₋₁₄ aryl-carbonyl optionally having substituents (preferably a halogen atom, etc.), a C₆₋₁₄ aryl-sulfonyl optionally having substituents (preferably an optionally halogenated C₁₋₆ alkyl, etc.), a C₇₋₁₉ aralkyloxy-carbonyl optionally having substituents, a heterocyclic carbonyl (preferably indolylcarbonyl, etc.) optionally having substituents (preferably an optionally halogenated C₁₋₆ alkyl, etc.), a 5- to 7-membered non-aromatic heterocyclic ring-C₁₋₆ alkyl (preferably pyrrolidinylmethyl, etc.), a C₆₋₁₄ aryl optionally having substituents (preferably a halogen atom, optionally halogenated C₁₋₆ alkyl, hydroxy, an optionally halogenated C₁₋₆ alkoxy, an optionally halogenated C₁₋₆ alkyl-carbonyl, etc.), an aromatic heterocyclic group (preferably pyridyl, pyrimidinyl, etc.) optionally having substituents, a 5- to 7-membered non-aromatic heterocyclic group (preferably pyrrolidinyl, piperidinyl, piperazinyl, etc.) optionally having substituents and a C₇₋₁₉ aralkyloxy optionally having substituents;
   Ya is a bond; and
   Za is a hydrogen atom.

Particularly preferable examples of the compound (I) include
N-[(1R,2S)-1-[((3R)-6-chloro-3-[(dimethylamino)methyl]-3,4-dihydro-1(2H)-quinolinyl)carbonyl]-2-(1H-indol-3-yl)propyl]-1-[(1-methyl-1H-indol-2-yl)carbonyl]-4-piperidinecarboxamide,
N-[(1R,2S)-1-[((3R)-6-chloro-3-[(dimethylamino)methyl]-3,4-dihydro-1(2H)-quinolinyl)carbonyl]-2-(1H-indol-3-yl)propyl]-4-phenoxy-1-piperidinecarboxamide,
(-)-N-[1-[((3R)-6-chloro-3-[(dimethylamino)methyl]-3,4-dihydro-1(2H)-quinolinyl)carbonyl]-2-(1H-indol-3-yl)propyl]-4-(4-hydroxyphenoxy)-1-piperidinecarboxamide,
N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-4-(4-fluorophenoxy)-1-piperidinecarboxamide,
4-benzoyl-N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-1-piperazinecarbokamide, and a salt thereof.

When the compound (I) is in the form of a salt, concrete examples thereof include salts with inorganic bases, ammonium salts, salts with organic bases, salts with inorganic acids, salts with organic acids and salts with basic or acidic amino acids.

Preferable examples of the salts with inorganic bases include alkali metal salts such as sodium salts, potassium salts, etc; alkaline earth metal salts such as calcium salts, magnesium salts, barium salts, etc; aluminum salts, etc.

Preferable examples of the salts with organic bases include salts with trimethylamine, triethylamine, pyridine, picoline, ethanolamine, diethanolamine, triethanolamine, dicyclohexylamine, N,N'-dibenzylethylenediamine and the like.

Preferable examples of the salts with inorganic acids include salts with hydrochloric acid, hydrobromic acid, nitric acid, sulfuric acid, phosphoric acid and the like.

Preferable examples of the salts with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of the salts with basic amino acids include salts with arginine, lysine, ornithine and the like.

Preferable examples of the salts with acidic amino acids include salts with aspartic acid, glutamic acid and the like.

Of these, pharmaceutically acceptable salts are preferable.

For instance, when compound (I) has an acidic functional group, compound (I) may be in the form of inorganic salts such as alkali metal salts (e.g., sodium salt, potassium salt, etc.) and alkaline earth metal salts (e.g., calcium salt, magnesium salt, barium salt, etc.), ammonium salts, etc. When compound (I) has a basic functional group, compound (I) may be in the form of inorganic salts such as hydrochloride, sulfate, phosphate and hydrobromide; or organic salts such as acetate, maleate, fumarate, succinate, methanesulfonate, p-toluenesulfonate, citrate and tartrate.

The prodrug of the compound (I) means a compound which is converted into the compound (I) through a reaction due to an enzyme, a gastric acid, etc. under the physiological condition in the living body, that is, a compound which is enzymatically converted into the compound (I) through oxidation, reduction, hydrolysis, etc.; a compound which is converted into the compound (I) through hydrolysis, etc. with a gastric acid, etc.; and the like.

Examples of the prodrug of the compound (I) include a compound wherein an amino group of the compound (I) is substituted with an acyl, an alkyl, phosphoric acid, etc. (e.g., a compound wherein an amino group of the compound (I) is substituted with eicosanoyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl, tert-butyl, etc.);
a compound wherein a hydroxy group of the compound (I) is substituted with an acyl, an alkyl, phosphoric acid, boric acid, etc. (e.g., a compound wherein a hydroxy group the compound (I) is substituted with acetyl, palmitoyl, propanoyl, pivaloyl, succinyl, fumaryl, alanyl, dimethylaminomethylcarbonyl, etc.);
a compound wherein a carboxyl group of the compound (I) is modified with ester, amide, etc. (e.g., a compound wherein a carboxyl group of the compound (I) is modified with ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester, methyl amide, etc.); and the like. These prodrugs can be produced by methods known *per se* from the compound (I).

The prodrug of the compound (I) may be a compound which is converted into the compound (I) under the physiological conditions as described in the "Pharmaceutical Research and Development", Vol. 7 (Drug Design), pp. 163-198 published in 1990 by Hirokawa Publishing Co.

The process for producing the compound (I) is mentioned below.

The compound (I) can be produced by a means known *per se*, for example, by the methods exemplified in the following schemes, or a method similar thereto, and the like.

Compounds described in the following schemes may be in the form of salts. Such salts are exemplified by those similar to the salts of the compound (I).

The "room temperature" generally means a temperature falling between 0°C and 30°C in the present specification.

The following reactions such as alkylation, hydrolysis, amination, esterification, amidation, etherification, oxidation, reduction, urea formation, etc. may be conducted according to methods known per se, such as those described in Organic Functional Group Preparations, 2nd Ed., Academic Press Inc., 1989 and in Comprehensive Organic Transformations, VCH Publishers Inc., 1989. or a method similar thereto. 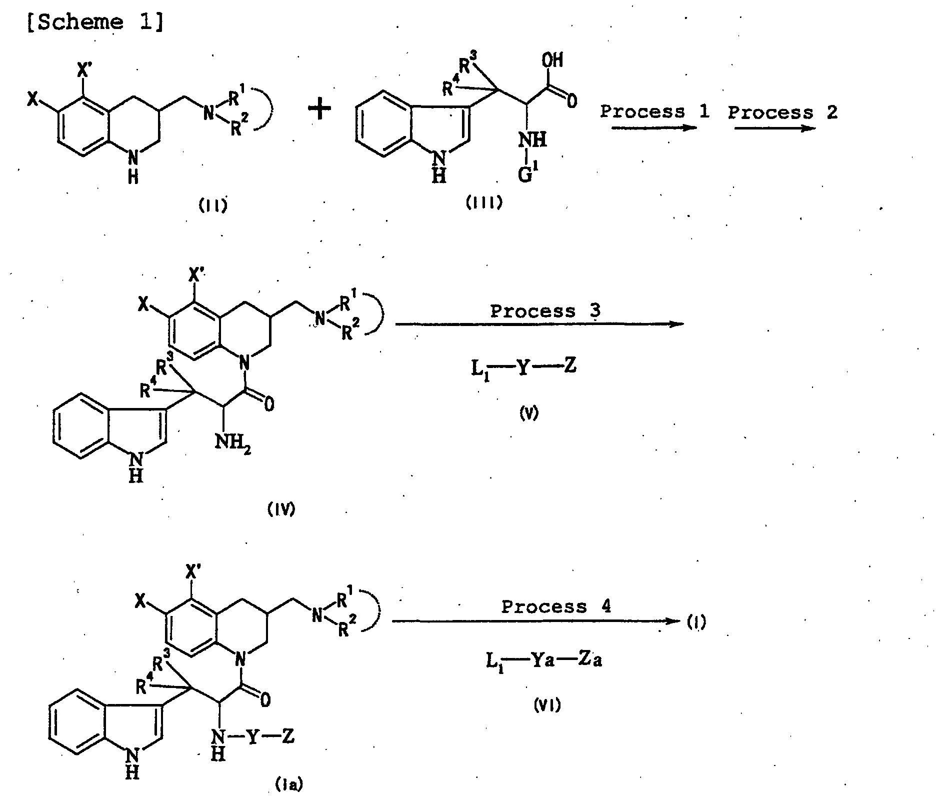 wherein G¹ represents a protective group of amino, L¹ represents a leaving group, and other symbols are as defined above.

The protective group of amino represented by G includes, for example, formyl, a C₁₋₆ alkyl-carbonyl (e.g., acetyl, propionyl, etc.), a C₁₋₆ alkoxy-carbonyl (e.g., methoxycarbonyl, ethoxycarbonyl, tert-butoxycarbonyl, etc.), benzoyl, a C₇₋₁₀ aralkyl-carbonyl (e.g., benzylcarbonyl, etc.), a C₇₋₁₄ aralkyloxy-carbonyl (e.g., benzyloxycarbonyl, 9-fluorenylmethoxycarbonyl, etc.), trityl, phthaloyl, N,N-dimethylaminomethylene, a silyl (e.g., trimethylsilyl, triethylsilyl, dimethylphenylsilyl, tert-butyldimethylsilyl, tert-butyldiethylsilyl, etc.), a C₂₋₆ alkenyl (e.g., 1-allyl, etc.), and the like. These groups may be substituted by 1 to 3 of halogen atoms (e.g., fluorine, chlorine, bromine, iodine, etc.), a C₁₋₆ alkoxy (e.g., methoxy, ethoxy, propoxy, etc.), nitro, and the like. The protective group of amino is preferably 9-fluorenylmethoxycarbonyl, and the like.

The "leaving group" represented by L¹ includes, for example, (1) a halogen atom (e.g., chlorine, bromine, iodine, etc.), (2) an optionally halogenated C₁₋₆ alkylsulfonyloxy (e.g., methanesulfonyloxy, ethanesulfonyloxy, trifluoromethanesulfonyloxy, etc.), (3) a C₆₋₁₀ arylsulfonyloxy optionally having substituents, (4) hydroxy, and the like.

The "substituent" of the "C₆₋₁₀ arylsulfonyloxy optionally having substituents" includes, for example, a halogen atom, an optionally halogenated C₁₋₆ alkyl, an optionally halogenated C₁₋₆ alkoxy, and the like. The number of the substituents is, for example, 1 to 3.

Concrete examples of the "C₆₋₁₀ arylsulfonyloxy optionally having substituents" include benzenesulfonyloxy, p-toluenesulfonyloxy, 1-naphthalenesulfonyloxy, 2-naphthalenesulfonyloxy, and the like.

Each process of Scheme 1 is described in detail in the following.

### Process 1 (amidation)

Said "amidation" includes, for example, the below mentioned method such as i) the method using a dehydrating/condensing agent or ii) the method using a reactive derivative of carboxy.
i) The method using a dehydrating/condensing agent
   Compound (II), about 1 to about 5 equivalents of Compound (III), and about 1 to about 2 equivalents of a dehydrating/condensing agent are reacted in an inert solvent.
   Said "dehydrating/condensing agent" includes, for example, dicyclohexylcarbodiimide (DCC), 1-ethyl-3-(3-dimethylaminopropyl)carbodiimide hydrochloride (WSC), and the like. Of these, WSC is preferable.
   The "inert solvent" includes, for example, nitriles, amides, halogenated hydrocarbons, ethers, and the like. These may be used on mixing two or more kinds at a suitable proportion. Of these, preferred are acetonitrile, DMF, dichloromethane, THF, and the like.
   The reaction temperature is generally about -20°C to about 50°C, preferably at room temperature.
   The reaction time is generally about 10 hours to about 24 hours.
   In the present reaction, about 1 to about 1.5 equivalents of 1-hydroxybenzotriazole (HOBt) or 1-hydroxy-7-azabenzotriazole (HOAt) is used as necessary.
   In the present reaction, about 1 to about 5 equivalents of a base is also used as necessary.
   Said "base" includes, for example; 1) strong bases such as alkali metal or alkaline earth metal hydrides (e.g., lithium hydride, sodium hydride, potassium hydride, calcium hydride, etc.), alkali metal or alkaline earth metal amides (e.g., lithium amide, sodium amide, lithium diisopropylamide, lithium dicyclohexylamide, lithium hexamethyldisilazide, sodium hexamethyldisilazide, potassium hexamethyldisilazide, etc.), alkali metal or alkaline earth metal lower-alkoxides (e.g., sodium methoxide, sodium ethoxide, potassium tert-butoxide, etc.), and the like;
2) inorganic bases such as alkali metal or alkaline earth metal hydroxides (e.g., sodium hydroxide, potassium hydroxide, lithium hydroxide, barium hydroxide, etc.), alkali metal or alkaline earth metal carbonates (e.g., sodium carbonate, potassium carbonate, cesium carbonate, etc.), alkali metal or alkaline earth metal hydrogen carbonates (e.g., sodium hydrogen carbonate, potassium hydrogen carbonate, etc.), and the like; and
3) organic bases such as amines exemplified by triethylamine, diisopropylethylamine, N-methylmorpholine, dimethylaminopyridine, DBU (1,8-diazabicyclo[5.4.0]undec-7-ene), DBN (1,5-diazabicyclo[4.3.0]non-5-ene), etc., basic heterocyclic compounds exemplified by pyridine, imidazole, 2,6-lutidine, etc. Of these, preferred are triethylamine and 4-dimethylaminopyridine, and the like.

### ii) Method using a reactive derivative of carboxy

The reactive derivative of Compound (III) and about 1 to about 5 equivalents (preferably about 1 to about 3 equivalents) of Compound (II) are reacted in an inert solvent.

The reactive derivatives of the "reactive derivative of Compound (III)" include an acid halide (e.g., an acid chloride, an acid bromide, etc.), a mixed acid anhydride (e.g., an anhydride with a C₁₋₆ alkyl carboxylic acid, a C₆₋₁₀ aryl carboxylic acid or a C₁₋₆ alkyl carbonic acid, etc.), and an activated ester (e.g., an ester with phenol optionally having substituents, 1-hydroxybenzotriazole, 1-hydroxy-7-azabenzotriazole, 1-hydroxy-5-norbornen-2,3-dicarboxyimide or N-hydroxysuccinimide, etc.).

The "substituent" of the "phenol optionally having substituents" includes, for example, a halogen atom, nitro, an optionally halogenated C₁₋₆ alkyl, an optionally halogenated C₁₋₆ alkoxy, and the like. The number of the substituents is, for example, 1 to 5. Concrete examples of the "phenol optionally having substituents" include phenol, pentachlorophenol, pentafluorophenol, p-nitrophenol, and the like. The reactive derivative is preferably an acid halide.

The "inert solvent" includes, for example, ethers, halogenated hydrocarbons, aromatic solvents, nitriles, amides, ketones, sulfoxides, water, esters, and the like. These may be used on mixing two or more kinds at a suitable proportion. Of these, preferred are tetrahydrofuran (THF), acetonitrile, dichloromethane, chloroform, ethyl acetate, and the like.

The reaction temperature is generally about -20°C to about 50°C, preferably at room temperature.

The reaction time is generally about 5 minutes to about 40 hours, preferably about 1 to about 5 hours.

In the present reaction, about 1 to about 10 equivalents, preferably about 1 to about 3 equivalents of a base is used if necessary.

As said "base", those exemplified for the above-mentioned "the method using a dehydrating/condensing agent" are used. Of these, preferred are sodium hydride, potassium carbonate, sodium carbonate, sodium hydroxide, potassium hydroxide, sodium hydrogen carbonate, potassium hydrogen carbonate, triethylamine, pyridine, and the like.

In the present reaction, about 0.1 to about 1 equivalent, preferably about 0.1 to about 0.5 equivalent, of a phase-transfer catalyst is used where necessary.

Said "phase-transfer catalyst" includes, for example, quaternary ammonium salt such as tetrabutylammonium hydrogensulfate, benzyltriethylammonium chloride, and the like. Of these, preferred is tetrabutylammonium hydrogensulfate.

The aforementioned compound (II) can be produced according to a method known per se, such as the method described in WO99/52875 or a similar method thereof.

The aforementioned compound (III) can be produced by introducing a protective group of amino G¹ into a compound represented by the formula: 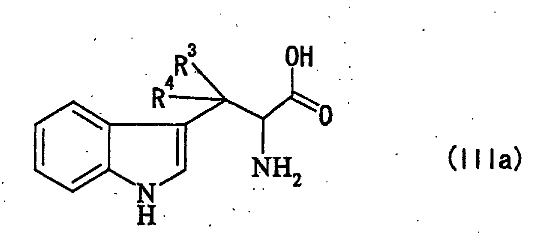 wherein the symbols in the formula are as defined above, or a salt thereof.

Here, introduction of the protective group of amino is conducted according to a method known *per se*, such as the methods described in Protective Groups in Organic Synthesis (1980) and the like.

The aforementioned compound (IIIa) can be produced according to a method known *per se*. Such method includes, for example, methods described in Tetrahedron Letters, 39, 3445(1998); Tetrahedron Letters, 39, 8729(1998) and the like, or a similar method thereof and the like can be mentioned.

The compound (III) can be also produced by subjecting a compound obtained by subjecting compound (IIIa) to protection of carboxy group and introducing a protective group of amino G¹ to deprotection of carboxy group.

Here, protection of carboxy group and deprotection of carboxy group can be carried out according to a method known per se, for example, methods described in Protective Groups in Organic Synthesis (1980) and the like.

The protective group of carboxy group used in these reactions includes, for example, a C₁₋₆ alkyl (e.g., methyl, ethyl, propyl, isopropyl, butyl, tert-butyl, etc.), a C₇₋₁₁ aralkyl (e.g., benzyl, etc.), phenyl, trityl, a silyl (e.g., trimethylsilyl, triethylsilyl, dimethylphenylsilyl, tert-butyldimethylsilyl, tert-butyldiethylsilyl, etc.), a C₂₋₆ alkenyl (e.g., 1-allyl, etc.), and the like. These groups may be substituted by 1 to 3 halogen atoms (e.g., fluorine, chlorine, bromine, iodine, etc.), a C₁₋₆ alkoxy (e.g., methoxy, ethoxy, propoxy, etc.) or nitro, and the like.

### Process 2 (deprotection)

The present reaction can be carried out by methods known *per se* according to the kind of G¹ which is a protective group of amino.

### Process 3 (Introduction of the group represented by the formula: -Y-Z (wherein each symbol is as defined above))

For Y in compound (V), when a functional group adjacent to the leaving group L¹ is CO, SO or SO₂, the present process is conducted in the same manner as the amidation in the above-mentioned Process 1.

Moreover, when a functional group adjacent to the leaving group L¹ is CONR⁵ (R⁵ has the same meaning as above) or COO for Y in compound (V), the present process is conducted by urea formation and carbamoylation.

Said urea formation and carbamoylation are conducted by, for example, reacting compound (IV) with 1 to 2 equivalents of the compound represented by the formula: L²-CO-L³ (VII) wherein L² and L³ represent leaving groups, in an inert solvent at room temperature for about 0.5 to 5 hours, and then reacting the obtained compound with 1 to 2 equivalents of the compound represented by the formula: H-L⁴-Y'-Z (VIII) wherein L⁴ represents NR⁵ (R⁵ has the same meaning as above) or oxygen atom, Y' represents a spacer having a main chain of 1 to 7 atoms and Z has the same meaning as above, in an inert solvent at room temperature for about 0.5 to 24 hrs.

The "spacer having a main chain of 1 to 7 atoms", which is represented by Y', is exemplified by the above-mentioned "spacer having a main chain of 1 to 8 atoms" exemplified for Y, which has a main chain of 1 to 7 atoms.

The "leaving group" represented by L² or L³ is exemplified by one mentioned as the above L¹. Of these, preferred are chlorine and succinimidoxy, and succinimidoxy is specifically preferred.

The "inert solvent" includes, for example, nitriles, ethers, halogenated hydrocarbons, etc. These may be used on mixing two or more kinds at a suitable proportion. Of these, preferred are acetonitrile, THF, dichloromethane, and the like.

In the present reaction, about 1 to 5 equivalents of a base (e.g., N-ethyldiisopropylamine, etc.) is also used where necessary.

Moreover, when a functional group adjacent to the leaving group L¹ is non-carbonyl carbon atom for Y in compound (V), the present reaction is conducted by alkylation.

Said alkylation is conducted by, for example, reacting compound (IV) with about 1 to 5 equivalents (preferably about 1 to 2 equivalents) of compound (V) in an inert solvent in the presence of a base.

As said "base", those exemplified in the above-mentioned Process 1 are used. Of those, preferred are potassium carbonate, sodium hydride, potassium hydroxide, and the like. The amount of the base to be used is, for example, about 1 to 5 equivalents of compound (IV).

The "inert solvent" includes, for example, alcohols, ethers, halogenated hydrocarbons, aromatic solvents, nitriles, amides, ketones, sulfoxides, water, and the like. These may be used on mixing two or more kinds at a suitable proportion. Of these, preferred are acetonitrile, N,N-dimethyl formamide (DMF), acetone, ethanol, pyridine, water, and the like.

The reaction temperature is generally -20°C to 100°C, preferably at room temperature to 80°C.

The reaction time is generally 0.5 hr to 1 day.

The aforementioned compound (V), compound (VII) and compound (VIII) can be produced according to a method known *per se.*

In the aforementioned Process 3, a compound represented by the formula: OHC-Y'-Z (IX)
[wherein the symbols in the formula are as defined above] may be used instead of compound (V).

The compound (Ia) can be produced by subjecting compound (IX) and compound (IV) to a reductive alkylation. The present reaction can be conducted according to a method known *per se*.

Said reductive alkylation can be conducted, for example, by reacting compound (IV) and about 1 to 5 equivalents (preferably 1 to 2 equivalents) of the compound (IX) in an inert solvent in the presence of metal hydride.

The "metal hydride" includes, for example, aluminum hydride, lithium aluminum hydride, sodium borohydride, lithium borohydride, sodium cyanoborohydride, lithium cyanoborohydride, sodium triacetoxyborohydride, borane complexes (e.g., borane-THF complex, catechol-borane, etc.), dibutyl aluminum hydride, a mixture of these metal hydrides with Lewis acids (e.g., aluminum chloride, titanium tetrachloride, cobalt chloride, etc.) or phosphorus oxychloride, and the like. The metal hydride is preferably sodium cyanoborohydride, sodium triacetoxyborohydride, and the like.

The amount of the metal hydride to be used is, for example, generally about 1 to 5 equivalents of compound (IV).

The reaction temperature varies depending on the kind of metal hydride to be used, but is generally about -70°C to 100°C, preferably at room temperature to 80°C.

The reaction time is generally about 0.1 hr to 48 hrs.

The "inert solvent" includes, for example, alcohols (preferably ethanol), ethers (preferably THF), nitriles (preferably acetonitrile), acetic acid and the like. These may be used on mixing two or more kinds at a suitable proportion.

The aforementioned compound (IX) can be produced according to a method known *per se*.

### Process 4 (Introduction of the group represented by the formula: -Ya-Za (each symbol is as defined above))

The present reaction is conducted in the similar manner to the aforementioned process 3.

In the thus-obtained compound (I), functional groups in a molecule can be converted into the desired functional groups by combination of *per se* known chemical reactions. Examples of the chemical reactions include oxidation, reduction, alkylation, hydrolysis, amination, esterification, aryl-coupling reaction, deprotection, and the like.

The above-mentioned "alcohols" includes, for example, methanol, ethanol, isopropanol, tert-butanol, and the like.

The above-mentioned "ethers" includes, for example, diethyl ether, tetrahydrofuran (THF), 1,4-dioxane, 1,2-dimethoxyethane, and the like.

The above-mentioned "halogenated hydrocarbons" includes, for example, dichloromethane, chloroform, 1,2-dichloroethane, carbon tetrachloride, and the like,

The above-mentioned "aromatic solvents" includes, for example, benzene, toluene, xylene, pyridine, and the like.

The above-mentioned "amides" includes, for example, N,N-dimethylformamide (DMF), N,N-dimethylacetamide, N-methylpyrrolidone, and the like.

The above-mentioned "ketones" includes, for example, acetone, methyl ethyl ketone, and the like.

The above-mentioned "sulfoxides" includes, for example, dimethylsulfoxide (DMSO), and the like.

The above-mentioned "nitriles" includes, for example, acetonitrile, propionitrile, and the like.

The above-mentioned "esters" includes, for example, ethyl acetate, and the like.

In the above-mentioned reactions where the starting compounds are substituted by any of amino, carboxy, hydroxy and carbonyl, those groups may be protected by ordinary protective groups which are generally used in peptide chemistry, and the like. The protective groups may be removed after the reaction, if necessary, to give the desired compounds.

The protective group of amino is exemplified by one mentioned as the above G.

The protective group of carboxy includes, for example, a C₁₋₆ alkyl (e.g., methyl, ethyl, propyl, isopropyl, butyl, tert-butyl, etc.), a C₇₋₁₁ aralkyl (e.g., benzyl, etc.), phenyl, trityl, a silyl (e.g., trimethylsilyl, triethylsilyl, dimethylphenylsilyl, tert-butyldimethylsilyl, tert-butyldiethylsilyl, etc.), a C₂₋₆ alkenyl (e.g., 1-allyl, etc.), and the like. These groups may be substituted by 1 to 3 halogen atoms (e.g., fluorine, chlorine, bromine, iodine, etc.), a C₁₋₆ alkoxy (e.g., methoxy, ethoxy, propoxy, etc.) or nitro, and the like.

The protective group of hydroxy includes, for example, a C₁₋₆ alkyl (e.g., methyl, ethyl, propyl, isopropyl, butyl, tert-butyl, etc.), phenyl, trityl, a C₇₋₁₀ aralkyl (e.g., benzyl, etc.), formyl, a C₁₋₆ alkyl-carbonyl (e.g., acetyl, propionyl, etc.), benzoyl, a C₇₋₁₀ aralkyl-carbonyl (e.g., benzylcarbonyl, etc.), 2-tetrahydropyranyl, 2-tetrahydrofuranyl, a silyl (e.g., trimethylsilyl, triethylsilyl, dimethylphenylsilyl, tert-butyldimethylsilyl, tert-butyldiethylsilyl, etc.), a C₂₋₆ alkenyl (e.g., 1-allyl, etc.), and the like. These groups may be substituted by 1 to 3 of halogen atoms (e.g., fluorine, chlorine, bromine, iodine, etc.), a C₁₋₆ alkyl (e.g., methyl, ethyl, propyl, etc.), a C₁₋₆ alkoxy (e.g., methoxy, ethoxy, propoxy, etc.), nitro, and the like.

The protective group of carbonyl includes, for example, a cyclic acetal (e.g., 1,3-dioxane, etc.), a non-cyclic acetal (e.g., di-C₁₋₆ alkylacetal, etc.), and the like.

These protective groups may be removed by methods known *per se*, such as the methods described in Protective Groups in Organic Synthesis, published by John Wiley and Sons, 1980, and the like. For example, employed are the methods using acids, bases, ultraviolet ray, hydrazine, phenylhydrazine, sodium N-methyldithiocarbamate, tetrabutylammonium fluoride, palladium acetate, trialkylsilylhalide (e.g., trimethylsilyliodide, trimethylsilylbromide, etc.), etc.; reduction, and the like.

The compound (I) can be isolated and purified by any known procedures, such as through solvent extraction, pH adjustment, redistribution, crystallization, recrystallization, chromatography, and the like. The starting compounds for compound (I) and their salts can be also isolated and purified according to the same known procedures as above, but without any isolation procedure, they may be used in the next process while they are in reaction mixtures.

The compound (I) may also be in the form of hydrates or non-hydrates thereof.

Where compound (I) includes optical isomers, stereo isomers, regio isomers and rotational isomers, those are within the scope of compound (I), and can be isolated as their single compound through synthesis or separation known *per se.* For example, where optical isomers of compound (I) exist, those separated from their mixtures through optical resolution are within the scope of compound (I).

The optical isomers can be produced by methods known *per se*. Concretely, optically active synthetic intermediates may be used, or mixtures of racemate of the final product are subjected to ordinary optical resolution to give the corresponding optical isomers.

For the optical resolution, employable are methods known *per se*, such as a fractional recrystallization method, a chiral column method, a diastereomer method, and the like.

### 1) Fractional Recrystallization Method

The method which comprises allowing a racemate to react with an optically active compound (e.g., (+)-mandelic acid, (-)-mandelic acid, (+)-tartaric acid, (-)-tartaric acid, (+)-1-phenethylamine, (-)-1-phenethylamine, cinchonine, (-)-cinchonidine, brucine, etc.) to give a salt, which is then isolated through fractional recrystallization method, followed by, where desired, subjecting the isolated compound to neutralization to obtain free optical isomers.

### 2) Chiral Column Method

The method of separating a racemate or a salt thereof, which comprises utilizing a column for fractionating optical isomers (chiral column). In the case of liquid column chromatography, for example, a mixture of optical isomers is applied to a chiral column, such as ENANTIO-OVM (manufactured by Tosoh Corp.), CHIRAL SERIES (manufactured by Daicel Co.), etc., which is then eluted with water, various buffers (e.g., phosphate buffer) and organic solvents (e.g., ethanol, methanol, isopropanol, acetonitrile, trifluoroacetic acid, diethylamine, etc.), singly or as a suitable mixture of them, to isolate the individual optical isomers. In the case of gas chromatography, for example, a chiral column such as CP-Chirasil-DeX CB (manufactured by GL Science Co.), etc. is used for isolation.

### 3) Diastereomer Method

A racemic mixture is chemically reacted with an optically-active reagent to give a mixture of diastereomer, which is subjected to ordinary separation means (e.g., fractional recrystallization, chromatography, etc.) to give single compounds. The thus-isolated single compounds are then chemically processed, for example, through hydrolysis to thereby remove the optically-active reagent site from the compounds to obtain optical isomers. For example, where compound (I) has a hydroxy group or a primary or secondary amino group in the molecule, it is condensed with an optically-active organic acid (e.g., MTPA [α-methoxy-α-(trifluoromethyl)phenyl-acetic acid], (-)-merithoxyacetic acid, etc.) or the like to give the corresponding ester-type or amide-type diastereomer. On the other hand, where compound (I) has a carboxylic acid group, it is condensed with an optically-active amine or alcohol reagent to give the corresponding amide-type or ester-type diastereomer. The thus-isolated diastereomer is then subjected to acidic or basic hydrolysis, through which it is converted into the optical isomer of the original compound.

The compound (I) has an optically active center at 2-position in 3-(indol-3-yl)propanoyl group, and in said optically active center, there exist (R)-isomer and (S)-isomer. Of these, preferred is (R)-isomer.

In addition, compound (I) has an optically active center at the 3-position in 1,2,3,4-tetrahydroquinolinyl. And in said optically active center, there exist (R)-isomer and (S)-isomer. The compound (I) is preferably (R)-isomer or (S)-isomer. Of these, preferred is (R)-isomer.

The compound (I) has an excellent somatostatin receptor binding inhibitory activity (i.e., somatostatin receptor agonist activity and antagonist activity). The somatostatin receptor here includes somatostatin subtypes 1, 2, 3, 4, 5, and the like. Especially, the compound (I) has a selective somatostatin subtype 2 receptor (SSTR2) binding inhibitory activity, particularly a somatostatin subtype 2 receptor agonist activity.

The compound (I) acts through various intracellular signal transduction systems with which somatostatin is associated. The "intracellular signal transduction systems" include, for example, that which involves adenylate cyclase, K⁺ channels, Ca²⁺ channels, dephosphorylation of protein, phospholipase C/inositol trisphosphate production systems, MAP kinase, Na⁺/H⁺ exchanger, phospholipase A2, a transcription factor such as NF-κB. The compound (I) modulates a direct or indirect cell proliferation inhibitory action or apoptosis both of which are associated with somatostatin.

Further, the compound (I) is low in its toxicity, and enhances or inhibits production and/or secretion of a variety of hormones, growth factors and physiologically active substances, etc. by effecting on somatostatin receptors in mammals (e.g., human, cattle, horse, dog, cat, monkey, mouse and rat, especially, human).

The "hormones" include, for example, growth hormone (GH), growth hormone-releasing hormones (GHRH), ghrelin, thyroid stimulating hormone (TSH), prolactin, insulin, glucagon, etc. The "growth factors" include, for example, insulin-like growth factor-1 (IGF-1) and vascular endothelial cell growth factor (VEGF). Said "physiologically active substances" include, for example, vasoactive intestinal polypeptide (VIP); gastrin; glucagon-like peptide-1 (GLP-1); glucose-dependent insulinotropic polypeptide (GIP); amylin; substance-P, CCK (cholecystokinin); amylase; interleukins such as interleukin-6 (IL-6), interleukin-1 (IL-1), etc.; cytokines such as TNF-α, etc.; cardiotropin, and the like.

Therefore, the compound (I) is safe and useful for disorders of the above intracellular signal transduction systems (e.g., diseases accompanied by excess sthenia or suppression, etc.); disease accompanied by disorders of regulating cell proliferation; diseases accompanied by disorders of production and/or secretion of hormones, growth factors, physiologically active substances, etc.; or facilitating growth, immune, gastroenteric or metabolic functions, etc; and the like.

For example, the compound (I) is useful (1) for drugs for treatment of tumors such as acromegaly, TSH-producing tumors, nonsecretory (afunctional) hypophysial tumors, ectopic ACTH (adrenocorticotrophic hormone)-producing tumors, medullar thyroid carcinoma, VIP-producing tumors, glucagon-producing tumors, gastrin-producing tumors, insulinoma and carcinoid, (2) for drugs for treatment of diabetes such as insulin-dependent (type I) and non-insulin dependent (type II) diabetes mellitus or a variety of diseases associated with them, namely, diabetic complications such as diabetic retinopathy, diabetic nephropathy, diabetic neuropathy, Doan syndrome and orthostatic hypotension, (3) for drugs for treatment of obesity or overeating, etc. by improvement of hyperinsulinemia or inhibition of appetite, etc. (4) for drugs for treatment of acute pancreatitis, chronic pancreatitis, pancreal/intestinal fistula, hemorrhagic ulcer, peptic ulcer, gastritis, hyperchylia, regurgitant esophagitis, etc. (5) for drugs for improvement of various symptoms accompanied by the *Helicobacter pylori* infection, for example, inhibitors of gastrin hypersecretion, etc. (6) for drugs for inhibition of amylase secretion accompanied by endoscopic cholangiopancreatography, and drugs for prognostic treatment of surgical operation of pancreas, (7) for drugs for treatment of diarrhea due to intestinal malabsorption, promotion of secretion or dyskinesia of the digestive tracts (for example, short bowel syndrome, etc.), diarrhea due to the drugs for cancer chemotherapy, diarrhea due to congenital small intestine atrophy, diarrhea due to neuroendocrine tumors such as VIP-producing tumors, diarrhea due to AIDS, diarrhea due to graft versus host reaction accompanied by bone marrow transplantation, diarrhea due to diabetes mellitus, diarrhea due to celiac plexus blocking, diarrhea due to systemic sclerosis and diarrhea due to eosinophilia, etc. (8) for drugs for treatment of dumping syndrome, irritable colitis, Crohn disease and inflammatory bowel disease, etc. (9) for drugs for treatment of tumors or cancers (e.g., thyroid cancer, colon cancer, breast cancer, prostate cancer, small cell lung cancer, non-small cell lung cancer, pancreatic cancer, stomach cancer, cholangiocarcinoma, hepatic cancer, vesical cancer, ovarian cancer, melanoma, osteosarcoma, chondrosarcoma, malignant pheochromocytoma, neuro-blastoma, brain tumors, thymoma, renal cancers, etc.), leukemia (e.g., leukemia of basophilic leukocyte, chronic lymphocytic leukemia, chronic myeloid leukemia, Hodgkin disease, and non-Hodgkin lymphoma, etc.); the drugs can be used for monotherapy or concomitant therapy with other anticancer drugs such as Tamoxifen, LHRH agonists, LHRH antagonists, interferon-α, β and γ, interleukin-2, etc.), (10) for drugs for prevention or treatment of hypertrophic cardiomyopathy, arteriosclerosis, valvular disease, myocardiac infarction (especially, myocardiac infarction post percutaneous transluminal coronary arterioplasty) and reangioplasty, (11) for drugs for treatment of hemorrhage of esophageal varicosis, cirrhosis and peripheral blood vessel disorders, (12) for drugs for treatment of diseases accompanied by general or local inflammation, for example, polyarteritis, rheumatoid arthritis, psoriasis, sunburn, eczema and allergy (e.g., asthma, atopic dermatitis and allergic rhinitis, etc.), because they modulate the secretion of physiologically active substances acting on the immune system (e.g., Substance P, tachykinin and cytokines), (13) for drugs for treatment of dementia (e.g., Alzheimer's disease, Alzheimer-type senile dementia, vascular/multi-infarct dementia, etc.), schizophrenia, epilepsy, depression, generalized anxiety disorder, sleep disorder, and multiple sclerosis, because they give influence on the production or secretion of nerve regulators, (14) for drugs for treatment of oculopathy (e.g., glaucoma, etc.), (15) for drugs for prevention or treatment of acute bacterial meningitis, acute virus encephalitis, adult respiratory distress syndrome, bacterial pneumonia, severe systemic mycotic infection, tuberculosis, spinal damage, bone fracture, hepatic failure, pneumonia, alcoholic hepatitis, virus A hepatitis, virus B hepatitis, virus C hepatitis, AIDS infection, human papilloma virus infection, influenza infection, metastasis of cancer, multiple myeloma, osteomalacia, osteoporosis, bone Paget disease, nephritis, renal failure, sepsis, septic shock, hypercalcemia, hypercholesterolemia, hypertriglyceridemia, hyperlipemia, systemic lupus erythematosus, transient ischemic attack and alcoholic hepatitis, etc., (16) for cure of organ transplantation, burns, trauma, and alopecia, etc. (17) as analgesics to suppress or relieve chronic or acute pain (e.g., postoperative pain, inflammatory pain, dental pain, bone disease (e.g., arthritis, rheumatism, osteoporosis, etc.) derived pain). Further, the compound (I) is useful for imaging of tumors having somatostatin receptors after introducing radioactive substance (e.g., ¹²³I, ¹²⁵I, ¹¹¹In, etc.) to compound (I) directly or via a suitable spacer, and for targeting tumors having somatostatin receptors after introducing anticancer drugs to compound (I) directly or via a suitable spacer.

Somatostatin is associated with secretion of hormone such as growth hormone, gastrin, glucagon (especially in the case of SSTR2), and therefore, when the compound (I) has somatostatin receptor antagonist activity, the compound can be used for the purpose of promoting secretion of these hormones. Thus, the compound (I) can be used for the prevention or treatment of diseases or symptoms caused by insufficiency of growth hormone or IGF-1.

The "prevention or treatment of diseases or symptoms caused by insufficiency of growth hormone or IGF-1" includes, for example, treatment of diabetes such as insulin-dependent (type I) and non-insulin dependent (type II) diabetes mellitus or a variety of diseases associated with them, namely diabetic complications such as diabetic retinopathy, diabetic nephropathy, diabetic neuropathy, Doan syndrome and orthostatic hypotension, etc.; prevention of adverse effects caused by disassimilation of glucocorticoid; prevention or treatment of osteoporosis; stimulation of immune system (e.g., promotion of increase in hemocytes such as lymphocyte; strengthening of an antimicrobial activity or an antiviral activity); promotion of cure of burns and trauma; acceleration in the treatment of bone fracture; treatment of acute or chronic renal diseases; treatment or improvement of diseases or symptoms (short stature, delayed growth) associated with insufficiency of growth hormone in adults or infants; treatment of obesity; promotion of recovery after surgical operations; improvement of delayed growth associated with Prader-Willi syndrome and Turner's syndrome; treatment of delayed intrauterine growth and skeletogenous disorders; treatment of peripheral neuropathy; treatment of Noonan's syndrome, schizophrenia and depression; treatment or prevention of neurodegenerative diseases such as Alzheimer's disease and Parkinson's disease; treatment of pulmonary insufficiency and ventilation dependence; treatment of malabsorption syndrome; improvement of cachexia or protein loss caused by cancer or AIDS; promotion of weight increase or proteopexis in patients in the case of TPN (total parenteral nutrition); treatment of hyperinsulinemia; promotion of induction of ovulation; improvement of menopausal disorders; improvement of senile constitution (e.g., increase in bone mass; enhancement of motor function; improvement of kidney function and cardiac function; increase in motor function and mental activity, etc.); treatment of heart diseases (e.g., cardiac muscle hypertrophy in cardiac failure, improvement of cardiac function, increase in the amount of cardiac muscle in congestive cardiomyopathy, etc.), etc.

Further, the compound (I) is useful in mammals such as domestic animals for promotion of growth; increase in milk production; strengthening of an antimicrobial or antiviral activity by stimulation of immune system; stimulation in growth of wool in sheep.

The compound (I) is especially useful as an agent for preventing or treating diabetes or diabetic complications.

As mentioned above, since the compound (I) has a selective SSTR2 binding inhibitory activity (preferably agonist activity), it is useful as an agent for preventing or treating diabetes or diabetic complications (preferably diabetic nephropathy) without side effect based on its superior glucagon secretion inhibitory activity.

Moreover, the compound (I) is superior in metabolic stability and can exhibit efficacy in a sustained manner.

The compound (I) can be used with various concomitant drugs.

Examples of the concomitant drugs include a "agents for treating diabetes", "agents for treating diabetic complications", "agents for treating obesity", "agents for treating hypertension", "agents for treating hyperlipidemia", "agents for treating arthritis", "antianxiety agents", "antidepressants", "agents for treating osteoporosis", etc. Two or more kinds of these concomitant drugs can be combined in an appropriate ratio for use.

Examples of the above "agents for treating diabetes" include insulin sensitizers, insulin secretagogues, biguanides, insulins, α-glucosidase inhibitors, β3 adrenaline receptor agonists, dipeptidylpeptidase IV inhibitors, amyrin agonist, phosphotyrosine phosphatase inhibitors, gluconeogenesis inhibitors, SGLUT (sodium-glucose cotransporter) inhibitors, etc.

Examples of the insulin sensitizers include pioglitazone or its salt (preferably hydrochloride), troglitazone, rosiglitazone or its salt (preferably maleate), JTT-501, GI-262570, MCC-555, YM-440, DRF-2593, BM-13.1258, KRP-297, R-119702, CS-011, FK-614, compounds described in WO99/58510 (e.g., (E)-4-[4-(5-methyl-2-phenyl-4-oxazolylmethoxy)benzyloxyimino]-4-phenyl butyric acid), Tesaglitazar (AZ-242), Ragaglitazar (NN-622), BMS-298585, ONO-5816, LM-4156, MBX-102, LY-519818, MX-6054, LY-510929, and the like.

Examples of the insulin secretagogues include sulfonylureas. Concrete examples of the sulfonylureas include tolbutamide, chlorpropamide, tolazamide, acetohexamide, glyclopyramide and its ammonium salt, glibenclamide, gliclazide, glimepiride, glipizide, glybuzole, and the like.

Other than the above, examples of the insulin secretagogues include repaglinide, nateglinide, mitiglinide (KAD-1229), JTT-608, and the like.

Examples of the biguanides include metformin, buformin, phenformin, and the like.

Examples of the insulins include animal insulins extracted from bovine or porcine pancreas; semi-synthetic human insulin which is enzymatically synthesized from insulin extracted from porcine pancreas; human insulin synthesized by genetic engineering, using *Escherichi Coli* or yeast. As insulin, also employed are insulin-zinc containing 0.45 to 0.9 (w/w)% of zinc; protamine-insulin-zinc produced from zinc chloride, protamine sulfate and insulin; and the like. In addition, insulin can be an insulin fragment or derivative (e.g., INS-1, etc.).

Insulin can also include various types such as ultra immediate action type, immediate action type, two-phase type, intermediate type, prolonged action type, etc., and these can be selected depending on the pathological conditions of patients.

Examples of the α-glucosidase inhibitors include acarbose, voglibose, miglitol, emiglitate, and the like.

Examples of the β3 adrenaline receptor agonists include AJ-9677, BMS-196085, SB-226552, AZ40140, CL-316243, SR-58611-A, UL-TG-307, and the like.

Examples of the dipeptidylpeptidase IV inhibitors include NVP-DPP-278, PT-100, NVP-DPP-728, LAF237, P32/98, and the like.

Examples of the amyrin agonist include pramlintide, etc.

Examples of the phosphotyrosine phosphatase inhibitors include vanadic acid, and the like.

Examples of the gluconeogenesis inhibitors include glycogen phosphorylase inhibitors, glucose-6-phosphatse inhibitors, glucagon antagonists, and the like.

Examples of the SGLUT (sodium-glucose cotransporter) inhibitors include T-1095, and the like.

Other than the above, examples of the "agents for treating diabetes" include ergoset, leptin, BAY-27-9955, GLP-1, Exendine-4, and the like.

Examples of the above "agents for treating diabetic complications" include aldose reductase inhibitors, glycation inhibitors, protein kinase C inhibitors, neurotrophic factors, neurotrophin increasing drugs, nerve regeneration stimulators, and the like.

Examples of the aldose reductase inhibitors include tolrestat; epalrestat; imirestat; zenarestat; SNK-860; zopolrestat; ARI-509; AS-3201, and the like.

Examples of the glycation inhibitors include pimagedine, ALT946, pyradoxatine, N-phenacylthiazolium bromide (ALT766), EXO-226, and the like.

Examples of the protein kinase C inhibitors include LY-333531, and the like.

Examples of the neurotrophic factors include, for example, NGF, NT-3, BDNF, and the like.

Examples of the neurotrophin increasing drugs include, for example, a neurotrophin production/secretion promoting agent (e.g., 4-(4-chlorophenyl)-2-(2-methyl-1-imidazolyl)-5-[3-(2-methylphenoxy)propyl]oxazole, etc.) described in WO01/14372, and the like.

Examples of the nerve regeneration stimulators include Y-128, VX-853, prosaptide, and the like.

Other than the above, examples of the "agents for treating diabetic complications" include alprostadil, thiapride hydrochloride, cilostazol, mexiletine hydrochloride, ethyl eicosapentate, memantine, pimagedline (ALT-711), and the like.

Examples of the above "agents for treating obesity" include pancreatic lipase inhibitors, anti-obesity drugs acting on the central nervous system, anorectic peptides, cholecystokinin agonists, and the like.

Examples of the pancreatic lipase inhibitors include orlistat, and the like.

Examples of the anti-obesity drugs acting on the central nervous system include mazindol, dexfenfluramine, fluoxetine, sibutramine, baiamine, fenfluramine, phentermine, amfepramone, dexamphetamine, phenylpropanolamine, clobenzorex, and the like.

Examples of the anorectic peptides include leptin, CNTF (Ciliary Neurotrophic Factor), and the like.

Examples of the cholecystokinin agonists include lintitript, FPL-15849, and the like.

Other than the above, examples of the "agents for treating obesity" include lipstatin, and the like.

Examples of the above "agents for treating hypertension" include angiotensin converting enzyme inhibitors, calcium antagonists, potassium channel openers, angiotensin II antagonists, and the like.

Examples of the angiotensin converting enzyme inhibitors include captopril, enarapril, alacepril, delapril (hydrochloride), lisinopril, imidapril, benazepril, cilazapril, temocapril, trandolapril, manidipine (hydrochloride), and the like.

Examples of the calcium antagonists include nifedipine, amlodipine, efonidipine, nicardipine, and the like.

Examples of the potassium channel openers include levcromakalim, L-27152, AL0671, NIP-121, and the like.

Examples of the angiotensin II antagonists include losartan, candesartan cilexetil, valsartan, irbesartan, CS-866, E4177, and the like.

Examples of the above "agents for treating hyperlipidemia" include HMG-CoA reductase inhibitors, fibrate compounds, squalene synthase inhibitors, and the like.

Examples of the HMG-CoA reductase inhibitors include pravastatin, simvastatin, lovastatin, atorvastatin, fluvastatin, lipantil, cerivastatin, itavastatin, ZD-4522, or their salts (e.g., sodium salts, etc.), and the like.

Examples of the fibrate compounds include bezafibrate, clinofibrate, clofibrate, simfibrate, and the like.

Examples of the squalene synthase inhibitors include compounds described in WO97/10224 (e.g., N-[[(3R,5S)-1-(3-acetoxy-2,2-dimethylpropyl)-7-chloro-5-(2,3-dimethoxyphenyl)-2-oxo-1,2,3,5-tetrahydro-4,1-benzooxazepin-3-yl]acetyl]piperidine-4-acetic acid, etc.), and the like.

Examples of the above "agents for treating arthritis" include ibuprofen, and the like.

Examples of the above "antianxiety agents" include chlordiazepoxide, diazepam, oxazolam, medazepam, cloxazolam, bromazepam, lorazepam, alprazolam, fludiazepam, and the like.

Examples of the above "antidepressants" include fluoxetine, fluvoxamine, imipramine, paroxetine, sertraline, and the like.

Examples of the above "agents for treating osteoporosis" include bisphosphonates, vitamin D preparations, calcitonin preparations, PTH preparations, Osten, and the like.

Other than the above, the concomitant drugs include "hormones promoting other growth hormone secretion (e.g., GHRH), GH or IGF-1", "cytokines or cytokine activity enhancing agents", and the like.

The time of administration of the above-mentioned concomitant drug are not limited, but the compound (I) and the concomitant drug can be administered simultaneously or at staggered times to the administration subject. The dose of the concomitant drug can be appropriately selected based on the dose which is clinically employed, and can be appropriately selected according to the administration subject, administration route, target disease, combination and the like.

The method for administrating concomitant drug is not limited as long as the compound (I) and the concomitant drug are combined at the time of administration. Examples of such methods include 1) administration of a single preparation prepared from the compound (I) and the concomitant drug at the same time; 2) concomitant administration of two kinds of preparations prepared from the compound (I) and the concomitant drug separately by the same administration route; 3) staggered administration of two kinds of preparations prepared from the compound (I) and the concomitant drug separately by the same administration route; 4) concomitant administration of two kinds of preparations prepared from the compound (I) and the concomitant drug separately by different administration routes; 5) staggered administration of two kinds of preparations prepared from the compound (I) and the concomitant drug separately by different administration routes (e.g., administration of the compound (I) and the concomitant drug in this order, or reverse order); and the like.

The proportion of the compound (I) and the concomitant drug can be appropriately selected according to the administration subject, administration route, target disease and the like.

In improvement of menopausal disorders, a hormone supplemental therapy (e.g., therapy by estrogen preparations, Raloxifene, Tamoxifen) can be used concomitantly.

A pharmaceutical composition of the present invention can be produced according to a method known *per se*. Said pharmaceutical composition can be produced by mixing the compound (I) and a pharmacologically acceptable carrier according to any per se known pharmaceutical manufacturing techniques.

The dosage forms of the pharmaceutical composition include, for example, tablets (including sugar-coated tablets, film-coated tablets), powders, granules, capsules (including soft capsules), liquids, injections, suppositories, sustained-release preparations (e.g., sustained-release microcapsules, etc.), etc. The compound (I) and the pharmaceutical composition of the present invention can be safely administered orally or parenterally (e.g., by local, rectal and intravenous administration, etc.).

The content of the compound (I) in a pharmaceutical composition of the present invention is 0.1 to 100 weight percent of the whole composition. The dose of the pharmaceutical composition can be appropriately selected depending on the administration subject, administration route, target disease, etc. For instance, the dose per administration when a pharmaceutical composition of the invention is orally administered as an agent for treating glaucoma to an adult patient (body weight: about 60 kg), is about 0.1 to about 500 mg, preferably about 1 to about 100 mg, more preferably about 5 to about 100 mg in terms of an effective ingredient (compound (I)). These amounts can be divided into one to several doses per day for administration.

Examples of the pharmacologically acceptable carriers used for production of a pharmaceutical composition of the present invention include various organic or inorganic carrier substances which are commonly used as materials for pharmaceutical preparations, such as excipients, lubricants, binders, and disintegrators in solid preparations; solvents, solubilizing agents, suspending agents, isotonizing agents, buffering agents, soothing agents, in liquid preparations. In addition, additives such as antiseptics, antioxidants, coloring agents, sweeteners, absorbents and moistening agents can be used, if necessary.

Examples of the excipients include lactose, sucrose, D-mannitol, starch, cornstarch, crystalline cellulose, light anhydrous silicic acid, and the like.

Examples of the lubricants include magnesium stearate, calcium stearate, talc, colloidal silica, and the like.

Examples of the binders include crystalline cellulose, sucrose, D-mannitol, dextrin, hydroxypropylcellulose, hydroxypropylmethylcellulose, polyvinylpyrrolidone, starch, saccharose, gelatin, methylcellulose, carboxymethylcellulose sodium, and the like.

Examples of the disintegrators include starch, carboxymethylcellulose, carboxymethylcellulose calcium, crosscarmellose sodium, carboxymethylstarch sodium, L-hydroxypropylcellulose, and the like.

Examples of the solvents include water for injection, alcohol, propylene glycol, macrogol, sesame oil, corn oil, and the like.

Examples of the solubilizing agents include polyethylene glycol, propylene glycol, D-mannitol, benzyl benzoate, ethanol, trisaminomethane, cholesterol, triethanolamine, sodium carbonate, sodium citrate, and the like.

Examples of the suspending agents include surfactants such as stearyltriethanolamine, sodium lauryl sulfate, lauryl amino propionic acid, lecithin, benzalkonium chloride, benzethonium chloride, glyceryl monostearate, etc.; or hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose, and the like.

Examples of the isotonizing agents include glucose, D-sorbitol, sodium chloride, glycerin, D-mannitol, and the like.

Examples of the buffering agents include buffer solutions of phosphate, acetate, carbonate and citrate, and the like.

Examples of the soothing agents include benzyl alcohol, and the like.

Examples of the antiseptics include paraoxybenzoates, chlorobutanol, benzyl alcohol, phenethyl alcohol, dehydroacetic acid, and sorbic acid, and the like.

Examples of the antioxidants include sulfite, ascorbic acid, and the like.

Examples of the coloring agents include a water-soluble edible tar pigments (e.g., edible pigments such as edible color Red No.2 and No.3, edible color Yellow No.4 and No.5, edible color Blue No.1 and No.2), a water-insoluble lake pigments (e.g., aluminum salts of the water-soluble edible tar pigments listed above), a natural pigment (e.g., β-carotene, chlorophyll, red iron oxide), and the like.

Examples of the sweeteners include saccharin sodium, dipotassium glycyrrhizinate, aspartame, stevia, and the like.

The present invention will be explained in more detail by the following Reference Examples, Examples, Formulation Example and Experimental Examples. These are not intended to restrict the present invention, and may be modified within the range of not deviating the scope of this invention.

"Room temperature" in the following Reference Examples and Examples means a temperature of 0°C to 30°C. For drying an organic layer, anhydrous magnesium sulfate or anhydrous sodium sulfate was employed. Unless otherwise specifically indicated, "%" means percent by weight. The solvent ratio when a mixed solvent is used is a volume ratio.

The IR absorption spectra were measured in a diffused reflection method using a Fourier transform infrared spectrophotometer.

The mass spectrum was measured by FAB or APCI.

The meanings of the abbreviations used in the present specification are as follows:
s: singlet
d: doublet
dd: double doublet
dt: double triplet
t: triplet
q: quartet
sep: septet
m: multiplet
br: broad
J: coupling constant
Hz: Hertz
CDCl₃: deuterated chloroform
DMSO-d₆: deuterated dimethylsulfoxide
THF: tetrahydrofuran
DMF: N,N-dimethylformamide
DMSO: dimethylsulfoxide
WSC: 1-ethyl-3-(3-dimethylaminopropyl)carbodiimide hydrochloride
¹H-NMR: proton nuclear magnetic resonance spectrum (generally measured as the free form of each sample in CDCl₃)
IR: infrared absorption spectrum
Me: methyl
Et: Ethyl
HOBt: 1-hydroxy-1H-benzotriazol
HOAt:1-hydroxy-7-azabenzotriazole
IPE: diisopropyl ether

### Examples

### Reference Example 1

### N-[1-(1H-indol-3-yl)ethyl]-N-isopropylpropan-2-amine

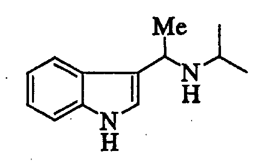

To a solution of indole (50 g) in acetic acid (200 mL) was added a solution of isopropylamine (27.8 g) and 90% acetaldehyde (21.9 g) in toluene (50 mL) under ice-cooling. The mixture was stirred for 2 hrs. and left standing in a refrigerator for 3 days. To the reaction solution were added ice water (500 mL) and ethyl acetate (70 mL), and the mixture was partitioned. The organic layer was extracted with water (70 mL). The extract was combined with the aqueous layer. The mixture was adjusted to pH 10 - 11 with 8 N aqueous sodium hydroxide solution under ice-cooling and extracted with ethyl acetate (300 mL). The aqueous layer was extracted with ethyl acetate (100 mL) and the extract was combined with the organic layer. The mixture was washed with water (200 mL), dried over magnesium sulfate and concentrated under reduced pressure. The obtained residue was washed with IPE to give the title compound (55.8 g, yield 64.6%).
¹H-NMR (CDCl₃) δ 1.07 (3H, d, J = 6.4 Hz), 1.08 (3H, d, J = 5.8 Hz), 1.51 (3H, d, J = 6.6 Hz), 1.64 (1H, brs), 2.87 (1H, sep, J = 6.2 Hz), 4.26 (1H, q, J = 6.6 Hz), 7.07 - 7.23 (3H, m), 7.37 (1H, dd, J = 1.4, 7.8 Hz), 7.71 (1H, dd, J = 1.2, 7.2 Hz), 8.15 (1H, brs).
IR (KBr) 3414, 2967, 1456, 741 cm⁻¹

### Reference Example 2

### methyl (2RS,3SR)-3-(1H-indol-3-yl)-2-nitrobutanoate

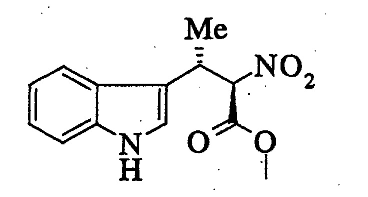

To a solution of N-[1-(1H-indol-3-yl)ethyl]-N-isopropylpropan-2-amine (20.23 g) in toluene (200 mL) were added methyl nitroacetate (11.91 g) and triethylamine (13.94 mL) at room temperature, and the mixture was stirred for 30 min and heated under reflux for 3 hrs. After cooling, the reaction solution was washed successively with 1N hydrochloric acid (200 mL x 3) and then water (200 mL x 2). The mixture was dried over magnesium sulfate and concentrated under reduced pressure to give a Syn/Anti mixture. To the obtained residue was added ethanol (50 mL), and the mixture was stirred at room temperature to allow precipitation of crystals. To the precipitated crystals was added hexane (100 mL), and the crystals were collected by filtration (17.68 g, yield 33.7%). The mother liquor was purified by repeating silica gel column chromatography (developing solvent; ethyl acetate/hexane=1:4-1:2) and recrystallization to give the title compound (14.09 g, yield 26.9%).
¹H-NMR (CDCl₃) δ 1.55 (3H, d, J = 6.8 Hz), 3.56 (3H, s), 4.18 (1H, dq, J = 8.8, 6.8 Hz), 5.44 (1H, d, J = 8.8 Hz), 7.10 (1H, d, J = 2.6 Hz), 7.15 - 7.23 (2H, m), 7.38 (1H, dd, J = 1.2, 7.8 Hz), 7.63 (1H, dd, J = 1.4, 7.8 Hz), 8.14 (1H, brs).
IR (KBr) 3418, 1752, 1559, 1458, 745 cm⁻¹

### Reference Example 3

### methyl (2RS,3SR)-2-amino-3-(1H-indol-3-yl)butanoate

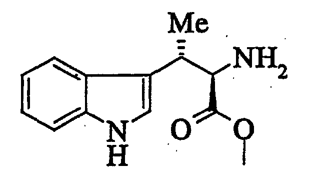

To a solution of methyl (2RS,3SR)-3-(1H-indol-3-yl)-2-nitrobutanoate (15.0 g) and trifluoroacetic acid (17.6 mL) in ethanol (300 mL) was added 10% palladium-carbon (3.0 g) under hydrogen atmosphere, and the mixture was stirred with heating at 50°C for 6 hrs. After cooling, insoluble materials were filtered off and the solvent was evaporated under reduced pressure. To the residue was added ethyl acetate (250 mL)-aqueous potassium carbonate solution (171 mmol in 150 mL) for partitioning. The organic layer was washed with 10% aqueous ammonium chloride solution (100 mL) and saturated brine (100 mL), dried over magnesium sulfate and concentrated. The obtained residue was washed with ethyl acetate/IPE to give the title compound (12.0 g, yield 90.6%).
¹H-NMR (CDCl₃) δ 1.32 (3H, d, J = 7.4 Hz), 1.50 (2H, brs), 3.68 (1H, m), 3.74 (3H, s), 3.94 (1H, d, J = 4.0 Hz), 7.06 (1H, d, J = 1.8 Hz), 7.17 (2H, ddd, J = 1.2, 7.4, 8.4 Hz), 7.36 (1H, dd,J = 1.2, 8.4 Hz), 7.68 (1H, d, J = 7.4 Hz), 8.20 (1H, brs).
IR(KBr) 2969, 1734, 1485, 1229, 743 cm⁻¹

### Reference Example 4

### methyl (2R,3S)-2-amino-3-(1H-indol-3-yl)butanoate

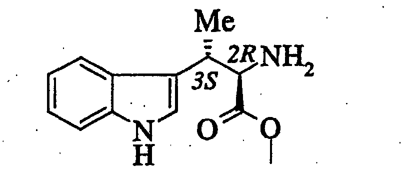

Methyl (2RS,3SR)-2-amino-3-(1H-indol-3-yl)butanoate (19.2 g) obtained in Reference Example 3 was fractionated by HPLC to give the title compound (8.19 g).
Fractionation conditions
column: CHRALCEL OD 50 mmID X 500 mmL
mobile phase: hexane/ethanol/trifluoroacetic acid=93/7/0.1
flow rate: 60 mL/min
temperature: 25°C
detection: UV 254 nm

| Elemental analysis for C₁₃H₁₆N₂O₂·CF₃COOH·0.5H₂O | | | |
|---|---|---|---|
| Calcd; | C, 50.70; | H, 5.11; | N, 7.88. |
| Found; | C, 50.91; | H, 5.18; | N, 7.65. |

### Reference Example 5

### methyl (2S,3R)-2-amino-3-(1H-indol-3-yl)butanoate

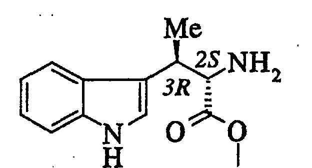

In the same manner as in Reference Example 4, methyl (2RS,3SR)-2-amino-3-(1H-indol-3-yl)butanoate (19.2 g) was fractionated by HPLC to give the title compound (8.60 g).

| Elemental analysis for C₁₃H₁₆N₂O₂·CF₃COOH·0.4H₂O | | | |
|---|---|---|---|
| Calcd; | C, 50.96; | H, 5.08; | N, 7.92. |
| Found; | C, 51.15; | H, 5.26; | N, 7.54. |

### Reference Example 6

### methyl (2RS,3SR)-2-{[(9H-fluoren-9-ylmethoxy)carbonyl]amino}-3-(1H-indol-3-yl)butanoate

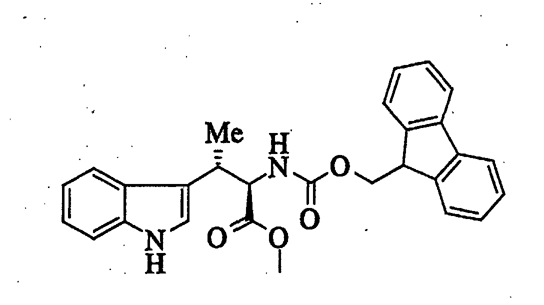

To a solution of methyl (2RS,3SR)-2-amino-3-(1H-indol-3-yl)butanoate (2.32 g) and triethylamine (1.01 g) in THF (20 mL) was added a solution of 9-fluorenylmethylchloroformate (2.59 g) in THF (10 mL) at 0°C (salt precipitated). After stirring for 10 min., the reaction solution was added to water and extracted with ethyl acetate. The organic layer was washed with saturated brine, dried over magnesium sulfate and concentrated. The residue was purified by silica gel column chromatography (developing solvent; ethyl acetate/hexane = 1:4 - 1:1) to give the title compound (4.6 g, yield 100%) as an amorphous powder.
¹H-NMR (CDCl₃) δ 1.46 (3H, d, J = 7.4 Hz), 3.62 (3H, s), 3.65 (1H, m), 4.10 - 4.27 (1H, m), 4.32 - 4.39 (1H, m), 4.68 (1H, dd, J = 5.6, 9.4 Hz), 5.36 (1H, d, J = 9.4 Hz), 7.02 (1H, d, J = 2.6 Hz), 7.09 - 7.78 (13H, m), 8.05 (1H, s).

The compounds described in the following Reference Examples 7-8 were synthesized in the similar manner as in Reference Example 6.

### Reference Example 7

### methyl (2R,3S)-2-{[(9H-fluoren-9-ylmethoxy)carbonyl]amino}-3-(1H-indol-3-yl)butanoate

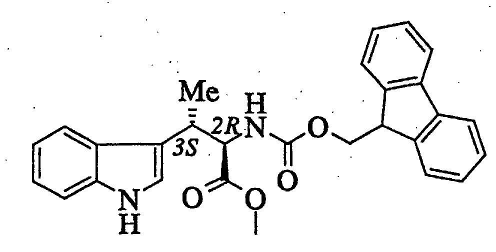

MASS (APCIMASS), m/z 455 [(M+H)⁺].

### Reference Example 8

### methyl (2S,3R)-2-{[(9H-fluoren-9-ylmethoxy)carbonyl]amino}-3-(1H-indol-3-yl)butanoate

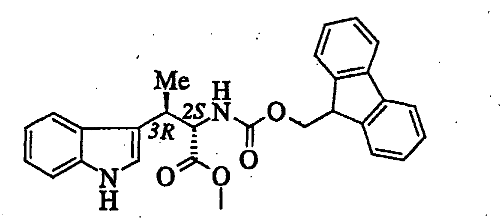

MASS (APCIMASS), m/z 455 [(M+H)⁺].

### Reference Example 9

### (2RS,3SR)-2-{[(9H-fluoren-9-ylmethoxy)carbonyl]amino}-3-(1H-indol-3-yl)butanoic acid

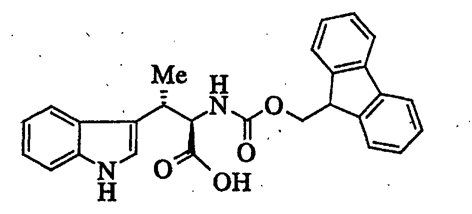

To a solution of methyl (2RS,3SR)-2-{[(9H-fluoren-9-ylmethoxy)carbonyl]amino}-3-(1H-indol-3-yl)butanoate (2.9 g) in dioxane (100 mL) was added 6N hydrochloric acid solution (10 mL), and the mixture was heated to 110°C under reflux for 40 hrs. After cooling, the reaction solution was added to water and extracted with ethyl acetate. The organic layer was washed with saturated brine, dried over magnesium sulfate and concentrated. The residue was purified by silica gel column chromatography (developing solvent; ethyl acetate/hexane = 1:1 - ethyl acetate/ethanol = 20:1) to give the title compound (2.9 g, yield 100%) as an amorphous powder.
¹H-NMR (CDCl₃) δ 1.47 (3H, d, J = 7.0 Hz), 3.6 (1H, m), 4.2 (1H, m), 4.4 (1H, m), 4.8 (1H, m), 5.4 (1H, m), 6.97 (1H, m), 7.15 - 7.77 (13H, m), 8.0 (1H, s).

The compounds described in the following Reference Examples 10-11 were synthesized in the similar manner as in Reference Example 9.

### Reference Example 10

### (2R,3S)-2-{[(9H-fluoren-9-ylmethoxy)carbonyl]amino}-3-(1H-indol-3-yl)butanoic acid

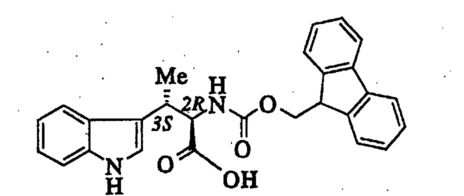

MASS (APCIMASS), m/z 441 [(M+H)⁺].

### Reference Example 11

### (2S,3R)-2-{[(9H-fluoren-9-ylmethoxy)carbonyl]amino}-3-(1H-indol-3-yl)butanoic acid

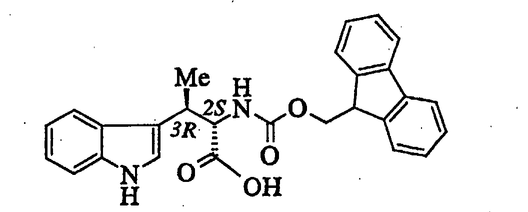

MASS (APCIMASS), m/z 441 [(M+H)⁺].

### Reference Example 12

### tert-butyl 4-(3-methylphenoxy)-1-piperidinecarboxylate

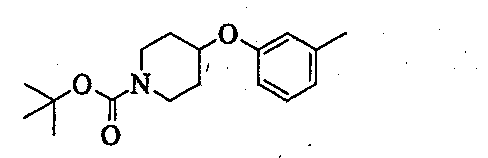

To a suspension of sodium hydride, 60% oily substance (0.8 g) in N,N-dimethylformamide (40 mL) was added 3-methylphenol (2.16 g) at room temperature, and the mixture was stirred for 15 min. To the reaction mixture was added tert-butyl 4-[(methylsulfonyl)oxy]-1-piperidinecarboxylate (6.15 g) at room temperature and stirred with heating at 80°C for 810 hrs. After cooling, the reaction mixture was poured into water and extracted with ethyl acetate. The organic layer was washed with water and dried. Then, the solvent was evaporated and the residue was purified by aminopropyl silica gel column chromatography (developing solvent; hexane/ethyl acetate = 30:1 - 10:1) to give the title compound (1.07 g, yield 18%).
¹H-NMR (CDCl₃) δ: 1.47 (9H, s), 1.67 - 1.98 (4H, m), 2.32 (3H, s), 3.27 - 3.39 (2H, m), 3.64 - 3.76 (2H, m), 4. 49 (1H, sept, J = 3.6 Hz), 6.70 - 6.79 (2H, m), 7.12 - 7.20 (2H, m).

The compound described in the following Reference Example 13 was synthesized in the similar manner as in Reference Example 12.

### Reference Example 13

### tert-butyl 4-[4-(aminosulfonyl)phenoxy]-1-piperidinecarboxylate

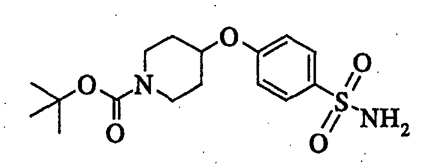

¹H-NMR (CDCl₃)δ:1.47 (9H, s), 1.72 - 1.95 (4H, m), 3.31 - 3.43 (2H, m), 3.62 - 3.75 (2H, m), 4.57 (1H, sept, J = 3.4 Hz), 4.92 (2H, s), 6.98 (2H, dd, J = 2.0, 6.9 Hz), 7.86 (2H, dd, J = 2.1, 6.9 Hz).

### Reference Example 14

### tert-butyl 4-[4-(benzyloxy)phenoxy]-1-piperidinecarboxylate

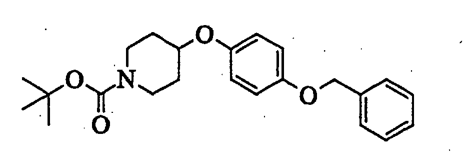

To a solution of 4-(benzyloxy)phenol (6.01 g), tert-butyl 4-(methylsulfonyl)oxy]-1-piperidinecarboxylate (7.25 g) and triphenylphosphine (11.8 g) in THF (100 mL) was added a solution of 40% diethylazodicarboxylate (17.0 g) in toluene at room temperature, and the mixture was stirred for 12 hrs. The reaction mixture was poured into saturated aqueous sodium hydrogen carbonate and extracted with ethyl acetate. The organic layer was washed with 2 N aqueous sodium hydroxide solution and dried. The solvent was evaporated and the residue was purified by silica gel column chromatography (developing solvent; hexane/ethyl acetate = 4:1 - 2:1 - 1:1) to give the title compound (6.83 g, yield 59%).
¹H-NMR (CDCl₃) δ: 1.47 (9H, s), 1.60 - 1.96 (4H, m), 3.22-3.35 (2H, m), 3. 65 - 3.76 (2H, m), 4.32 (1H, sept, J = 3.6 Hz), 5.01 (2H, s), 6.73 - 6.93 (4H, m), 7.31 - 7.42 (5H, m),

### Reference Example 15

### tert-butyl 4-[3-(benzoyloxy)phenoxy]-1-piperidinecarboxylate

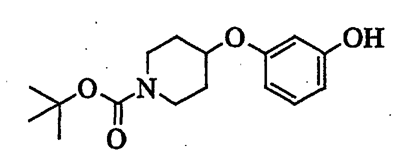

To a solution of 3-(benzoyloxy)phenol (4.28 g), tert-butyl 4-[(methylsulfonyl)oxy]-1-piperidinecarboxylate (4.83 g) and triphenylphosphine (7.87 g) in THF (75 mL) were added a solution of 40% diethylazodicarboxylate (11.3 g) in toluene at room temperature, and the mixture was stirred for 12 hrs. The reaction mixture was poured into saturated aqueous sodium hydrogen carbonate and extracted with ethyl acetate. The organic layer was washed with 2 N aqueous sodium hydroxide solution and dried. The solvent was evaporated and the residue was purified by silica gel column chromatography (developing solvent; hexane/ethyl acetate = 10:1 - 5:1 - 2:1).

To a solution of the obtained residue in methanol (35 mL) was added 2 N aqueous sodium hydroxide solution (10 mL) at room temperature and the mixture was stirred for 10 hrs. The reaction mixture was neutralized with 1N hydrochloric acid (20 mL) and extracted with ethyl acetate. The organic layer was dried and the solvent was evaporated to give the title compound (1.80 g, yield 37%).
¹H-NMR (DMSO-d₆) δ: 1.41 (9H, s), 1. 42 - 1.59 (2H, m), 1.80-1.91 (2H, m), 3.12 - 3.24 (2H, m), 3.58 - 3.71 (2H, m), 4.45 (1H, sept, J = 4.2 Hz), 6.32 - 6.39 (3H, m), 6.98 - 7.07 (1H, m), 9.31 (1H, s).

### Reference Example 16

### tert-butyl 4-[4-(1H-tetrazol-5-yl)phenoxy]-1-piperidinecarboxylate

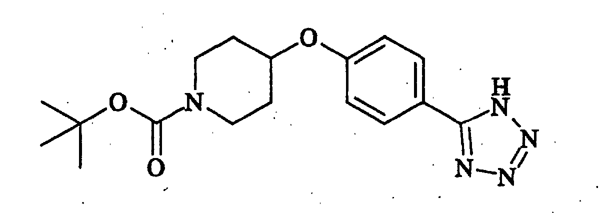

To a solution of tert-butyl 4-(4-cyanophenoxy)-1-piperidinecarboxylate (3.02 g) and sodium azide (1.30 g) in N,N-dimethylformamide (30 mL) was added ammonium chloride (1.07 g) at room temperature, and the mixture was stirred at 125°C for 2 days. After cooling, the reaction mixture was poured into water and adjusted to pH 5 with 1N hydrochloric acid. The resulting precipitates were collected by filtration and washed with ethyl acetate/hexane and dried to give the title compound (3.03 g, yield 87%).
¹H-NMR (CDCl₃) δ: 1.51 (9H, s), 1. 71 - 2.00 (4H, m) , 3.36-3.48 (2H, m), 3.64 - 3.75 (2H, m), 4.50 - 4.62 (1H, m), 6.32-6.39 (3H, m), 7.01 (2H, d, J = 8.8 Hz), 8.09 (2H, d, J = 8.4 Hz).

### Reference Example 17

### 4-(3-methylphenoxy)piperidine·hydrochloride

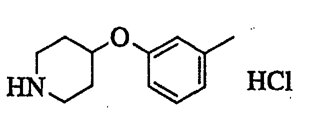

To a solution of tert-butyl 4-(3-methylphenoxy)-1-piperidinecarboxylate (1.07 g) in methanol (15 mL) was added concentrated hydrochloric acid (1.0 mL) at room temperature, and the mixture was stirred at 60°C for 1.5 hrs. After cooling, the solvent was evaporated. The obtained residue was washed with methanol/diethyl ether and dried to give the title compound (0.74 g, yield 88%).
¹H-NMR (DMSO-d₆)δ: 1.78 - 1.90 (2H, m), 2. 06 - 2.13 (2H, m), 2.27 (3H, s), 3.00 - 3.10 (2H, m), 3.18 - 3.24 (2H, m), 4.62 (1H, sept, J = 2.4 Hz), 6.75 - 6.81 (3H, m), 7.16 (1H, t, J = 5.4 Hz), 9.09 (2H, s).

The compounds described in the following Reference Examples 18-21 were synthesized in the similar manner as in Reference Example 17.

### Reference Example 18

### 3-(4-piperidinyloxy)phenol·hydrochloride

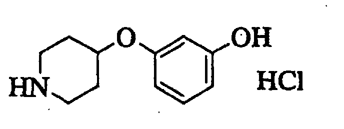

¹H-NMR (DMSO-d₆)δ: 1.72 - 1.91 (2H, m), 2.00 - 2.14 (2H, m), 3.04 - 3.20 (4H, m), 4.56 (1H, sept, J = 3.2 Hz), 6.37 - 6.43 (3H, m), 7.05 (1H, t, J = 8.6 Hz), 8.98 (2H, s), 9.44 (1H, s).

### Reference Example 19

### 4-(4-piperidinyloxy)benzenesulfonamide·hydrochloride

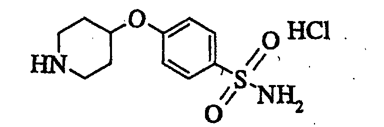

¹H-NMR (DMSO-d₆)δ: 1.80 - 1.96 (2H, m) , 2.08 - 2.20 (2H, m) , 3.02 - 3.30 (4H, m), 4.78 (1H, sept, J = 3.6 Hz), 7.16 (2H, d, J = 8.9 Hz), 7.25 (2H, s), 7.76 (2H, d, J = 8.8 Hz), 9.21 (2H, s).

### Reference Example 20

### 4-[4-(1H-tetrazol-5-yl)phenoxy]piperidine·hydrochloride

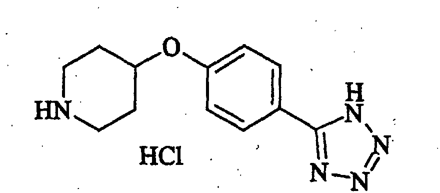

¹H-NMR (DMSO-d₆)δ: 1.82 - 2.00 (2H, m) , 2.14 - 2.23 (2H, m) , 3.10 - 3.25 (4H, m), 4.81 (1H, sept, J = 3.8 Hz), 7.22 (2H, d, J = 8.8 Hz), 8.05 (2H, d, J = 8.4 Hz), 9.09 (2H, s).

### Reference Example 21

### 4-[4-(benzyloxy)phenoxy]piperidine·hydrochloride

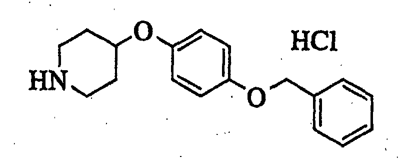

¹H-NMR (DMSO-d₆)δ: 1.74 - 1.92 (2H, m), 2.03 - 2.15 (2H, m), 2.99 - 3.11 (2H, m), 3.14 - 3.34 (2H, m), 4.51 (1H, sept, J = 3.4 Hz), 5.03 (2H, s), 6.93 (4H, s), 7.30 - 7.46 (5H, m), 9.08 (2H, s).

### Reference Example 22

### 4-(4-piperidinyloxy)phenol·hydrochloride

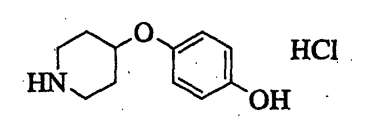

To a solution of 4-[4-(benzyloxy)phenoxy]piperidine-hydrochloride (3.90 g) in methanol (40 mL) was added 10% palladium-carbon (0.4 g) at room temperature, and the mixture was stirred under hydrogen atmosphere at room temperature for 16 hrs. After filtering off insoluble materials, the solvent was evaporated. The obtained residue was washed with methanol/diethyl ether and dried to give the title compound (2.47 g, yield 88%).
¹H-NMR (DMSO-d₆)δ: 1.74 - 1.88 (2H, m), 2.02 - 2.10 (2H, m) , 2.99 - 3.08 (2H, m), 3.16 - 3.24 (2H, m), 4.42 (1H, sept, J = 2.2 Hz), 6.67 - 6.71 (2H, m), 6.78 - 6.82 (2H, m), 9.02 (3H, s).

### Example 1

### (2RS,3SR)-1-((3R)-6-chloro-3-[(dimethylamino)methyl]-3,4-dihydro-1(2H)-quinolinyl)-3-(1H-indol-3-yl)-1-oxo-2-butanamine

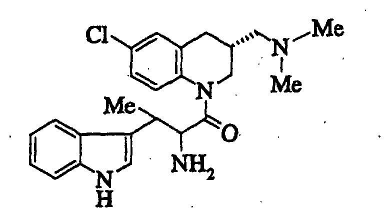

To a solution of (2RS,3SR)-2-{[(9H-fluoren-9-ylmethoxy)carbonyl]amino}-3-(1H-indol-3-yl)butanoic acid (2.6 g) and DMF (0.06 mL) in THF (60 mL) was added dropwise a solution of oxalyl chloride (0.63 mL) in THF (5 mL) at 0°C. The reaction solution was stirred at 0°C for 30 min. and concentrated. Then, THF (30 mL) was added to the residue and the mixture was concentrated again. The residue was dissolved in THF (30 mL) and added dropwise to a solution of 1-[(3S)-6-chloro-1,2,3,4-tetrahydroquinolin-3-yl]-N,N-dimethylmethanamine (0.90 g), tetrabutylammonium hydrogensulfate (0.04 g) and sodium hydroxide (powder, 0.34 g) in THF (10 mL) at 0°C. The solution was stirred at 0°C for 30 min and then further stirred at room temperature for 2 hrs. The reaction solution was added to ice water (100 mL) and extracted with ethyl acetate (100 mL x 2). The organic layer was washed with water (100 mL x 2) and saturated brine (100 mL), dried over magnesium sulfate and concentrated. The residue was purified by alumina column chromatography (developing solvent; ethyl acetate) and silica gel column chromatography (developing solvent; ethyl acetate, ethyl acetate-methanol = 20:1 - 10:1). The obtained pale yellow amorphous powder was dissolved in methanol (20 mL), and piperidine (0.2 mL) was added thereto and stirred at room temperature for 16 hrs. The reaction solution was concentrated and the residue was purified by alumina column chromatography (developing solvent; ethyl acetate/hexane = 1:1 - ethyl acetate/ethanol = 20:1) to give the title compound (0.83 g, yield 49%) as an amorphous powder.
IR (KBr) 2971, 1644, 1487, 743 cm⁻¹
MASS (FABMASS), m/z 425 [(M+H)⁺].

The compounds described in the following Examples 2-4 were synthesized in the similar manner as in Example 1.

### Example 2

### (2S,3R)-1-((3R)-6-chloro-3-[(dimethylamino)methyl]-3,4-dihydro-1(2H)-quinolinyl)-3-(1H-indol-3-yl)-1-oxo-2-butanamine

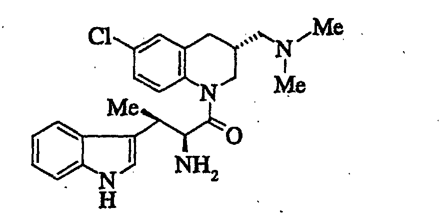

IR(KBr) 2969, 1645, 1487, 741 cm⁻¹

### Example 3

### (2R,3S)-1-((3R)-6-chloro-3-[(dimethylamino)methyl]-3,4-dihydro-1(2H)-quinolinyl)-3-(1H-indol-3-yl)-1-oxo-2-butanamine

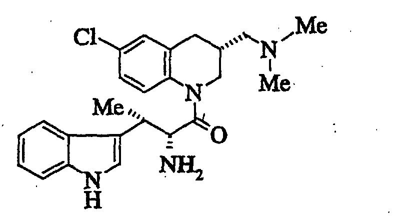

IR(KBr) 2971, 1645, 1487, 741 cm⁻¹

### Example 4

### (2RS,3SR)-1-((3S)-6-chloro-3-[(dimethylamino)methyl]-3,4-dihydro-1(2H)-quinolinyl)-3-(1H-indol-3-yl)-1-oxo-2-butanamine

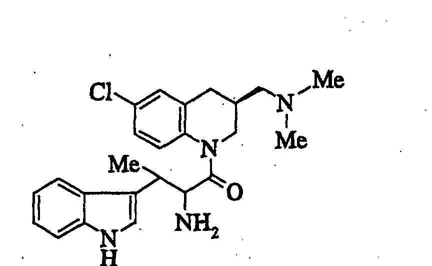

IR(KBr) 2971, 1651, 1487, 741 cm⁻¹

### Example 5

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-1-[(1-methyl-1H-indol-2-yl)carbonyl]-4-piperidinecarboxamide

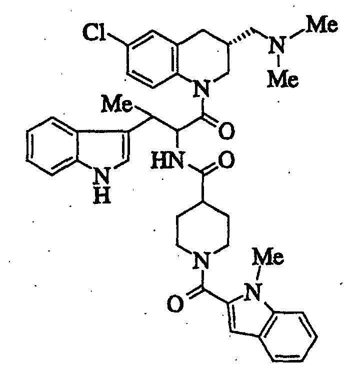

To a solution of (2RS,3SR)-1-((3R)-6-chloro-3-[(dimethylamino)methyl]-3,4-dihydro-1(2H)-quinolinyl)-3-(1H-indol-3-yl)-1-oxo-2-butanamine (0.20 g), 1-[(1-methyl-1H-indol-2-yl)carbonyl]-4-piperidinecarboxylic acid (0.15 g) and HOBt (0.08 g) in acetonitrile (10 mL) was added WSC (0.10 g), and the mixture was stirred at room temperature for 16 hrs. The reaction solution was poured into 10 % sodium carbonate solution (50 mL) and extracted with ethyl acetate (50 mL x 2). The extract was washed with water (50 mL) and saturated brine (50 mL), and dried (MgSO₄). The solvent was evaporated under reduced pressure and the residue was purified by alumina column chromatography (developing solvent; ethyl acetate/hexane = 1:1 - ethyl acetate/ethanol = 20:1) to give a pale yellow amorphous powder. The obtained powder was washed with diisopropyl ether to give the title compound as a pale yellow amorphous powder (0.21 g, yield 64%).
IR(KBr) 3287, 2940, 1628, 741 cm⁻¹
MASS (FABMASS), m/z 693 [(M+H)⁺].

The compounds described in the following Examples 6-8 were synthesized in the similar manner as in Example 5.

### Example 6

### N-[(1R,2S)-1-[((3R)-6-chloro-3-[(dimethylamino)methyl]-3,4-dihydro-1(2H)-quinolinyl)carbonyl]-2-(1H-indol-3-yl)propyl]-1-[(1-methyl-1H-indol-2-yl)carbonyl]-4-piperidinecarboxamide

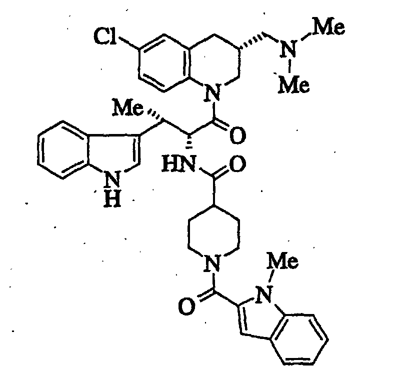

IR(KBr) 3297, 2940, 1624, 1489, 1458, 741 cm⁻¹

### Example 7

### N-[(1S,2R)-1-[((3R)-6-chloro-3-[(dimethylamino)methyl]-3,4-dihydro-1(2H)-quinolinyl)carbonyl]-2-(1H-indol-3-yl)propyl]-1-[(1-methyl-1H-indol-2-yl)carbonyl]-4-piperidinecarboxamide

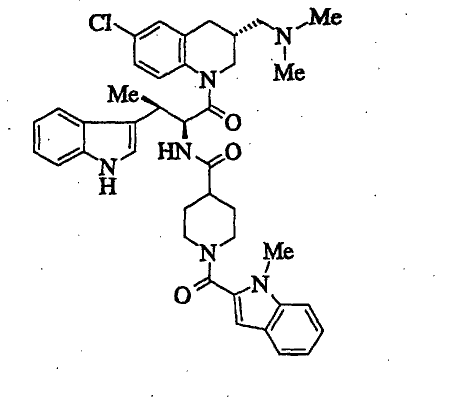

IR(KBr) 2938, 1624, 1489, 1458, 741 cm⁻¹

### Example 8

### N-[(1RS,2SR)-1-[((3S)-6-chloro-3-[(dimethylamino)methyl]-3,4-dihydro-1(2H)-quinolinyl)carbonyl]-2-(1H-indol-3-yl)propyl]-1-[(1-methyl-1H-indol-2-yl)carbonyl]-4-piperidinecarboxamide

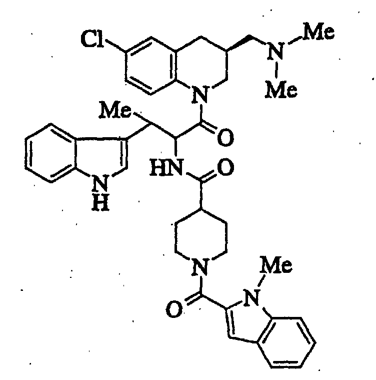

IR(KBr) 3303, 2942, 1628, 1487, 741 cm⁻¹

### Example 9

### 2,2-dichloro-N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]acetamide trifluoroacetate

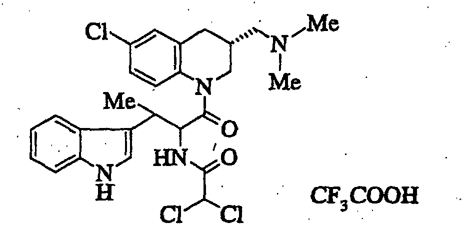

To a solution of (2RS,3SR)-1-((3R)-6-chloro-3-[(dimethylamino)methyl]-3,4-dihydro-1(2H)-quinolinyl)-3-(1H-indol-3-yl)-1-oxo-2-butanamine (30 mg), 2,2-dichloroacetic acid (11 mg) and HOAt (11 mg) in dichloromethane (2 mL) was added WSC (13 mg), and the mixture was stirred at room temperature for 16 hrs. The solvent was evaporated under reduced pressure. Then, to the residue were added water and dichloromethane and the mixture was vigorously shaken. After the mixture was left standing, the organic layer was separated, and the solvent was evaporated under reduced pressure. The residue was purified by HPLC (mobile phase; water (containing 0.03% trifluoroacetic acid)/acetonitrile(containing 0.03% trifluoroacetic acid) = 9:1 - 1:9) to give the title compound as a colorless amorphous powder (22 mg, yield 48%).
MASS (APCIMASS), m/z 535 [(M+H)⁺].

The compounds described in the following Examples 10-40 were synthesized in the similar manner as in Example 9.

### Example 10

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-2,2-difluoroacetamide trifluoroacetate

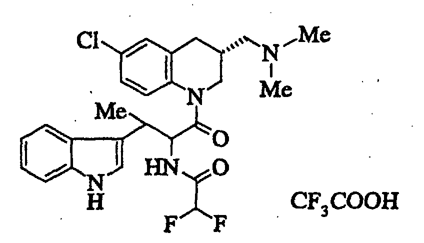

MASS (APCIMASS), m/z 503 [(M+H)⁺].

### Example 11

### (2S)-N-[(1RS,2SR)-1-[((3R)-chloro-3-[(dimethylamino)methyl]-3,4-dihydro-1(2H)-quinolinyl)carbonyl]-2-(1H-indol-3-yl)propyl]-2-hydroxy-2-phenylethanamide trifluoroacetate

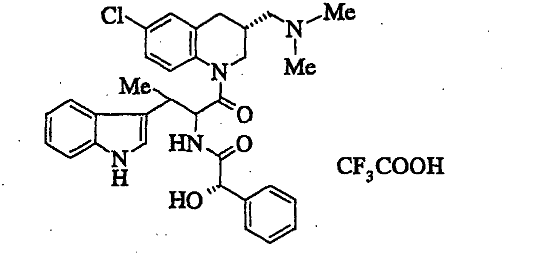

MASS (APCIMASS), m/z 559 [(M+H)⁺].

### Example 12

### 2-(acetylamino)-N-[(1RS,2SR)-1-[((3R)-6-chloro-3-[(dimethylamino)methyl]-3,4-dihydro-1(2H)-quinolinyl)carbonyl]-2-(1H-indol-2-yl)propylacetamide trifluoroacetate

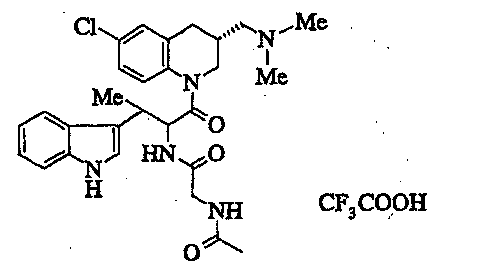

MASS (APCIMASS), m/z 524 [(M+H)⁺].

### Example 13

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]pentanamide trifluoroacetate

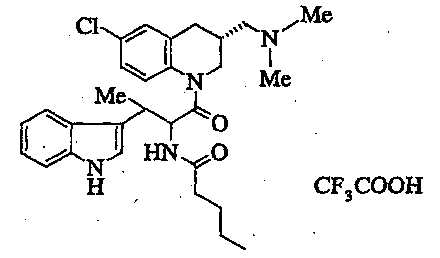

MASS (APCIMASS), m/z 509 [(M+H)⁺].

### Example 14

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-methoxybenzamide trifluoroacetate

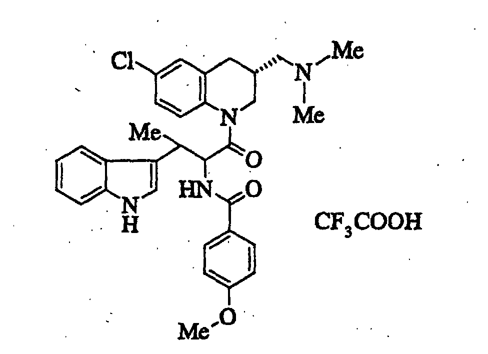

MASS (APCIMASS), m/z 559 [(M+H)⁺].

### Example 15

### N-[(1RS,2SR)-2-[((3R)-6-chloro-3-[(dimethylamino)methyl]-3,4-dihydro-1(2H)-quinolinyl)carbonyl]-2-(1H-indol-3-yl)propyl]-2-ethoxyacetamide trifluoroacetate

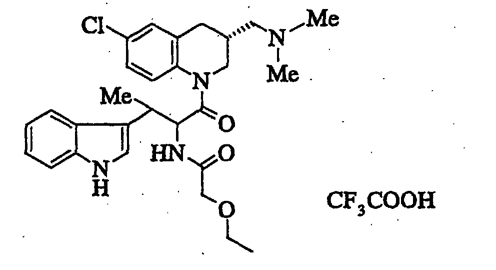

MASS (APCIMASS), m/z 511 [(M+H)⁺].

### Example 16

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-3,3-dimethylbutanamide trifluoroacetate

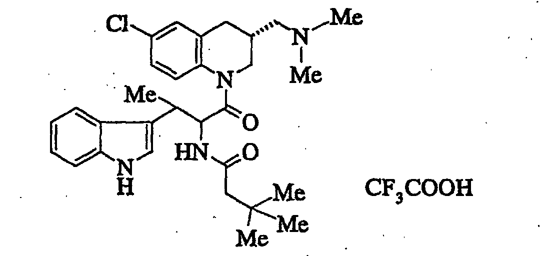

MASS (APCIMASS), m/z 523 [(M+H)⁺].

### Example 17

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-5-methylhexanamide trifluoroacetate

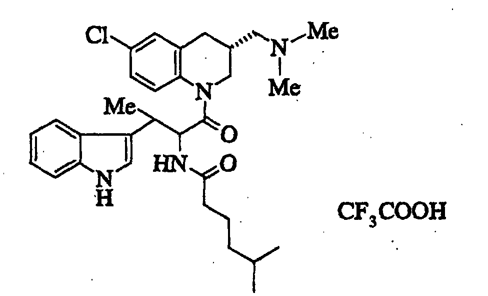

MASS (APCIMASS), m/z 537 [(M+H)⁺].

### Example 18

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-2-ethylbutanamide trifluoroacetate

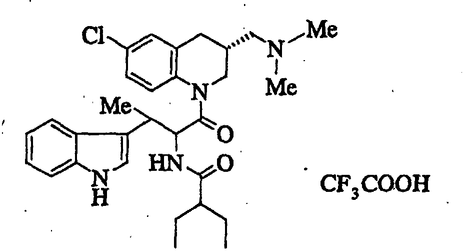

MASS (APCIMASS), m/z 523 [(M+H)⁺].

### Example 19

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-3-methylpentanamide trifluoroacetate

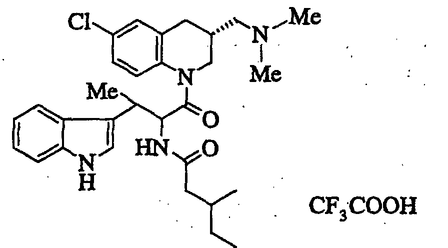

MASS (APCIMASS), m/z 523 [(M+H)⁺].

### Example 20

### 2-butoxy-N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]acetamide trifluoroacetate

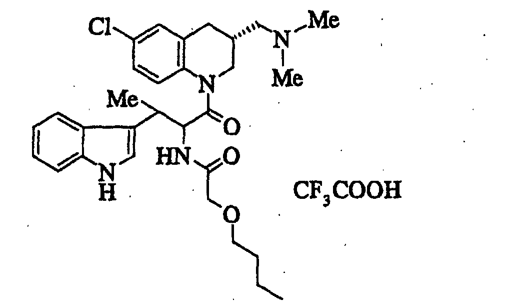

MASS (APCIMASS), m/z 539 [(M+H)⁺].

### Example 21

### (2R)-N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-5-oxo-2-pyrrolidinecarboxamide trifluoroacetate

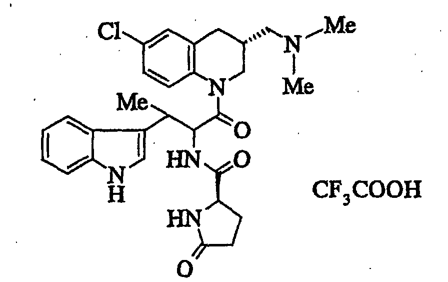

MASS (APCIMASS), m/z 536 [(M+H)⁺].

### Example 22

### (2S)-N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-oxo-2-azetidinecarboxamide trifluoroacetate

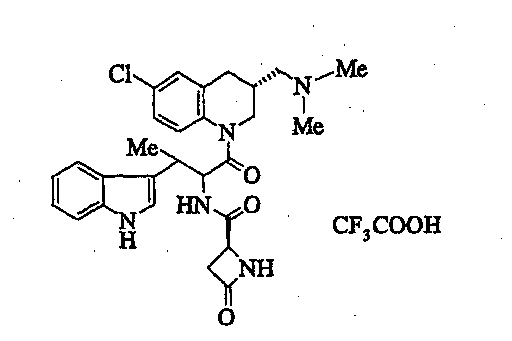

MASS (APCIMASS), m/z 522 [(M+H)⁺].

### Example 23

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-3,3,3-trifluoropropanamide trifluoroacetate

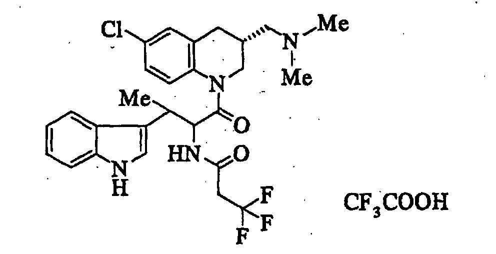

MASS (APCIMASS), m/z 535 [(M+H)⁺].

### Example 24

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-1H-pyrrole-2-carboxamide trifluoroacetate

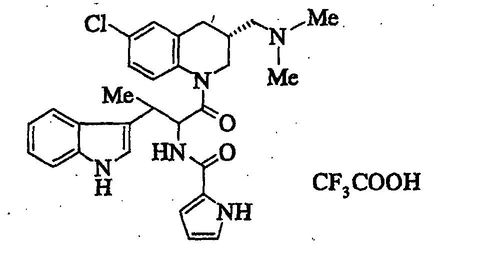

MASS (APCIMASS), m/z 518 [(M+H)⁺].

### Example 25

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-2-fluorobenzamide trifluoroacetate

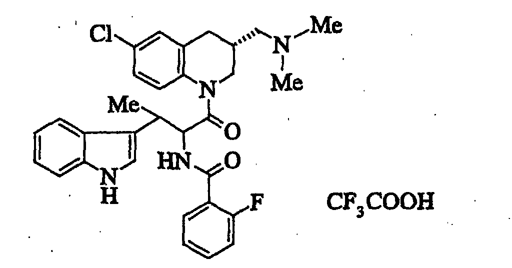

MASS (APCIMASS), m/z 547 [(M+H)⁺].

### Example 26

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-2,6-difluorobenzamide trifluoroacetate

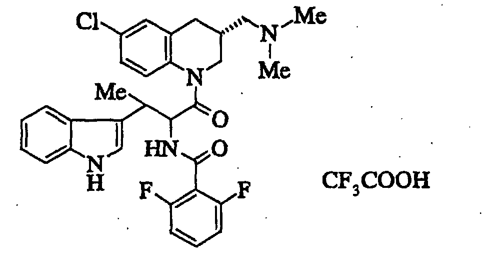

MASS (APCIMASS), m/z 565 [(M+H)⁺].

### Example 27

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-3-fluorobenzamide trifluoroacetate

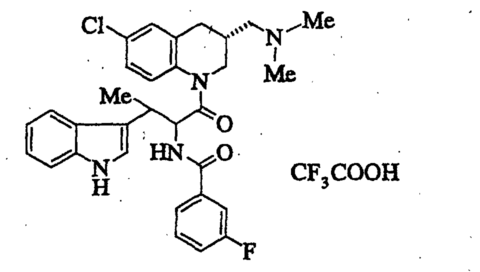

MASS (APCIMASS), m/z 547 [(M+H)⁺].

### Example 28

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-2,4-difluorobenzamide trifluoroacetate

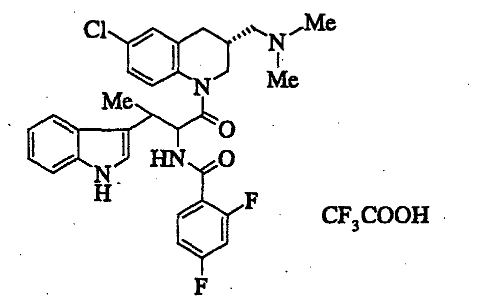

MASS (APCIMASS), m/z 565 [(M+H)⁺].

### Example 29

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-2-methyl-3-furanamide trifluoroacetate

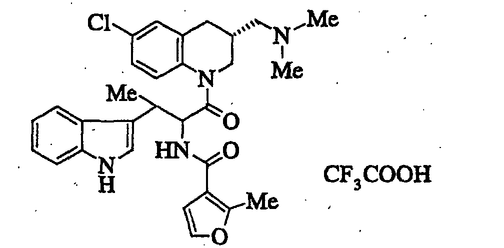

MASS (APCIMASS), m/z 533 [(M+H)⁺].

### Example 30

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-1H-imidazole-4-carboxamide ditrifluoroacetate

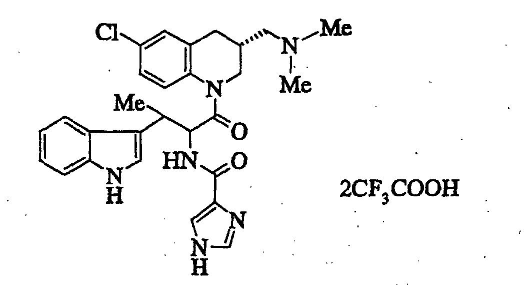

MASS (APCIMASS), m/z 519 [(M+H)⁺].

### Example 31

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-5-oxo-1-phenyl-3-pyrrolidinecarboxamide trifluoroacetate

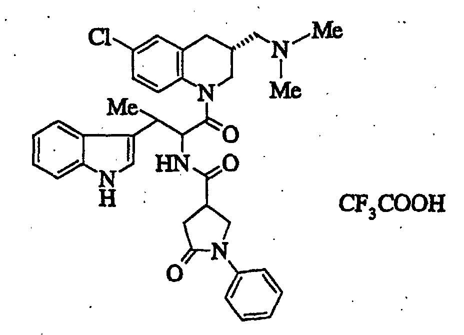

MASS (APCIMASS), m/z 612 [(M+H)⁺].

### Example 32

### 1-acetyl-N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-piperidinecarboxamide trifluoroacetate

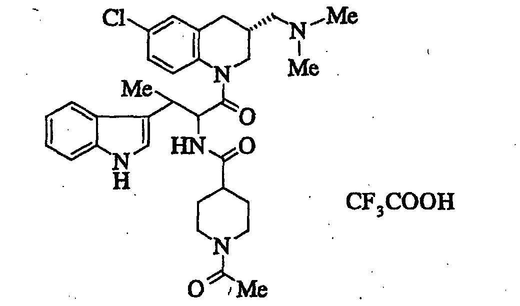

MASS (APCIMASS), m/z 578 [(M+H)⁺].

### Example 33

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-1-methylcyclopropanecarboxamide trifluoroacetate

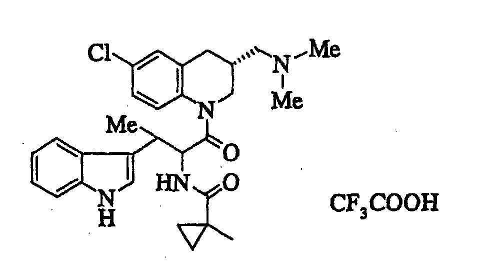

MASS (APCIMASS), m/z 507 [(M+H)⁺].

### Example 34

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-2-cyanoacetamide trifluoroacetate

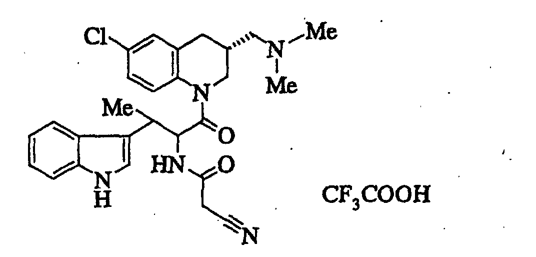

MASS (APCIMASS), m/z 492 [(M+H)⁺].

### Example 35

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-3-methoxypropanamide trifluoroacetate

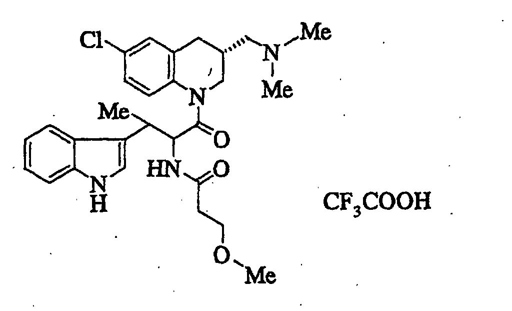

MASS (APCIMASS), m/z 511 [(M+H)⁺].

### Example 36

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-3-(methylthio)propanamide trifluoroacetate

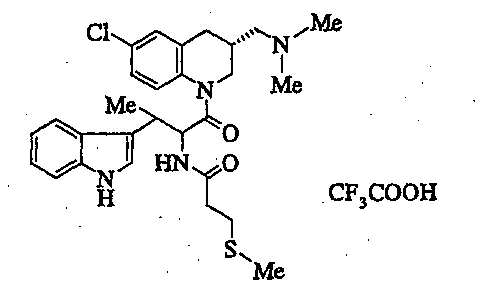

MASS (APCIMASS), m/z 527 [(M+H)⁺].

### Example 37

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-2-(dimethylamino)acetamide ditrifluoroacetate

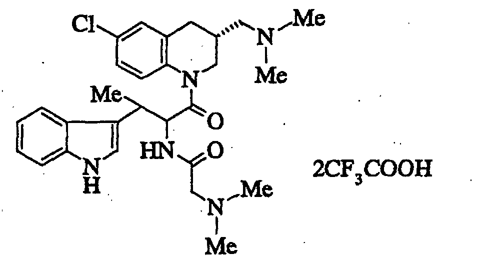

MASS (APCIMASS), m/z 510 [(M+H)⁺].

### Example 38

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-3-furanamide trifluoroacetate

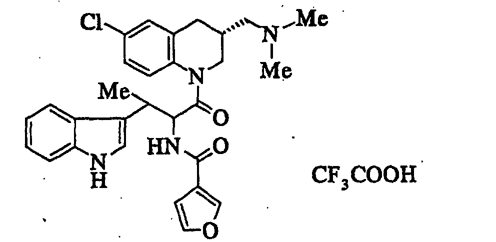

MASS (APCIMASS), m/z 519 [(M+H)⁺].

### Example 39

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-2,2-dimethylpropanamide trifluoroacetate

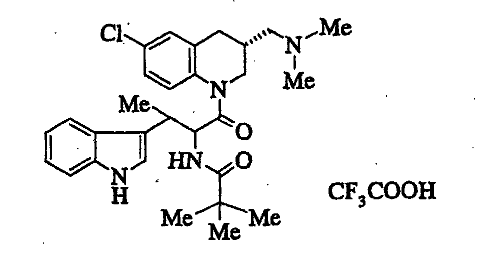

MASS (APCIMASS), m/z 509 [(M+H)⁺].

### Example 40

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]cyclohexanecarboxamide trifluoroacetate

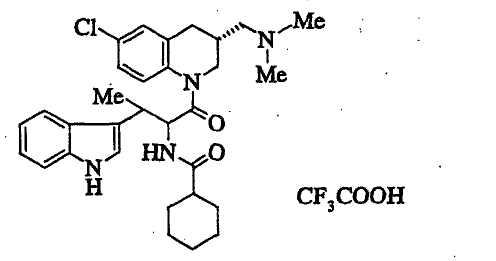

MASS (APCIMASS), m/z 535 [(M+H)⁺].

### Example 41

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-phenoxy-1-piperidinecarboxamide

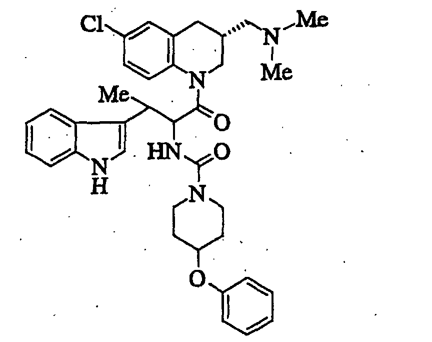

To a solution of (2RS,3SR)-1-((3R)-6-chloro-3-[(dimethylamino)methyl]-3,4-dihydro-1(2H)-quinolinyl)-3-(1H-indol-3-yl)-1-oxo-2-butanamine (0.20 g) and diisopropylethylamine (0.12 mL) in acetonitrile (10 mL) was added N,N'-disuccinimidyl carbonate (0.18 g) under ice-cooling, and the mixture was stirred for 30 min. To the solution was added a suspension of 4-phenoxypiperidine hydrochloride (0.15 g) and diisopropylethylamine (0.25 mL) in acetonitrile (2 mL) under ice-cooling. The reaction solution was stirred at room temperature for 16 hrs., added to 5% sodium hydrogencarbonate solution (50 mL) and extracted with ethyl acetate. The extract was washed with water (50 mL) and saturated brine (50 mL), and dried (MgSO₄). The solvent was evaporated under reduced pressure and the residue was purified by alumina column chromatography (developing solvent; ethyl acetate/hexane = 1:1 - ethyl acetate/ethanol = 20:1) to give a colorless amorphous powder. The powder was washed with diisopropyl ether to give the title compound as a colorless amorphous powder (0.18 g, yield 61%).
IR(KBr) 3299, 2934, 1626, 1489, 1229, 1040 cm⁻¹
MASS (FABMASS), m/z 628 [(M+H)⁺].

The compounds described in the following Examples 42-44 were synthesized in the similar manner as in Example 41.

### Example 42

### N-[(1R,2S)-1-[((3R)-6-chloro-3-[(dimethylamino)methyl]-3,4-dihydro-1(2H)-quinolinyl)carbonyl]-2-(1H-indol-3-yl)propyl]-4-phenoxy-1-piperidinecarboxamide

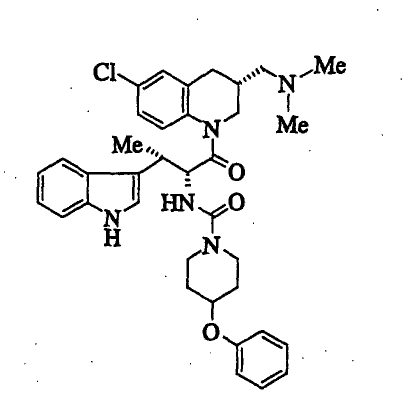

IR(KBr) 2938, 1628, 1489, 1229, 1040, 743, 694 cm⁻¹

### Example 43

### N-[(1S,2R)-1-[((3R)-6-chloro-3-[(dimethylamino)methyl]-3,4-dihydro-1(2H)-quinolinyl)carbonyl]-2-(1H-indol-3-yl)propyl]-4-phenoxy-1-piperidinecarboxamide

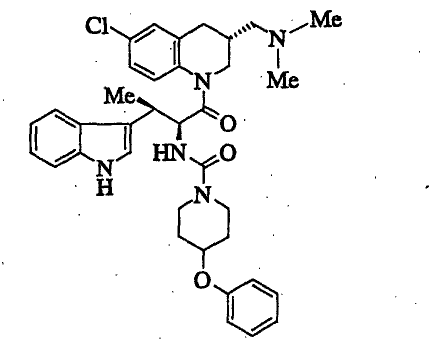

IR(KBr) 2932, 1626, 1489, 1229, 1040, 743, 694 cm⁻¹

### Example 44

### N-[(1RS,2SR)-1-[((3S)-6-chloro-3-[(dimethylamino)methyl]-3,4-dihydro-1(2H)-quinolinyl)carbonyl]-2-(1H-indol-3-yl)propyl]-4-phenoxy-1-piperidinecarboxamide

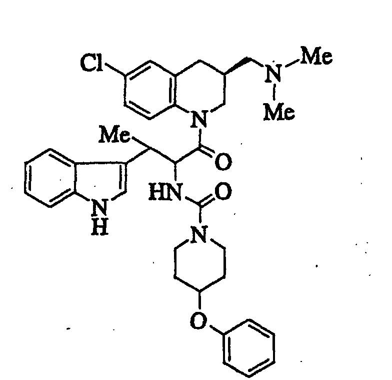

IR(KBr) 2938, 1626, 1489, 1229, 743, 693 cm⁻¹

### Example 45

### N-butyl-N'-[(1RS,2SR)-1-[((3R)-6-chloro-3-[(dimethylamino)methyl)-3,4-dihydro-1(2H)-quinolinyl)carbonyl]-2-(1H-indol-3-yl)propyl]urea trifluoroacetate

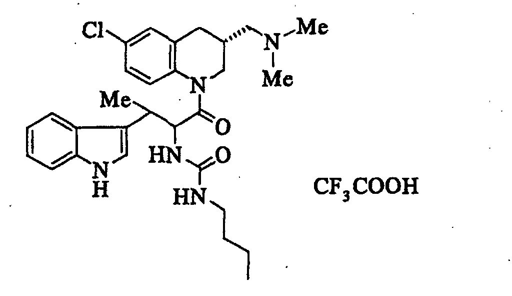

To a solution of (2RS,3SR)-1-((3R)-6-chloro-3-[(dimethylamino)methyl]-3,4-dihydro-1(2H)-quinolinyl)-3-(1H-indol-3-yl)-1-oxo-2-butanamine (20 mg) and diisopropylethylamine (0.016 mL) in acetonitrile (1.5 mL) was added N,N'-disuccinimidyl carbonate (13 mg) under ice-cooling, and the mixture was stirred for 60 min. To the solution was added a solution of butylamine (4.1 mg) and diisopropylethylamine (0.016 mL) in acetonitrile (0.5 mL) under ice-cooling. The reaction solution was stirred at room temperature for 10 hrs. The solvent was evaporated under reduced pressure, and then water and dichloromethane were added to residue and the mixture was vigorously shaken. After the mixture was left standing, the organic layer was separated, and the solvent was evaporated under reduced pressure. The residue was purified by HPLC (mobile phase; water (containing 0.03% trifluoroacetic acid)/acetonitrile (containing 0.03% trifluoroacetic acid) = 9:1 - 1:9) to give the title compound as a colorless amorphous powder (23.5 mg, yield 78%). MASS (APCIMASS), m/z 525 [(M+H)⁺].

The compounds described in the following Examples 46-116 were synthesized in the similar manner as in Example 45.

### Example 46

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-(cyclohexylmethyl)urea trifluoroacetate

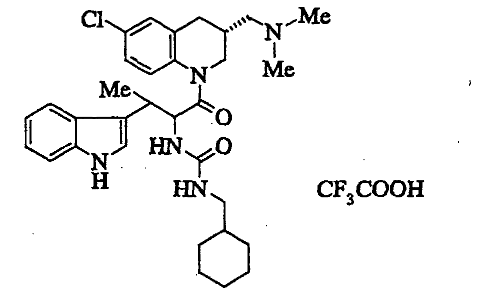

MASS (APCIMASS), m/z 564 [(M+H)⁺].

### Example 47

### N-benzyl-N'-[(1RS,2SR)-1-[((3R)-6-chloro-3-[(dimethylamino)methyl]-3,4-dihydro-1(2H)-quinolinyl)carbonyl]-2-(1H-indol-3-yl)propyl]urea trifluoroacetate

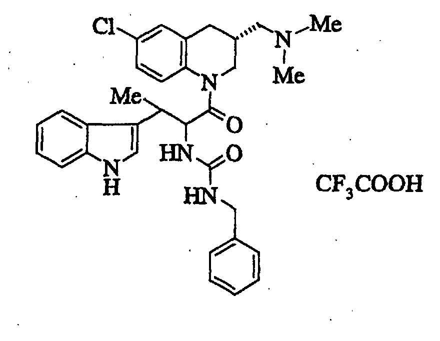

MASS (APCIMASS), m/z 558 [(M+H)⁺].

### Example 48

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-(2-methoxyethyl)urea trifluoroacetate

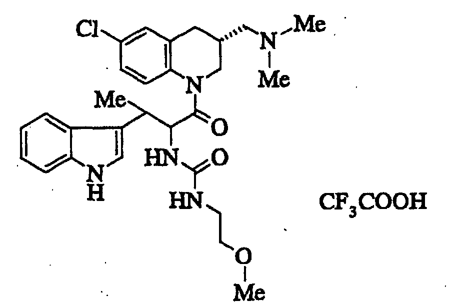

MASS (APCIMASS), m/z 526 [(M+H)⁺].

### Example 49

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-[3-(methylthio)propyl]urea trifluoroacetate

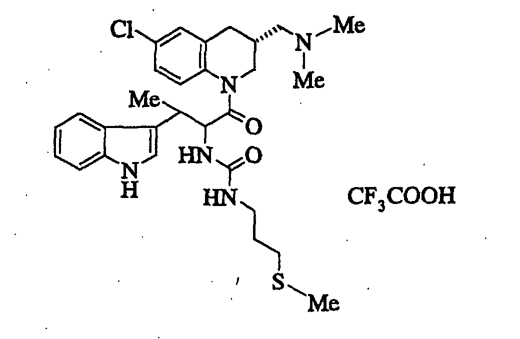

MASS (APCIMASS), m/z 556 [(M+H)⁺].

### Example 50

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-(tetrahydro-2-furanylmethyl)urea trifluoroacetate

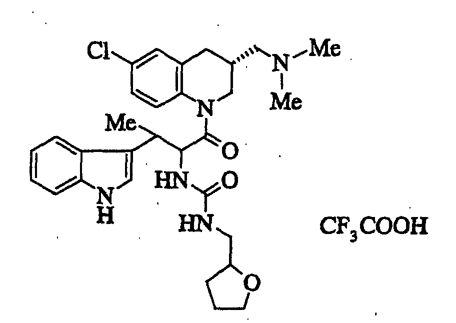

MASS (APCIMASS), m/z 552 [(M+H)⁺].

### Example 51

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-(1-ethylpropyl)urea trifluoroacetate

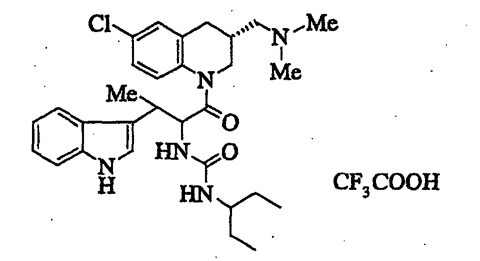

MASS (APCIMASS), m/z 538 [(M+H)⁺].

### Example 52

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-(2-propynyl)urea trifluoroacetate

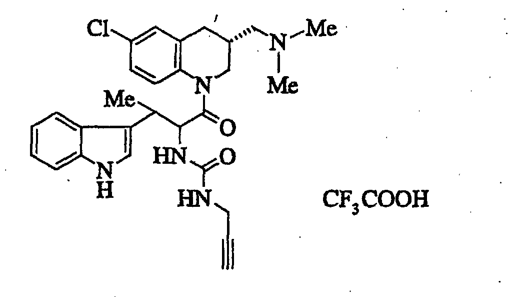

MASS (APCIMASS), m/z 506 [(M+H)⁺].

### Example 53

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-(2,3-dihydro-1H-inden-2-yl)urea trifluoroacetate

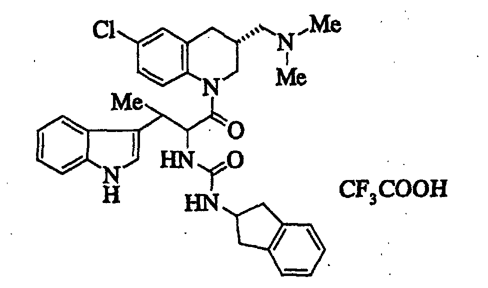

MASS (APCIMASS), m/z 584 [(M+H)⁺].

### Example 54

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-(3-isopropoxypropyl)urea trifluoroacetate

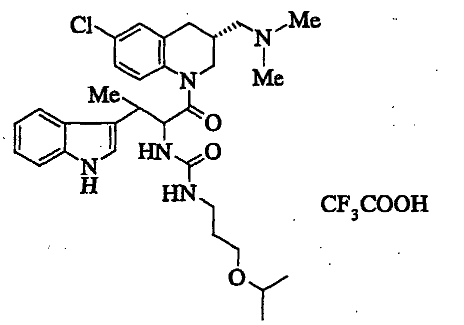

MASS (APCIMASS), m/z 568 [(M+H)⁺].

### Example 55

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-[3-(2-oxo-1-pyrrolidinyl)propyl]urea trifluoroacetate

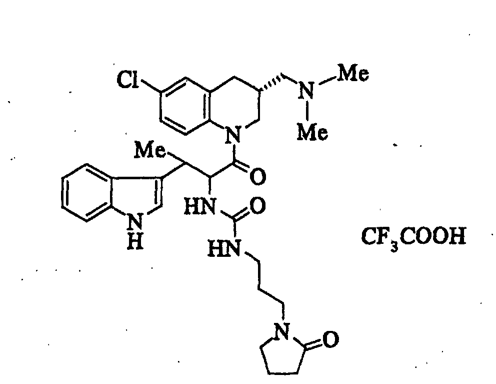

MASS (APCIMASS), m/z 593 [(M+H)⁺].

### Example 56

### N'-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N,N-bis(2-methoxyethyl)urea trifluoroacetate

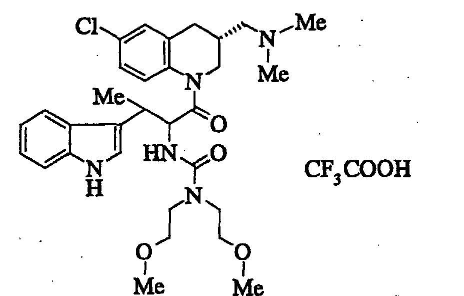

MASS (APCIMASS), m/z 584 [(M+H)⁺].

### Example 57

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-3,4-dihydro-2(1H)-isoquinolinecarboxamide trifluoroacetate

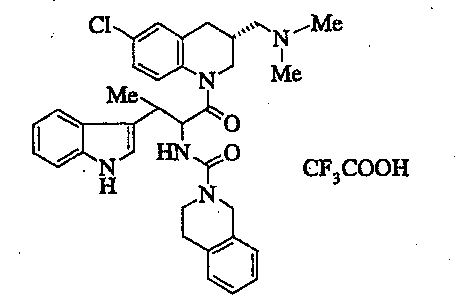

MASS (APCIMASS), m/z 584 [(M+H)⁺].

### Example 58

### N¹-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-1,4-piperidinedicarboxamide trifluoroacetate

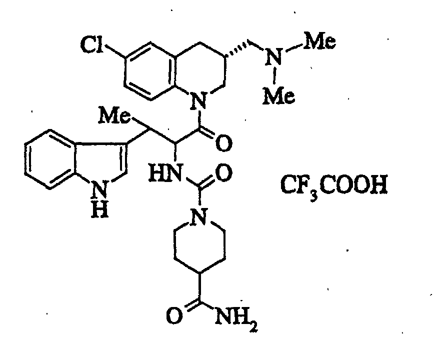

MASS (APCIMASS), m/z 579 [(M+H)⁺].

### Example 59

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-thiomorpholinecarboxamide trifluoroacetate

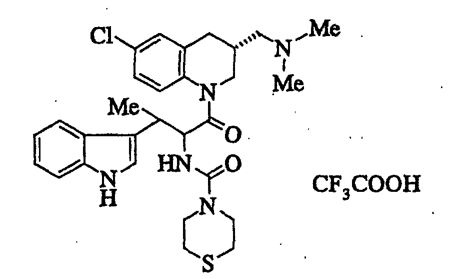

MASS (APCIMASS) , m/z 554 [(M+H)⁺].

### Example 60

### N-benzyl-N'-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N-methylurea trifluoroacetate

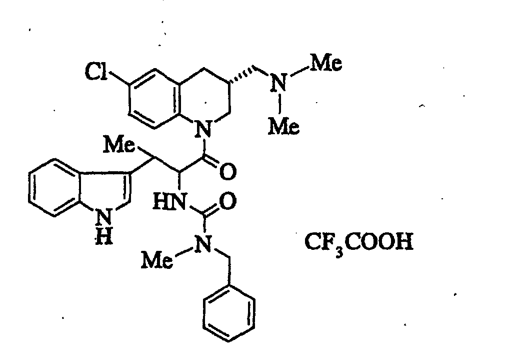

MASS (APCIMASS), m/z 572 [(M+H)⁺].

### Example 61

### N'-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N-ethyl-N-(2-methoxyethyl)urea trifluoroacetate

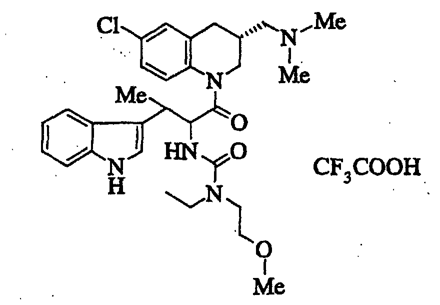

MASS (APCIMASS), m/z 554 [(M+H)⁺].

### Example 62

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-morpholinecarboxamide trifluoroacetate

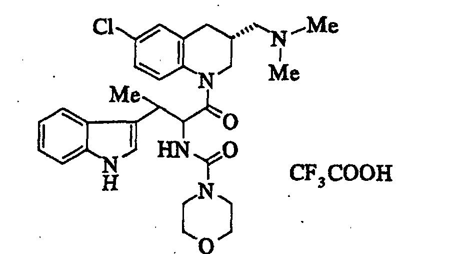

MASS (APCIMASS), m/z 538 [(M+H)⁺].

### Example 63

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]octahydro-2(1H)-isoquinolinecarboxamide trifluoroacetate

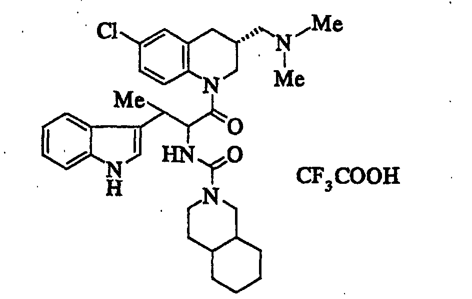

MASS (APCIMASS), m/z 590 [(M+H)⁺].

### Example 64

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-[2-(dimethylamino)ethyl]urea ditrifluoroacetate

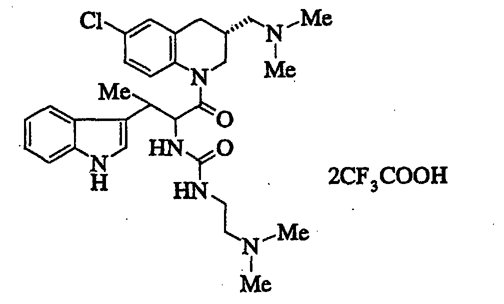

MASS (APCIMASS), m/z 539 [(M+H)⁺].

### Example 65 ,

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-[3-(diethylamino)propyl]urea ditrifluoroacetate

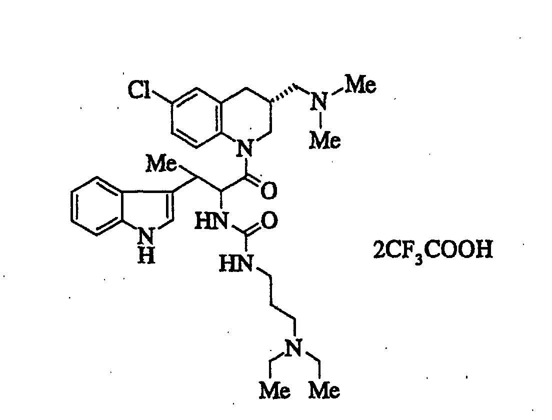

MASS (APCIMASS) , m/z 581 [(M+H)⁺].

### Example 66

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-[2-(1-piperidinyl)ethyl]urea ditrifluoroacetate

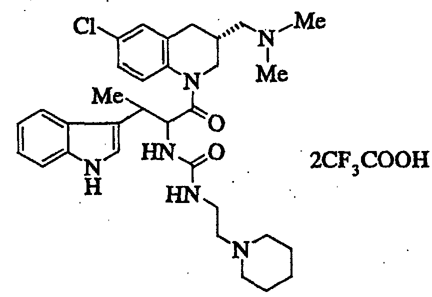

MASS (APCIMASS), m/z 579 [(M+H)⁺].

### Example 67

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-[2-(4-morpholinyl)ethyl]urea ditrifluoroacetate

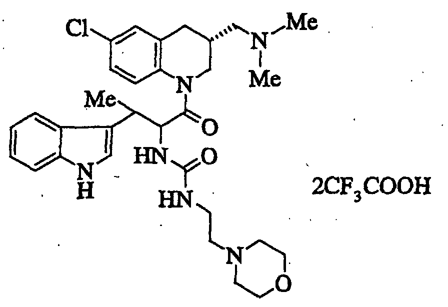

MASS (APCIMASS), m/z 581 [(M+H)⁺].

### Example 68

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-(2-pyridinylmethyl)urea ditrifluoroacetate

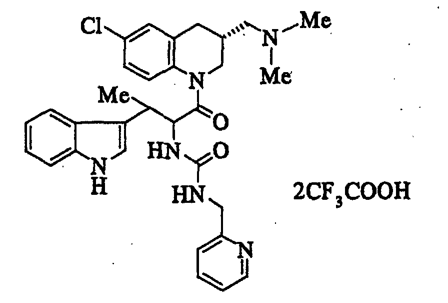

MASS (APCIMASS), m/z 559 [(M+H)⁺].

### Example 69

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-[3-(1H-imidazol-1-yl)propyl]urea ditrifluoroacetate

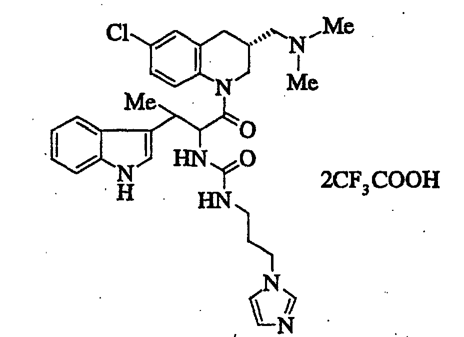

MASS (APCIMASS), m/z 576 [(M+H)⁺].

### Example 70

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-[2-(diisopropylamino)ethyl]urea ditrifluoroacetate

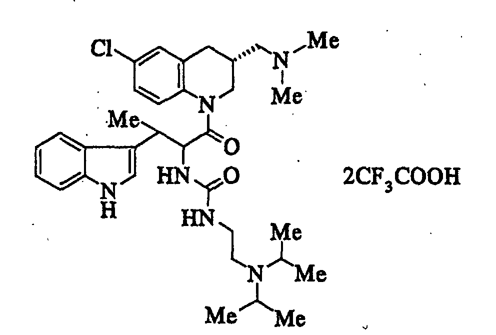

MASS (APCIMASS), m/z 595 [(M+H)⁺].

### Example 71

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-(4-pyridinylmethyl)urea ditrifluoroacetate

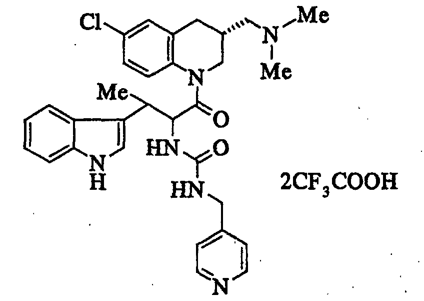

MASS (APCIMASS), m/z 559 [(M+H)⁺].

### Example 72

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-(3-pyridinylmethyl)urea ditrifluoroacetate

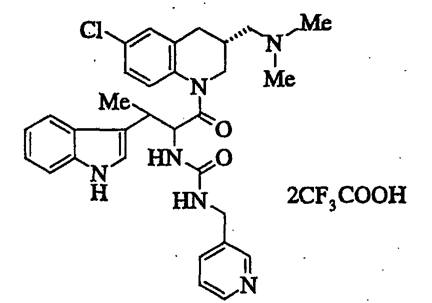

MASS (APCIMASS), m/z 559 [(M+H)⁺].

### Example 73

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-ethyl-1-piperazinecarboxamide ditrifluoroacetate

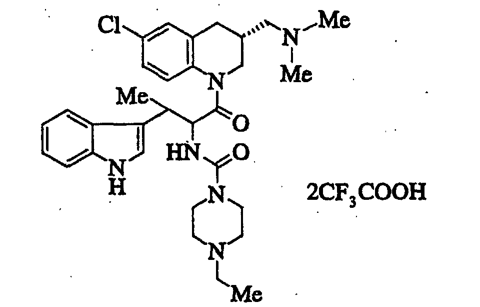

MASS (APCIMASS), m/z 565 [(M+H)⁺].

### Example 74

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-methyl-1,4-diazepane-1-carboxamide ditrifluoroacetate

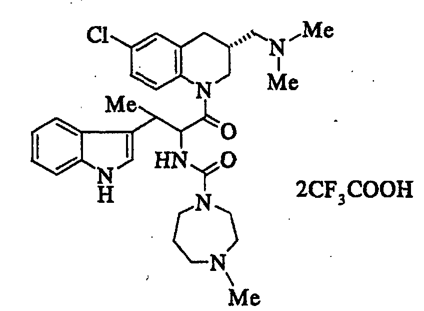

MASS (APCIMASS), m/z 565 [(M+H)⁺].

### Example 75

### (2R)-N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-2-(1-pyrrolidinylmethyl)-1-pyrrolidinecarboxamide ditrifluoroacetate

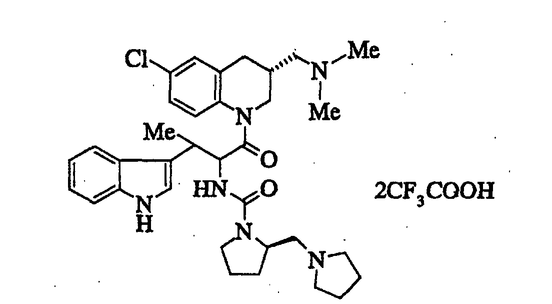

MASS (APCIMASS), m/z 605 [(M+H)⁺].

### Example 76

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(1-pyrrolidinyl)-1-piperidinecarboxamide ditrifluoroacetate

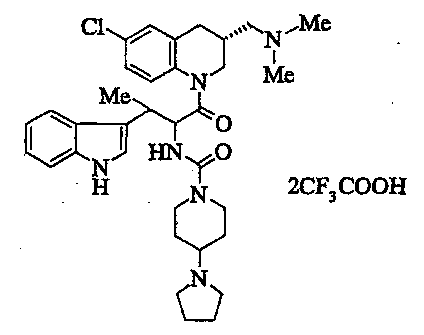

MASS (APCIMASS), m/z 605 [(M+H)⁺].

### Example 77

### N¹-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-1,3-piperidinedicarboxamide trifluoroacetate

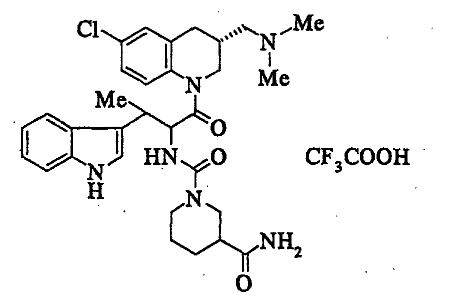

MASS (APCIMASS), m/z 579 [(M+H)⁺].

### Example 78

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-[3-(trifluoromethyl)phenyl]-1-piperazinecarboxamide ditrifluoroacetate

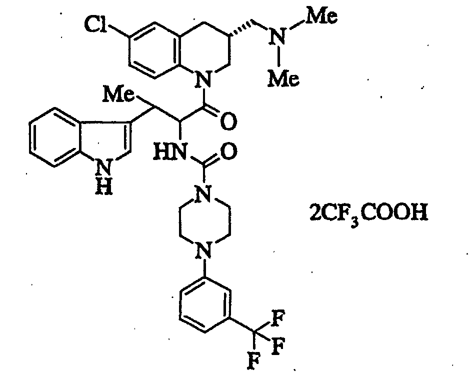

MASS (APCIMASS), m/z 681 [(M+H)⁺].

### Example 79

### 4-(4-acetylphenyl)-N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-1-piperazinecarboxamide ditrifluoroacetate

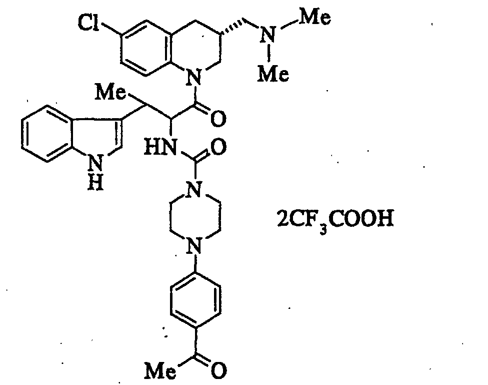

MASS (APCIMASS), m/z 655 [(M+H)⁺].

### Example 80

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(2-chlorophenyl)-1-piperazinecarboxamide ditrifluoroacetate

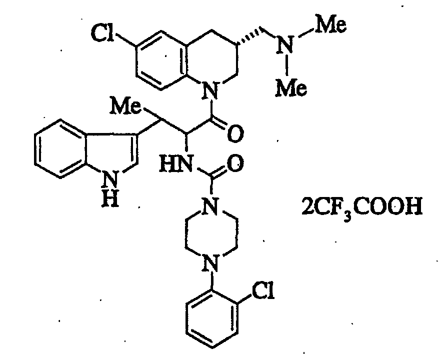

MASS (APCIMASS), m/z 647 [(M+H)⁺].

### Example 81

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(3-methoxyphenyl)-1-piperazinecarboxamide ditrifluoroacetate

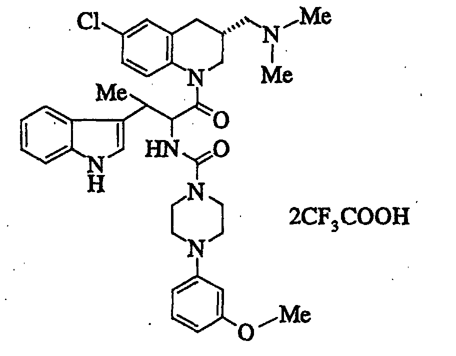

MASS (APCIMASS), m/z 643 [(M+H)⁺].

### Example 82

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(3-chlorophenyl)-1-piperazinecarboxamide ditrifluoroacetate

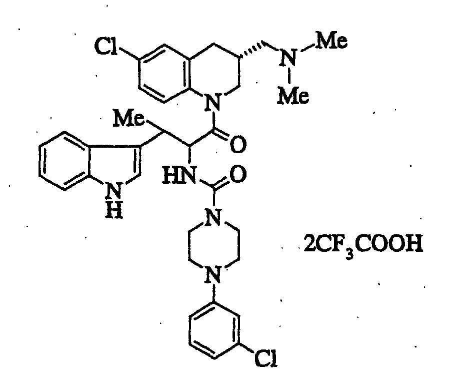

MASS (APCIMASS), m/z 647 [(M+H)⁺].

### Example 83

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(2-methylphenyl)-1-piperazinecarboxamide ditrifluoroacetate

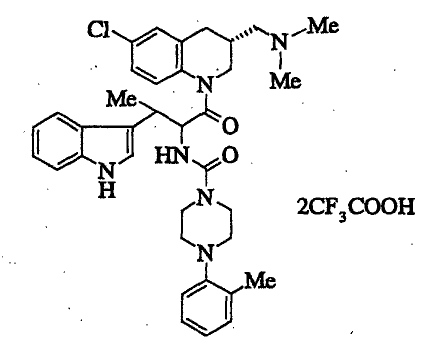

MASS (APCIMASS), m/z 627 [(M+H)⁺].

### Example 84

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(2-ethoxyphenyl)-1-piperazinecarboxamide ditrifluoroacetate

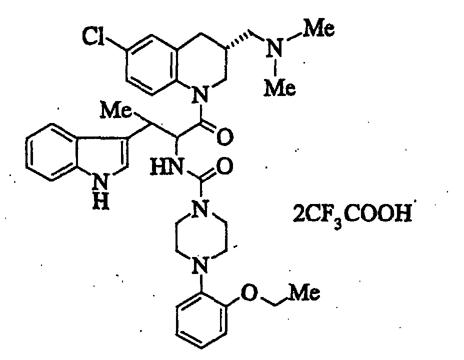

MASS (APCIMASS), m/z 657 [(M+H)⁺].

### Example 85

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(2-fluorophenyl)-1-piperazinecarboxamide ditrifluoroacetate

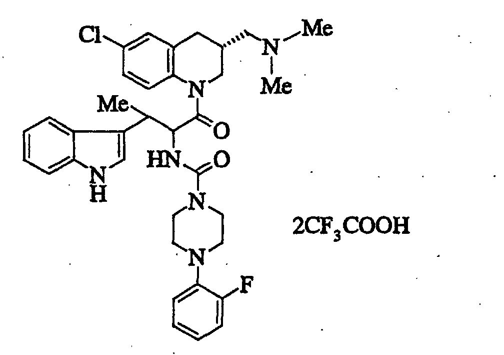

MASS (APCIMASS), m/z 631 [(M+H)⁺].

### Example 86

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(4-chlorophenyl)-1-piperazinecarboxamide ditrifluoroacetate

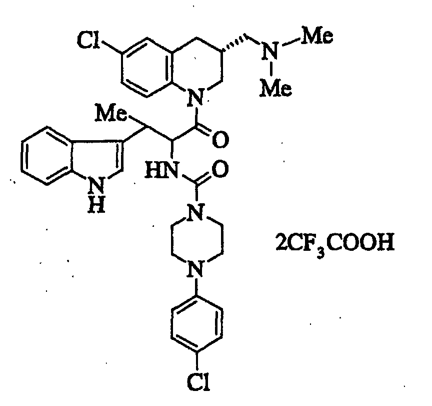

MASS (APCIMASS), m/z 647 [(M+H)⁺].

### Example 87

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(4-methoxyphenyl)-1-piperazinecarboxamide ditrifluoroacetate

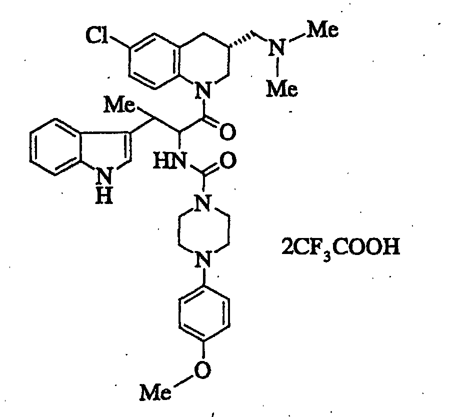

MASS (APCIMASS), m/z 643 [(M+H)⁺].

### Example 88

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(4-fluorophenyl)-1-piperazinecarboxamide ditrifluoroacetate

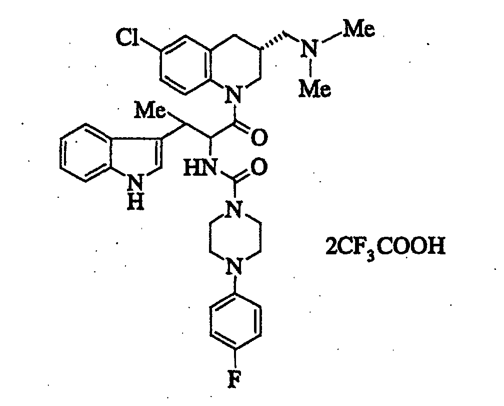

MASS (APCIMASS), m/z 631 [(M+H)⁺].

### Example 89

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(3-methylphenyl)-1-piperazinecarboxamide ditrifluoroacetate

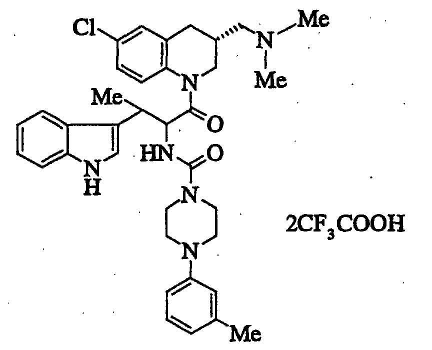

MASS (APCIMASS), m/z 627 [(M+H)⁺].

### Example 90

### 4-benzyl-N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-1-piperazinecarboxamide ditrifluoroacetate

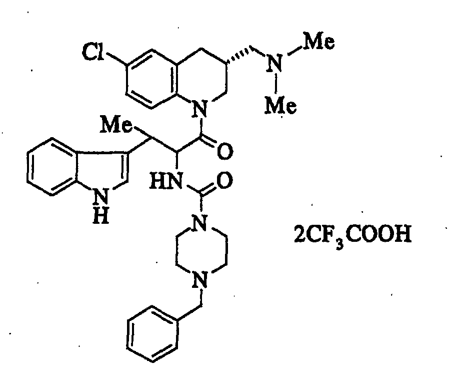

MASS (APCIMASS), m/z 627 [(M+H)⁺].

### Example 91

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(2-pyridinyl)-1-piperazinecarboxamide ditrifluoroacetate

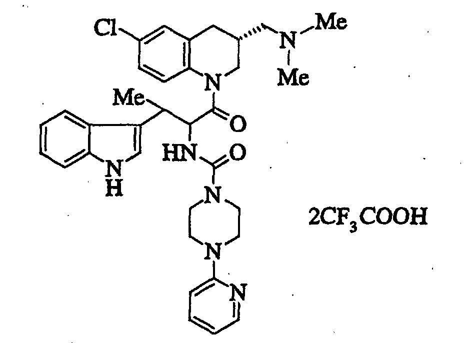

MASS (APCIMASS), m/z 614 [(M+H)⁺].

### Example 92

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-phenyl-1-piperazinecarboxamide ditrifluoroacetate

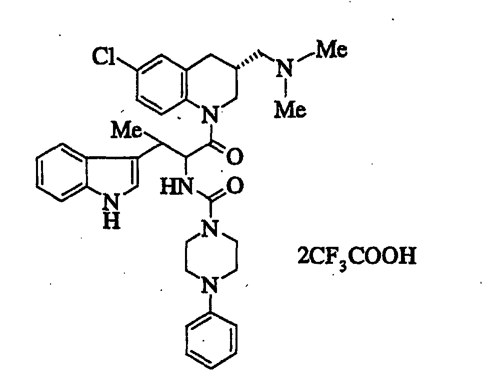

MASS (APCIMASS), m/z 613 [(M+H)⁺].

### Example 93

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(2-methoxyphenyl)-1-piperazinecarboxamide ditrifluoroacetate

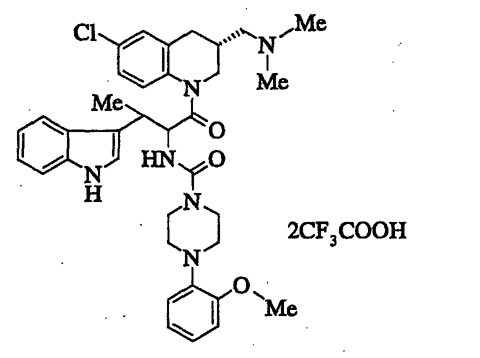

MASS (APCIMASS), m/z 643 [(M+H)⁺].

### Example 94

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(1-piperidinyl)-1-piperidinecarboxamide ditrifluoroacetate

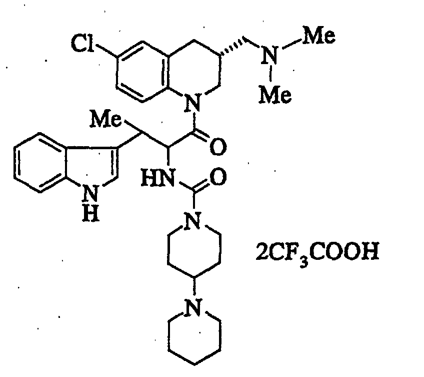

MASS (APCIMASS), m/z 619 [(M+H)⁺].

### Example 95

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(2-pyrimidinyl)-1-piperazinecarboxamide ditrifluoroacetate

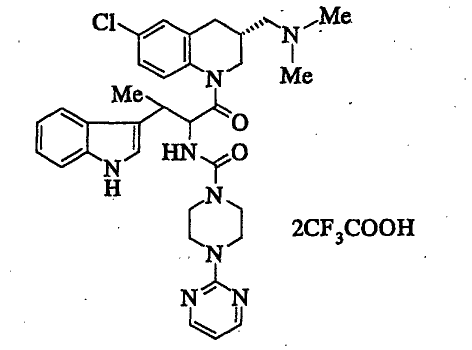

MASS (APCIMASS), m/z 615 [(M+H)⁺].

### Example 96

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-[4-(trifluoromethyl)benzyl]urea trifluoroacetate

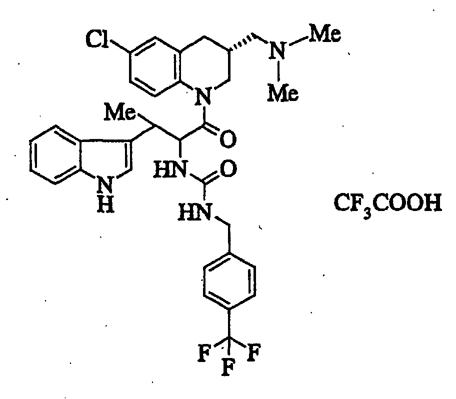

MASS (APCIMASS), m/z 626 [(M+H)⁺].

### Example 97

### 4-benzyl-N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-1-piperidinecarboxamide trifluoroacetate

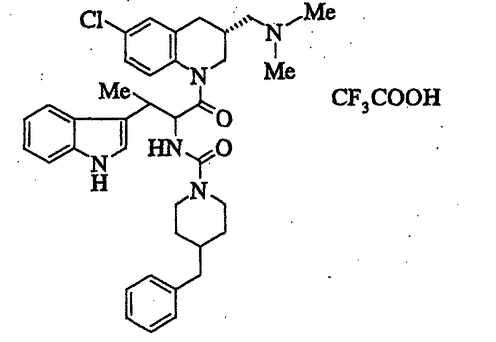

MASS (APCIMASS), m/z 626 [(M+H)⁺].

### Example 98

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-(2-pyridinyl)urea ditrifluoroacetate

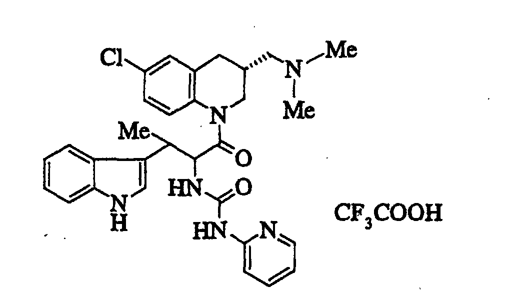

MASS (APCIMASS), m/z 545 [(M+H)⁺].

### Example 99

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-(4-fluorophenyl)urea trifluoroacetate

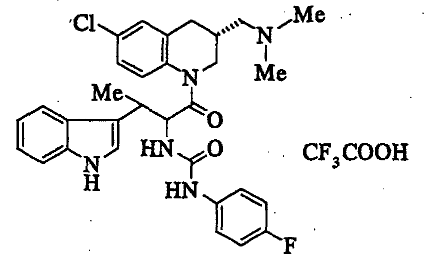

MASS (APCIMASS), m/z 562 [(M+H)⁺].

### Example 100

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-(4-chlorophenyl)urea trifluoroacetate

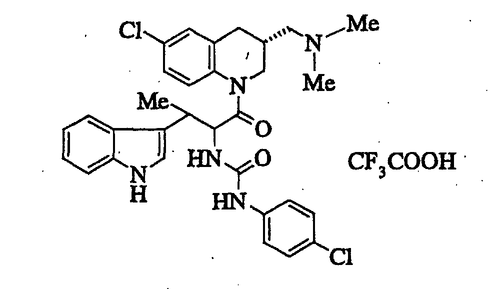

MASS (APCIMASS), m/z 578 [(M+H)⁺].

### Example 101

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-(2,3-dihydro-1H-inden-5-yl)urea trifluoroacetate

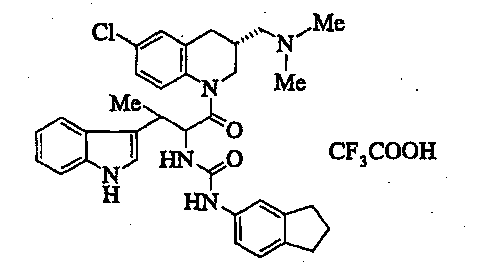

MASS (APCIMASS), m/z 584 [(M+H)⁺].

### Example 102

### 4-[({[(1RS,2SR)-1-[((3R)-6-chloro-3-[(dimethylamino)methyl]-3,4-dihydro-1(2H)-quinolinyl)carbonyl]-2-(1H-indol-3-yl)propyl]amino}carbonyl)amino]benzamide trifluoroacetate

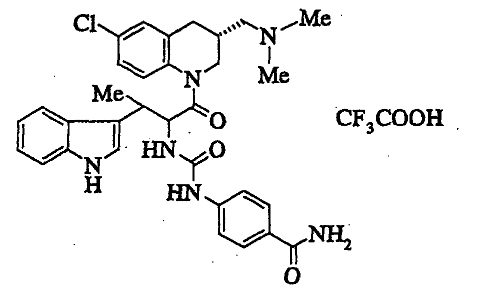

MASS (APCIMASS), m/z 587 [(M+H)⁺].

### Example 103

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-[4-(dimethylamino)phenyl]urea ditrifluoroacetate

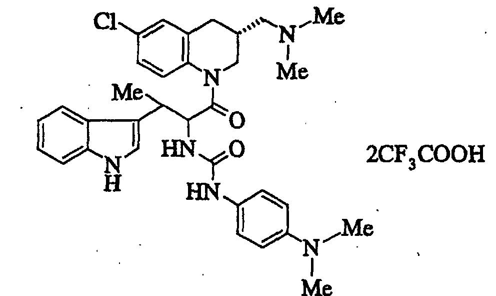

MASS (APCIMASS), m/z 587 [(M+H)⁺].

### Example 104

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-(2-ethoxyphenyl)urea trifluoroacetate

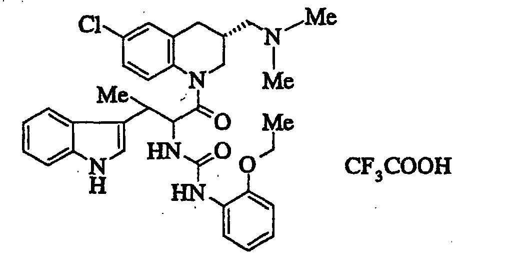

MASS (APCIMASS), m/z 588 [(M+H)⁺].

### Example 105

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-N'-[3-(methylthio)phenyl]urea trifluoroacetate

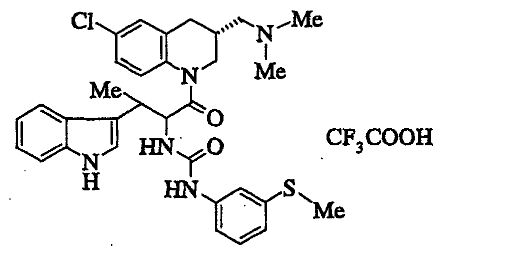

MASS (APCIMASS), m/z 590 [(M+H)⁺].

### Example 106

### N-(4-tert-butylphenyl)-N'-[(1RS,2SR)-1-[((3R)-6-chloro-3-[(dimethylamino)methyl]-3,4-dihydro-1(2H)-quinolinyl)carbonyl]-2-(1H-indol-3-yl)propyl]urea trifluoroacetate

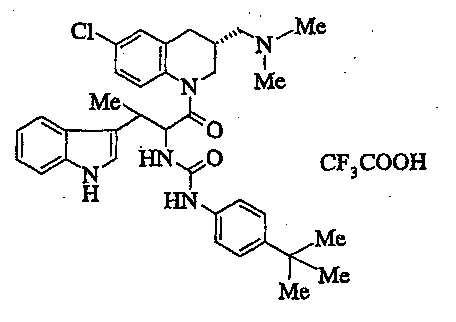

MASS (APCIMASS), m/z 600 [(M+H)⁺].

### Example 107

### N-{4-[({[(1RS,2SR)-1-[((3R)-6-chloro-3-[(dimethylamino)methyl]-3,4-dihydro-1(2H)-quinolinyl)carbonyl]-2-(1H-indol-3-yl)propyl]amino}carbonyl)amino]phenyl}acetamide trifluoroacetate

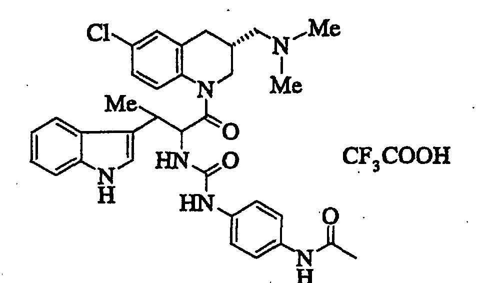

MASS (APCIMASS), m/z 601 [(M+H)⁺].

### Example 108

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-phenyl-1-piperidinecarboxamide trifluoroacetate

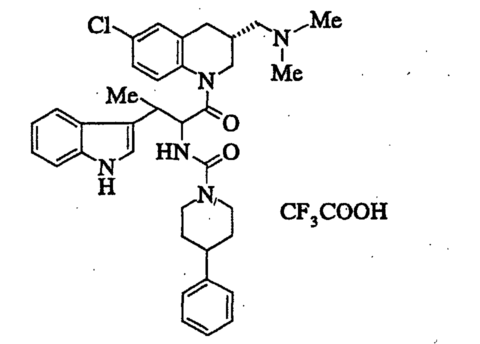

MASS (APCIMASS), m/z 612 [(M+H)⁺].

### Example 109

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(3-fluorophenyl)-1-piperidinecarboxamide trifluoroacetate

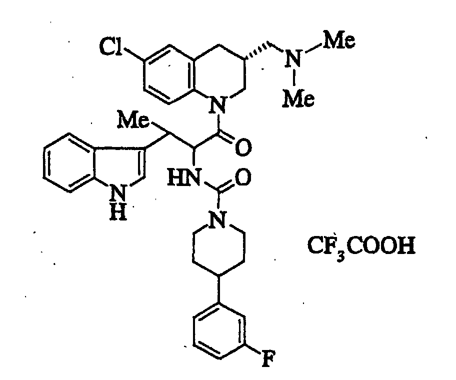

MASS (APCIMASS), m/z 630 [(M+H)⁺].

### Example 110

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(4-fluorophenyl)-1-piperidinecarboxamide trifluoroacetate

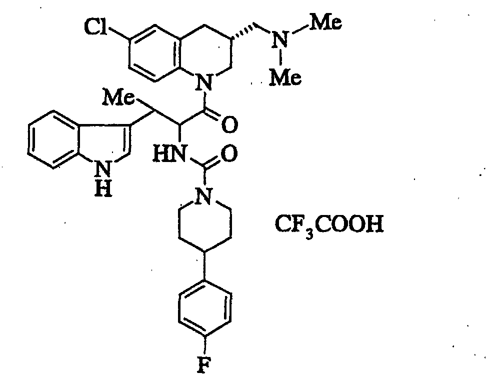

MASS (APCIMASS), m/z 630 [(M+H)⁺].

### Example 111

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(2-methylphenyl)-1-piperidinecarboxamide trifluoroacetate

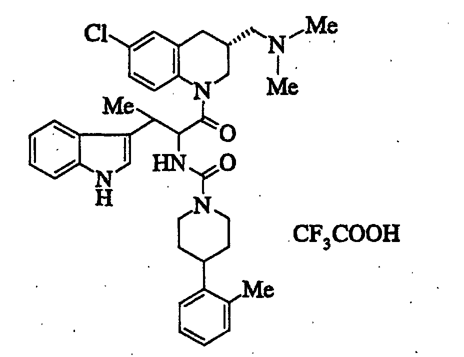

MASS (APCIMASS), m/z 626 [(M+H)⁺].

### Example 112

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(3-methylphenyl)-1-piperidinecarboxamide trifluoroacetate

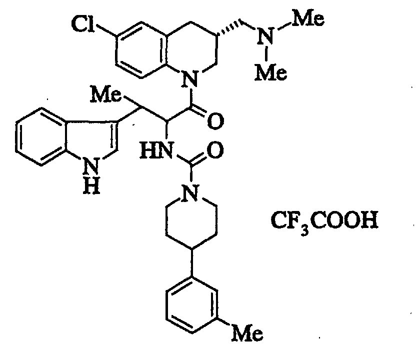

MASS (APCIMASS), m/z 626 [(M+H)⁺].

### Example 113

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(4-methylphenyl)-1-piperidinecarboxamide trifluoroacetate

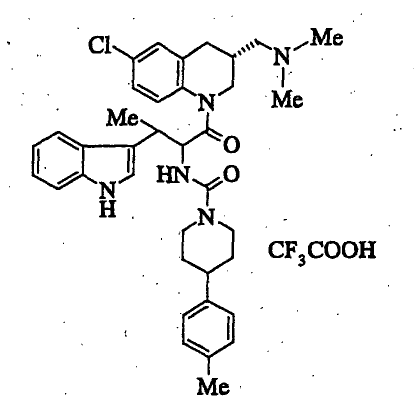

MASS (APCIMASS), m/z 626 [(M+H)⁺].

### Example 114

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(3-methoxyphenyl)-1-piperidinecarboxamide trifluoroacetate

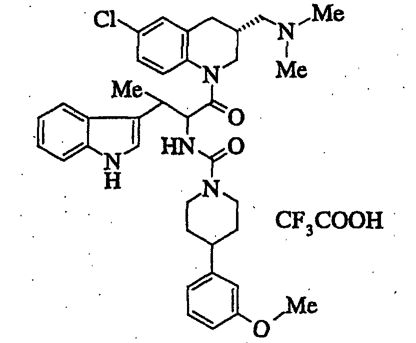

MASS (APCIMASS), m/z 642 [(M+H)⁺].

### Example 115

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(4-chlorophenyl)-1-piperidinecarboxamide trifluoroacetate

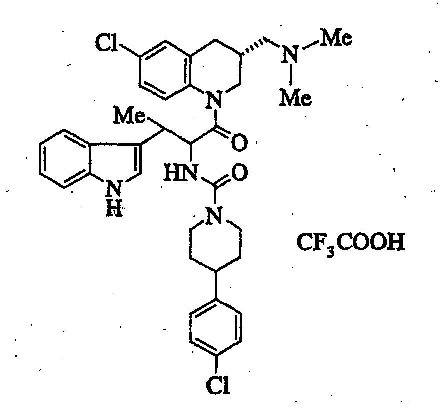

MASS (APCIMASS), m/z 646 [(M+H)⁺].

### Example 116

### 4-benzoyl-N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-1-piperidinecarboxamide trifluoroacetate

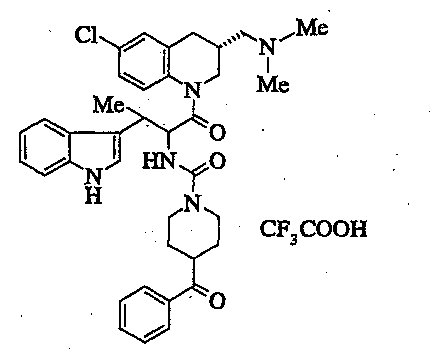

MASS (APCIMASS), m/z 640 [(M+H)⁺].

### Example 117

### (2RS,3SR)-1-((3R)-6-chloro-3-[(dimethylamino)methyl]-3,4-dihydro-1(2H)-quinolinyl)-3-(1H-indol-3-yl)-N-({1-[(1-methyl-1H-indol-2-yl)carbonyl]-4-piperidinyl}methyl)-1-oxo-2-butanamine ditrifluoroacetate

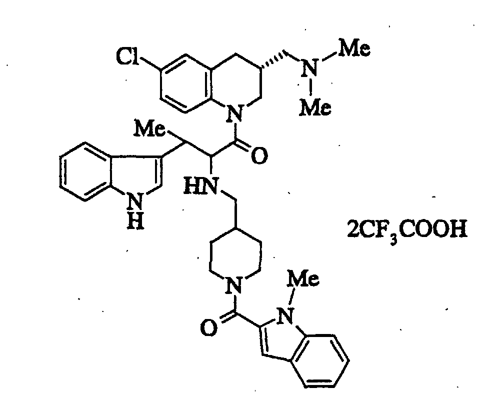

To a solution of (2RS,3SR)-1-((3R)-6-chloro-3-[(dimethylamino)methyl]-3,4-dihydro-1(2H)-quinolinyl)-3-(1H-indol-3-yl)-1-oxo-2-butanamine (20 mg) in dichloromethane (2 mL) was added 1-[(1-methyl-1H-indol-2-yl)carbonyl]-4-piperidinecarbaldehyde (15 mg) at room temperature, and the mixture was stirred for 15 min. To the reaction mixture was added sodium triacetoxyborohydride (13 mg) at room temperature. The reaction solution was stirred at room temperature for 10 hrs., and then water was added thereto and vigorously shaken. After the obtained mixture was left standing, the organic layer was separated, and the solvent was evaporated under reduced pressure. The residue was purified by HPLC (mobile phase; water (containing 0.03% trifluoroacetic acid)/acetonitrile (containing 0.03% trifluoroacetic acid)= 9:1 - 1:9) to give the title compound as a colorless amorphous powder (30.8 mg, yield 72%).
MASS (APCIMASS), m/z 679 [(M+H)⁺].

The compounds described in the following Examples 118-131 were synthesized in the similar manner as in Example 117.

### Example 118

### N-[(1-benzoyl-4-piperidinyl)methyl]-(2RS,3SR)-1-((3R)-6-chloro-3-[(dimethylamino)methyl]-3,4-dihydro-1(2H)-quinolinyl)-3-(1H-indol-3-yl)-1-oxo-2-butanamine ditrifluoroacetate

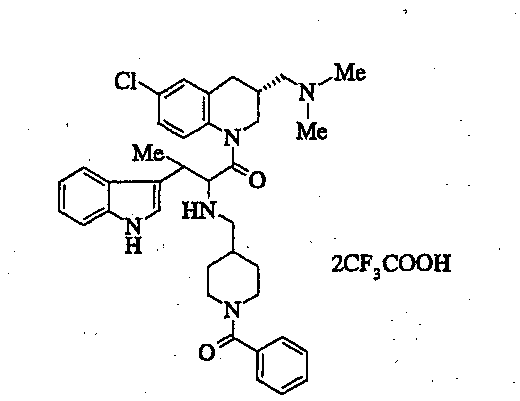

MASS (APCIMASS), m/z 626 [(M+H)⁺].

### Example 119

### benzyl 4-({[(1RS,2SR)-1-[((3R)-6-chloro-3-[(dimethylamino)methyl]-3,4-dihydro-1(2H)-quinolinyl)carbonyl]-2-(1H-indol-3-yl)propyl]amino}methyl)-1-piperidinecarboxylate ditrifluoroacetate

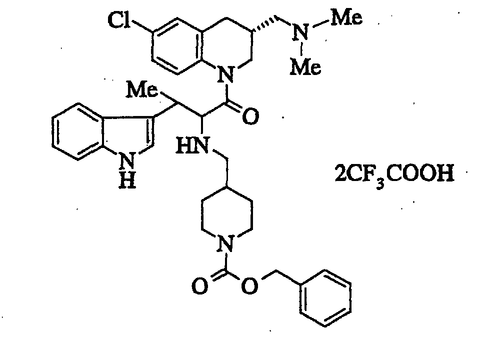

MASS (APCIMASS), m/z 656 [(M+H)⁺].

### Example 120

### (2RS,3SR)-1-((3R)-6-chloro-3-[(dimethylamino)methyl]-3,4-dihydro-1(2H)-quinolinyl)-3-(1H-indol-3-yl)-N-[4-(methylsulfonyl)benzyl]-1-oxo-2-butanamine ditrifluoroacetate

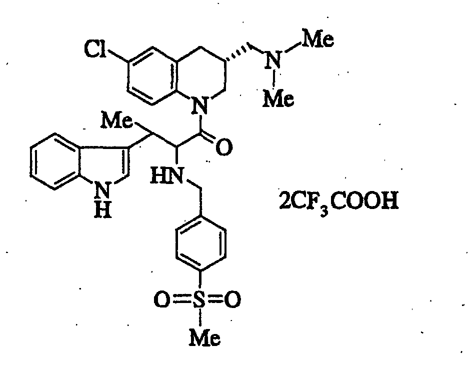

MASS (APCIMASS), m/z 593 [(M+H)⁺].

### Example 121

### (2RS,3SR)-1-((3R)-6-chloro-3-[(dimethylamino)methyl]-3,4-dihydro-1(2H)-quinolinyl)-3-(1H-indol-3-yl)-N-[4-(methylthio)benzyl]-1-oxo-2-butanamine ditrifluoroacetate

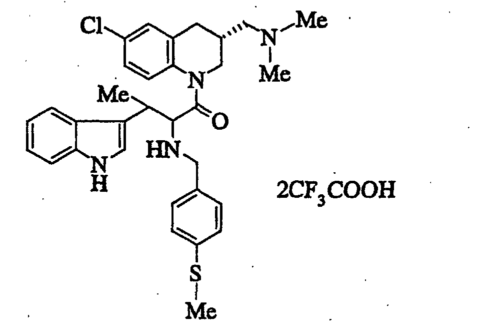

MASS (APCIMASS), m/z 561 [(M+H)⁺].

### Example 122

### (2RS,3SR)-1-((3R)-6-chloro-3-[(dimethylamino)methyl]-3,4-dihydro-1(2H)-quinolinyl)-N-(cyclohexylmethyl)-3-(1H-indol-3-yl)-1-oxo-2-butanamine ditrifluoroacetate

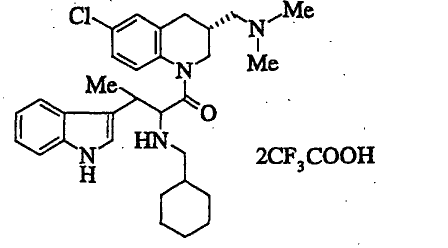

MASS (APCIMASS), m/z 521 [(M+H)⁺].

### Example 123

### N-(2-chlorobenzyl)-(2RS,3SR)-1-((3R)-6-chloro-3-[(dimethylamino)methyl]-3,4-dihydro-1(2H)-quinolinyl)-3-(1H-indol-3-yl)-1-oxo-2-butanamine ditrifluoroacetate

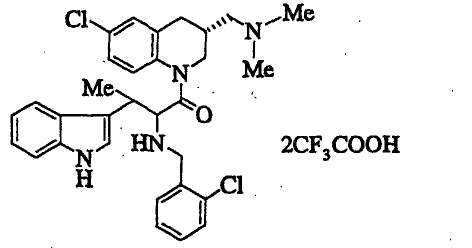

MASS (APCIMASS), m/z 549 [(M+H)⁺].

### Example 124

### N-(4-chlorobenzyl)-(2RS,3SR)-1-((3R)-6-chloro-3-[(dimethylamino)methyl]-3,4-dihydro-1(2H)-quinolinyl)-3-(1H-indol-3-yl)-1-oxo-2-butanamine ditrifluoroacetate

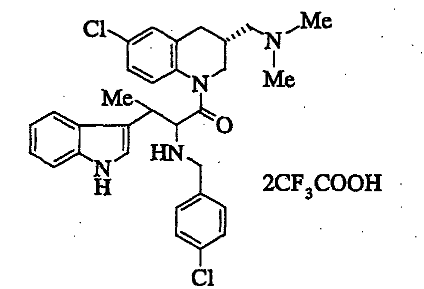

MASS (APCIMASS), m/z 549 [(M+H)⁺].

### Example 125

### (2RS,3SR)-1-((3R)-6-chloro-3-[(dimethylamino)methyl]-3,4-dihydro-1(2H)-quinolinyl)-3-(1H-indol-3-yl)-N-(2-methoxybenzyl)-1-oxo-2-butanamine ditrifluoroacetate

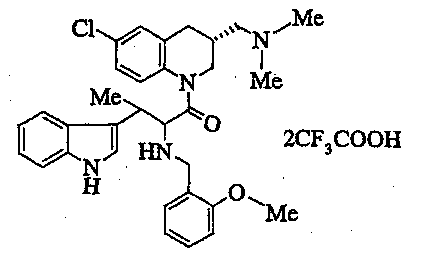

MASS (APCIMASS), m/z 545 [(M+H)⁺].

### Example 126

### (2RS,3SR)-1-((3R)-6-chloro-3-[(dimethylamino)methyl]-3,4-dihydro-1(2H)-quinolinyl)-3-(1H-indol-3-yl)-N-(4-methoxybenzyl)-1-oxo-2-butanamine ditrifluoroacetate

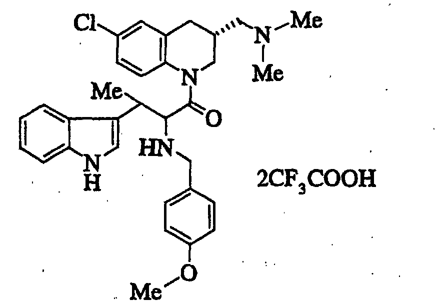

MASS (APCIMASS), m/z 545 [(M+H)⁺].

### Example 127

### (2RS,3SR)-1-((3R)-6-chloro-3-[(dimethylamino)methyl]-3,4-dihydro-1(2H)-quinolinyl)-N-(2-methylbenzyl)-3-(1H-indol-3-yl)-1-oxo-2-butanamine ditrifluoroacetate

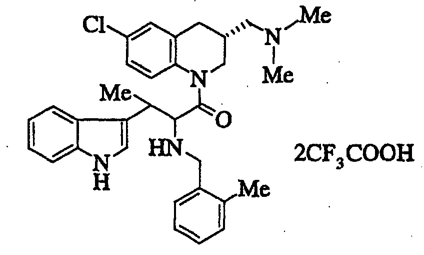

MASS (APCIMASS), m/z 529 [(M+H)⁺].

### Example 128

### (2RS,3SR)-1-((3R)-6-chloro-3-[(dimethylamino)methyl]-3,4-dihydro-1(2H)-quinolinyl)-N-(4-methylbenzyl)-3-(1H-indol-3-yl)-1-oxo-2-butanamine ditrifluoroacetate

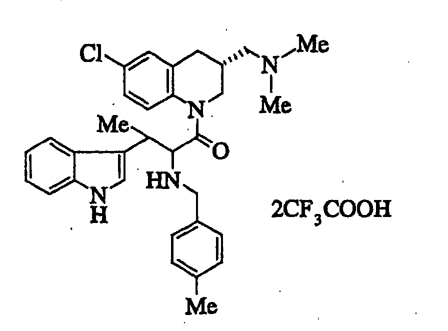

MASS (APCIMASS), m/z 529 [(M+H)⁺].

### Example 129

### (2RS,3SR)-1-((3R)-6-chloro-3-[(dimethylamino)methyl]-3,4-dihydro-1(2H)-quinolinyl)-N-(2-fluorobenzyl)-3-(1H-indol-3-yl)-1-oxo-2-butanamine ditrifluoroacetate

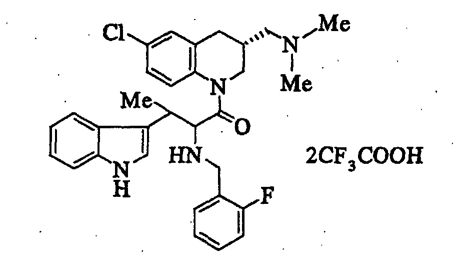

MASS (APCIMASS), m/z 533 [(M+H)⁺].

### Example 130

### (2RS,3SR)-1-((3R)-6-chloro-3-[(dimethylamino)methyl]-3,4-dihydro-1(2H)-quinolinyl)-N-(3-fluorobenzyl)-3-(1H-indol-3-yl)-1-oxo-2-butanamine ditrifluoroacetate

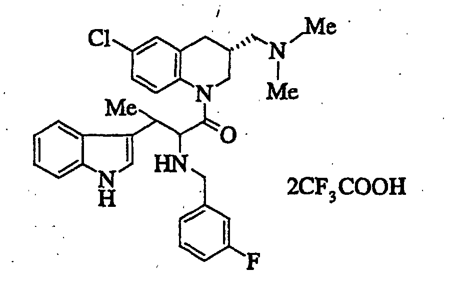

MASS (APCIMASS), m/z 533 [(M+H)⁺].

### Example 131

### (2RS,3SR)-1-((3R)-6-chloro-3-[(dimethylamino)methyl]-3,4-dihydro-1(2H)-quinolinyl)-N-(4-fluorobenzyl)-3-(1H-indol-3-yl)-1-oxo-2-butanamine ditrifluoroacetate

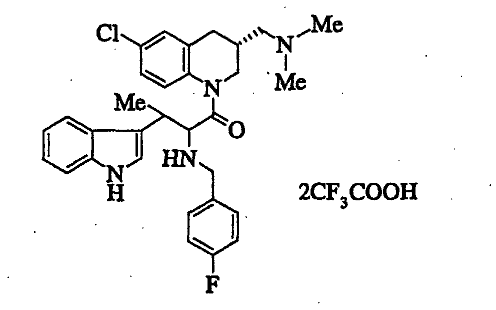

MASS (APCIMASS), m/z 533 [(M+H)⁺].

### Example 132

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-1-butanamine ditrifluoroacetate

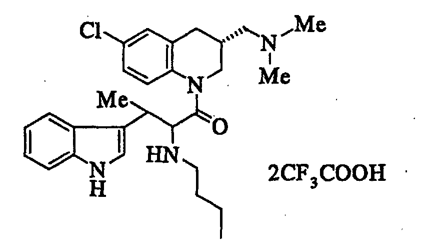

To a solution of (2RS,3SR)-1-((3R)-6-chloro-3-[(dimethylamino)methyl]-3,4-dihydro-1(2H)-quinolinyl)-3-(1H-indol-3-yl)-1-oxo-2-butanamine (20 mg) in dichloromethane (2 mL) was added butanal (4.0 mg) at room temperature and the mixture was stirred for 15 min. Sodium triacetoxyborohydride (13 mg) was added to the reaction mixture at room temperature. The reaction solution was stirred at room temperature for 10 hrs. and then water was added thereto and vigorously shaken. After the obtained mixture was left standing, the organic layer was separated, and the solvent was evaporated under reduced pressure. The residue was purified by HPLC (mobile phase; water (containing 0.03% trifluoroacetic acid)/acetonitrile (containing 0.03% trifluoroacetic acid) = 9:1 - 1:9) to give the title compound as a colorless amorphous powder (8.4 mg, yield 25%).
MASS (APCIMASS), m/z 481 [(M+H)⁺].

### Example 133

### N-butyl-N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-1-butanamine ditrifluoroacetate

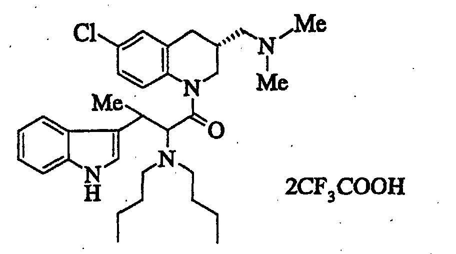

The title compound (7.1 mg, yield 20%) was obtained as a colorless amorphous powder in the similar manner as in Example 132.
MASS (APCIMASS), m/z 537 [(M+H)⁺].

### Example 134

### (2RS,3SR)-1-((3R)-6-chloro-3-[(dimethylamino)methyl]-3,4-dihydro-1(2H)-quinolinyl)-3-(1H-indol-3-yl)-1-oxo-N-(3-phenylpropyl)-2-butanamine ditrifluoroacetate

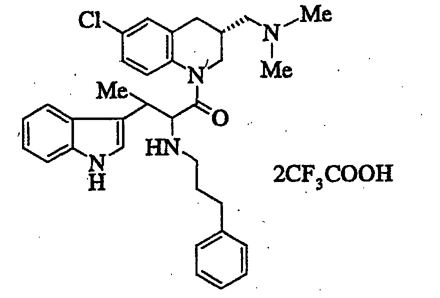

To a solution of (2RS,3SR)-1-((3R)-6-chloro-3-[(dimethylamino)methyl]-3,4-dihydro-1(2H)-quinolinyl)-3-(1H-indol-3-yl)-1-oxo-2-butanamine (20 mg) in dichloromethane (2 mL) was added 3-phenylpropanal (7.5 mg) at room temperature, and the mixture was stirred for 15 min. To the reaction mixture was added sodium triacetoxyborohydride (13 mg) at room temperature. The reaction solution was stirred at room temperature for 10 hrs. and then water was added thereto and vigorously shaken. After the obtained mixture was left standing, the organic layer was separated, the solvent was evaporated under reduced pressure. The residue was purified by HPLC (mobile phase; water (containing 0.03% trifluoroacetic acid)/acetonitrile (containing 0.03% trifluoroacetic acid) = 9:1 - 1:9) to give the title compound as a colorless amorphous powder (14.7 mg, yield 41%).
MASS (APCIMASS), m/z 543 [(M+H)⁺].

### Example 135

### (2RS,3SR)-1-((3R)-6-chloro-3-[(dimethylamino)methyl]-3,4-dihydro-1(2H)-quinolinyl)-3-(1H-indol-3-yl)-1-oxo-N,N-bis(3-phenylpropyl)-2-butanamine ditrifluoroacetate

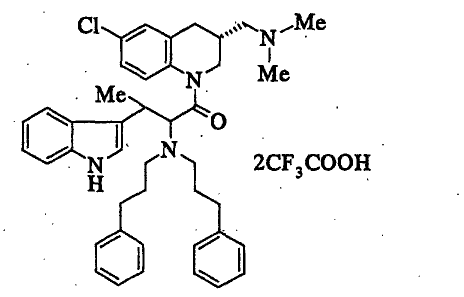

The title compound (9.3 mg, yield 22%) was obtained as a colorless amorphous powder in the similar manner as in Example 134.
MASS (APCIMASS), m/z 661 [(M+H)⁺].

### Example 136

### (-)-N-[1-[((3S)-6-chloro-3-[(dimethylamino)methyl]-3,4-dihydro-1(2H)-quinolinyl)carbonyl]-2-(1H-indol-3-yl)propyl]-1-[(1-methyl-1H-indol-2-yl)carbonyl]-4-piperidinecarboxamide

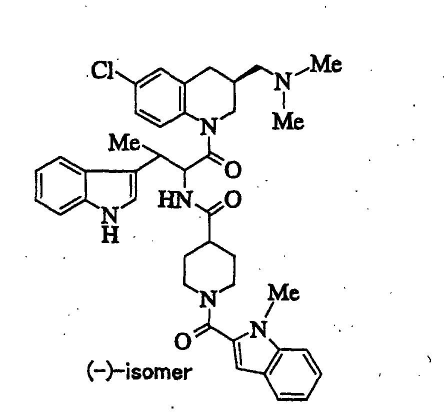

N-[(1RS,2SR)-1-[((3S)-6-chloro-3-[(dimethylamino)methyl]-3,4-dihydro-1(2H)-quinolinyl)carbonyl]-2-(1H-indol-3-yl)propyl]-1-[(1-methyl-1H-indol-2-yl)carbonyl]-4-piperidinecarboxamide (250 mg) was fractionated by HPLC to give the title compound (120 mg).
Fractionation conditions
column: CHRALCEL OD 50 mmID X 500 mmL
mobile phase: hexane/ethanol = 50/50
flow rate: 60 mL/min
temperature: 26°C
detection: UV 254 nm
[α]_{D}²⁸ = -125.05° (c=0.155, methanol).

### Example 137

### (+)-N-[1-[((3S)-6-chloro-3-[(dimethylamino)methyl]-3,4-dihydro-1(2H)-quinolinyl)carbonyl]-2-(1H-indol-3-yl)propyl]-1-[(1-methyl-1H-indol-2-yl)carbonyl]-4-piperidinecarboxamide

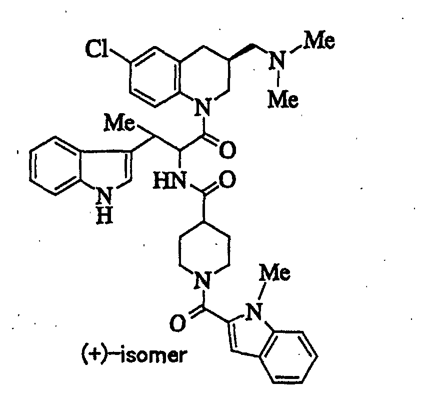

The title compound (120 mg) was obtained in the similar manner as in Example 136.
[α]_{D}²⁷ = +117.08° (c=0.210, methanol).

### Example 138

### (-)-N-[1-[((3S)-6-chloro-3-[(dimethylamino)methyl]-3,4-dihydro-1(2H)-quinolinyl)carbonyl]-2-(1H-indol-3-yl)propyl]-4-phenoxy-1-piperidinecarboxamide

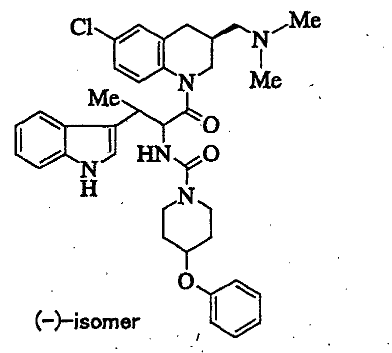

N-[(1RS,2SR)-1-[((3S)-6-chloro-3-[(dimethylamino)methyl]-3,4-dihydro-1(2H)-quinolinyl)carbonyl]-2-(1H-indol-3-yl)propyl]-4-phenoxy-1-piperidinecarboxamide (200 mg) was fractionated by HPLC to give the title compound (96 mg).
Fractionation conditions
column: CHRALCEL OD 50 mmID X 500 mmL
mobile phase: hexane/2-propanol/diethylamine = 85/15/0.1
flow rate: 80 mL/min
temperature: 26°C
detection: UV 254 nm
[α]_{D}²⁸ = -168.79° (c=0.165, methanol).

### Example 139

### (+)-N-[1-[((3S)-6-chloro-3-[(dimethylamino)methyl]-3,4-dihydro-1(2H)-quinolinyl)carbonyl]-2-(1H-indol-3-yl)propyl]-4-phenoxy-1-piperidinecarboxamide

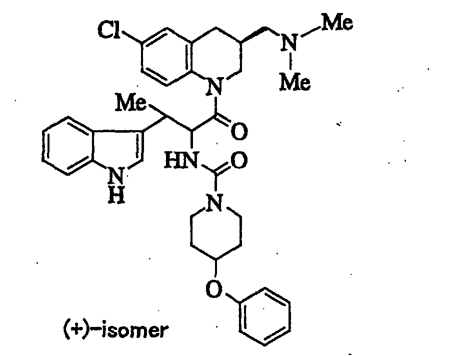

The title compound (94 mg) was obtained in the similar manner as in Example 138.
[α]_{D}²⁷=+169.10° (c=0.245, methanol).

### Example 140

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(4-hydroxyphenoxy)-1-piperidinecarboxamide

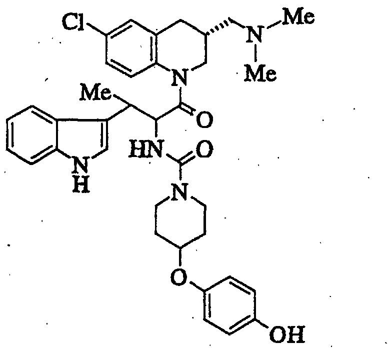

The title compound was obtained in the similar manner as in Example 41.
IR(KBr) 2938, 1622, 1508, 1227, 1040, 828, 743 cm⁻¹

### Example 141

### (-)-N-[1-[((3R)-6-chloro-3-[(dimethylamino)methyl]-3,4-dihydro-1(2H)-quinolinyl)carbonyl]-2-(1H-indol-3-yl)propyl]-4-(4-hydroxyphenoxy)-1-piperidinecarboxamide

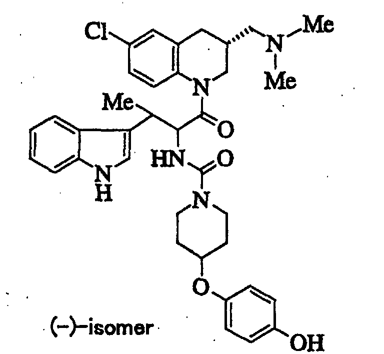

N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(4-hydroxyphenoxy)-1-piperidinecarboxamide (320 mg) was fractionated by HPLC to give the title compound (91 mg).
Fractionation conditions
column: CHRALCEL OD 50 mmID X 500 mmL
mobile phase: hexane/2-propanol = 85/15
flow rate: 80 mL/min
temperature: 30°C
detection: UV 254 nm
[α]_{D}²⁵ = -139.68° (c=0.100, methanol).

### Example 142

### (+)-N-[1-[((3R)-6-chloro-3-[(dimethylamino)methyl]-3,4-dihydro-1(2H)-quinolinyl)carbonyl]-2-(1H-indol-3-yl)propyl]-4-(4-hydroxyphenoxy)-1-piperidinecarboxamide

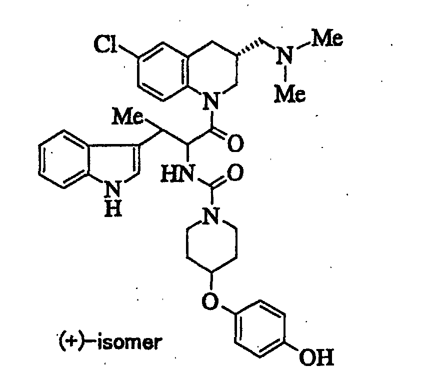

The title compound (109 mg) was obtained in the similar manner as in Example 141.
[α]_{D}²⁵=+158.88° (c=0.105, methanol).

The compounds described in the following Examples 143-152 were synthesized in the similar manner as in Example 41.

### Example 143

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(4-cyanophenoxy)-1-piperidinecarboxamide

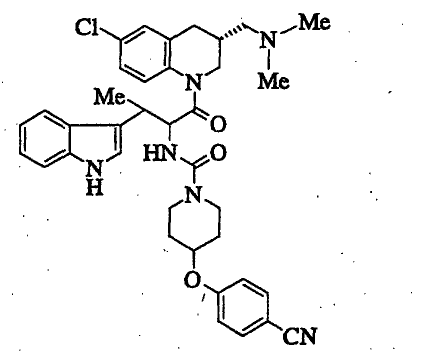

IR(KBr) 2948, 2224, 1634, 1507, 1254, 1036, 835, 741 cm⁻¹

### Example 144

### N-[1-[((3R)-6-chloro-3-[(dimethylamino)methyl]-3,4-dihydro-1(2H)-quinolinyl)carbonyl]-2-(1H-indol-3-yl)propyl]-4-(4-methoxyphenoxy)-1-piperidinecarboxamide

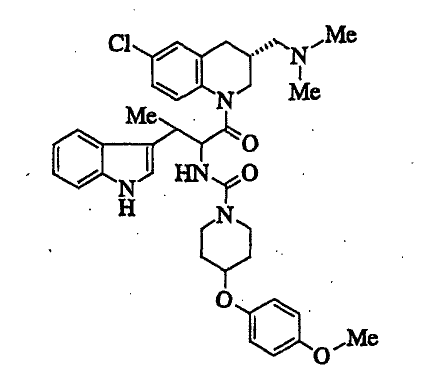

IR(KBr) 2944, 1634, 1507, 1227, 1038, 826, 743 cm⁻¹

### Example 145

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(3-hydroxyphenoxy)-1-piperidinecarboxamide

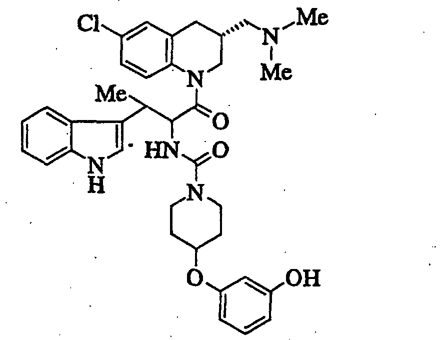

IR(KBr) 2936, 1626, 1487, 1231, 1146, 1038, 741 cm⁻¹

### Example 146

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-[4-(1H-tetrazol-5-yl)phenoxy]-1-piperidinecarboxamide

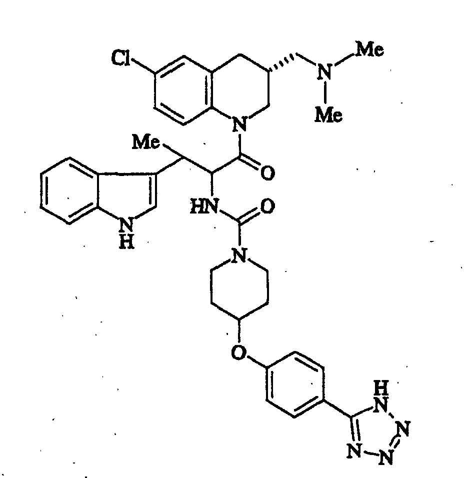

IR(KBr) 2969, 1645, 1487, 1231, 1038, 841, 743 cm⁻¹

### Example 147

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(4-chlorophenoxy)-1-piperidinecarboxamide

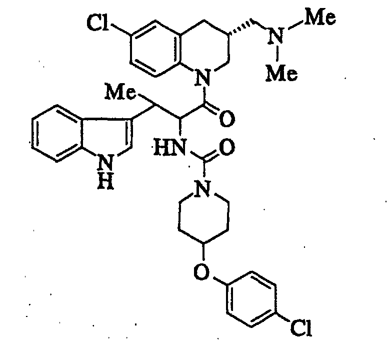

IR(KBr) 2944, 1634, 1489, 1235, 1038, 826, 741 cm⁻¹

### Example 148

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(4-methylphenoxy)-1-piperidinecarboxamide

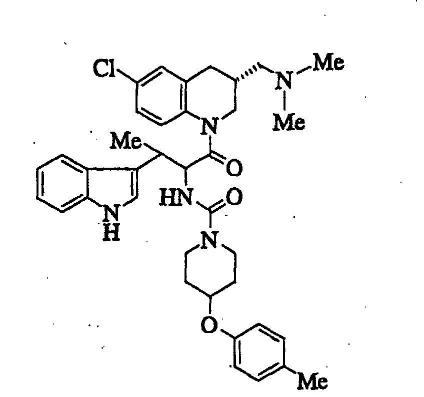

IR(KBr) 2934, 1628, 1508, 1231, 1040, 816, 741 cm⁻¹

### Example 149

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(4-fluorophenoxy)-1-piperidinecarboxamide

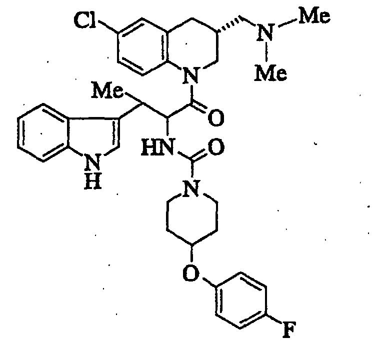

IR(KBr) 2934, 1628, 1505, 1206, 1042, 829, 743 cm⁻¹

### Example 150

### N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-4-(3-methylphenoxy)-1-piperidinecarboxamide

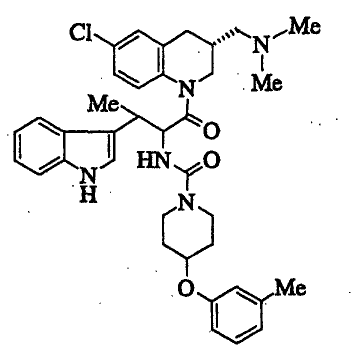

IR(KBr) 2932, 1628, 1489, 1258, 1040, 743 cm⁻¹

### Example 151

### (4-benzyloxy)-N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]- 1-piperidinecarboxamide

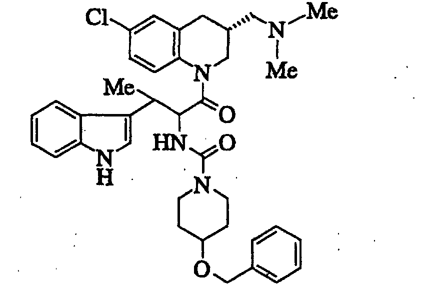

IR(KBr) 2944, 1628, 1489, 1229, 1098, 741, 700 cm⁻¹

### Example 152

### 4-[4-(aminosulfonyl)phenoxy]-N-[(1RS,2SR)-1-[((3R)-6-chloro-3-[(dimethylamino)methyl]-3,4-dihydro-1(2H)-quinolinyl)carbonyl]-2-(1H-indol-3-yl)propyl]-1-piperidinecarboxamide

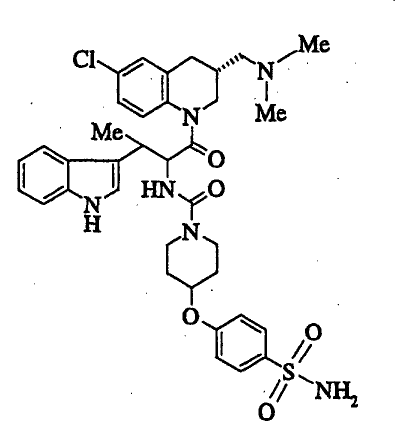

IR(KBr) 2938, 1624, 1489, 1250, 1157, 1098, 1034, 835, 743 cm⁻¹

The compounds described in the following Examples 153-170 were synthesized in the similar manner as in Example 41.

### Example 153

### N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-4-(4-fluorophenoxy)-1-piperidinecarboxamide

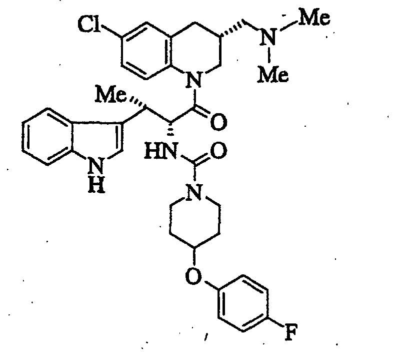

IR(KBr) 2942, 1628, 1505, 1209, 1038, 828, 764, 743 cm⁻¹

### Example 154

### N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-4-(3-methylphenoxy)-1-piperidinecarboxamide

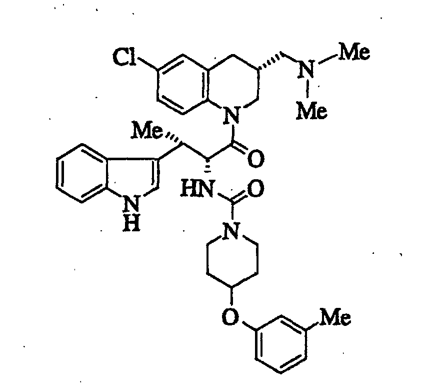

IR(KBr) 2936, 1630, 1489, 1258, 1229, 1038, 743 cm⁻¹

### Example 155

### N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-4-(phenylsulfonyl)-1-piperazinecarboxamide

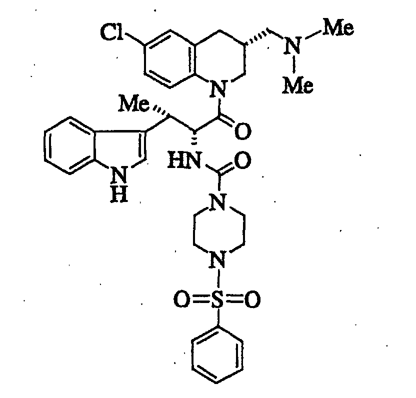

IR(KBr) 2934, 1632, 1489, 1169, 1094, 939, 741, 693 cm⁻¹

### Example 156

### N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-4-[(4-methylphenyl)sulfonyl]-1-piperazinecarboxamide

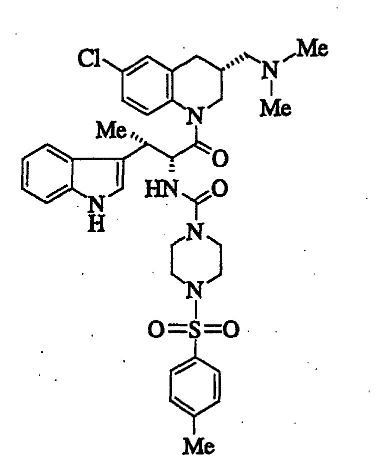

IR(KBr) 2932, 1630, 1489, 1165, 1094, 937, 727 cm⁻¹

### Example 157

### 4-benzoyl-N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-1-piperazinecarboxamide

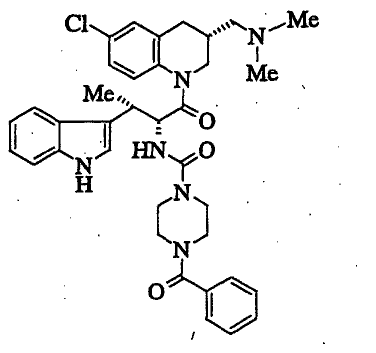

IR(KBr) 2934, 1628, 1260, 1007, 741, 710 cm⁻¹

### Example 158

### 4-benzoyl-N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-1-piperidinecarboxamide

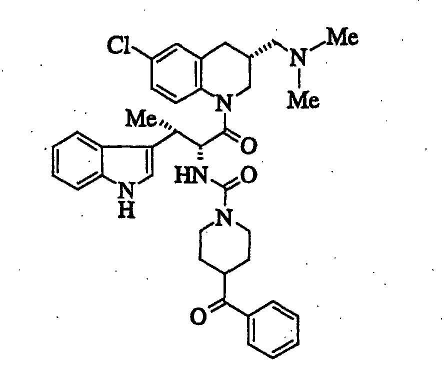

IR(KBr) 2943, 1630, 1489, 1209, 968, 743, 700 cm⁻¹

### Example 159

### 4-(benzyloxy)-N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-1-piperidinecarboxamide

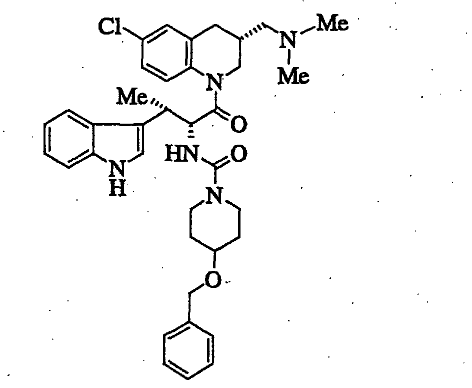

IR(KBr) 2942, 1628, 1489, 1229, 1100, 741, 700 cm⁻¹

### Example 160

### N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-4-(4-chlorophenoxy)-1-piperidinecarboxamide

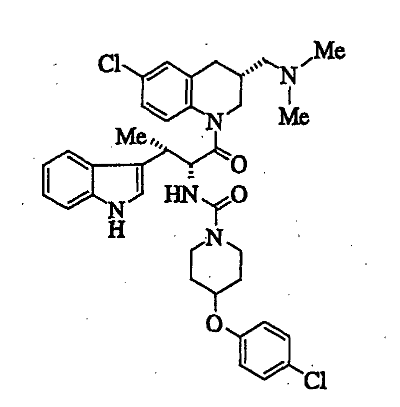

IR(KBr) 2934, 1630, 1489, 1233, 1094, 1038, 824, 743 cm⁻¹

### Example 161

### N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-4-(4-methylphenoxy)-1-piperidinecarboxamide

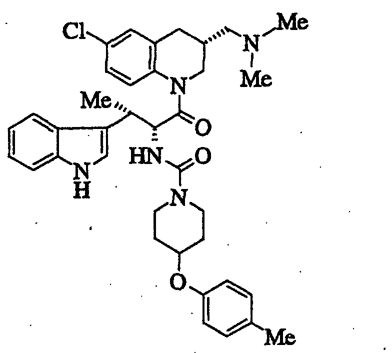

IR(KBr) 2942, 1630, 1508, 1229, 1040, 818, 743 cm⁻¹

### Example 162

### N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-4-[4-(methylsulfonyl)phenoxy]-1-piperidinecarboxamide

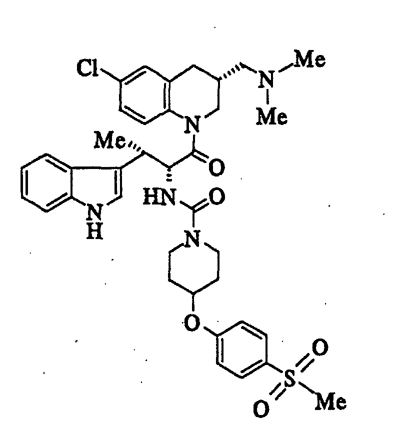

IR(KBr) 2934, 1632, 1489, 1254, 1146, 1092, 1034, 768, 743 cm⁻¹

### Example 163

### N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-4-[4-(methylthio)phenoxy]-1-piperidinecarboxamide

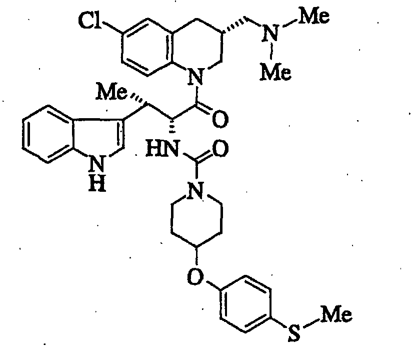

IR(KBr) 2932, 1630, 1489, 1231, 1038, 822, 743 cm⁻¹

### Example 164

### N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-4-(4-fluorobenzoyl)-1-piperazinecarboxamide

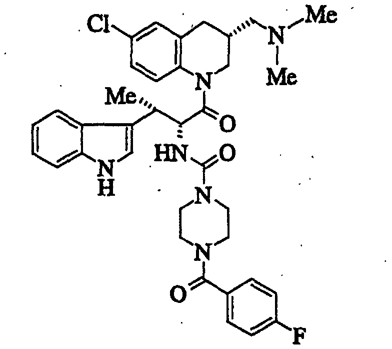

IR(KBr) 2934, 1630, 1489, 1229, 1009, 849, 743 cm⁻¹

### Example 165

### N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-4-(3-fluorobenzoyl)-1-piperazinecarboxamide

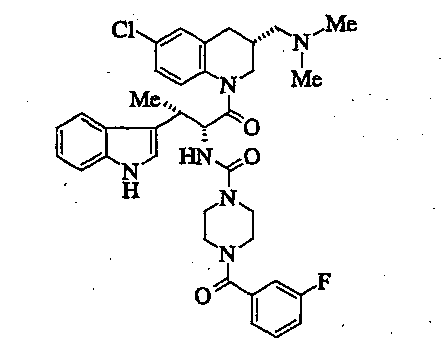

IR(KBr) 2934, 1632, 1489, 1262, 1013, 992, 745 cm⁻¹

### Example 166

### N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-4-(2-fluorobenzoyl)-1-piperazinecarboxamide

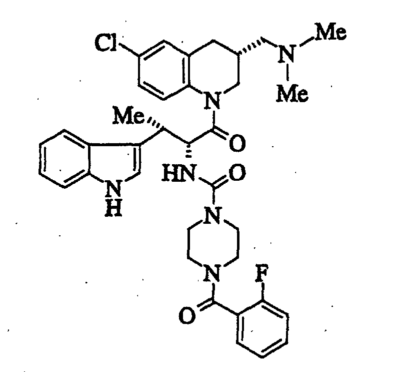

IR(KBr) 2934, 1634, 1254, 1227, 1009, 743 cm⁻¹

### Example 167

### 4-(4-chlorobenzoyl)-N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-1-piperazinecarboxamide

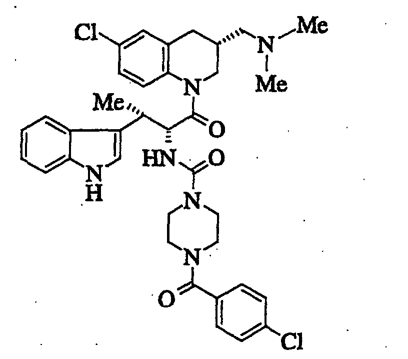

IR(KBr) 2934, 1632, 1489, 1258, 1092, 1007, 839, 743 cm⁻¹

### Example 168

### 4-(3-chlorobenzoyl)-N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-1-piperazinecarboxamide

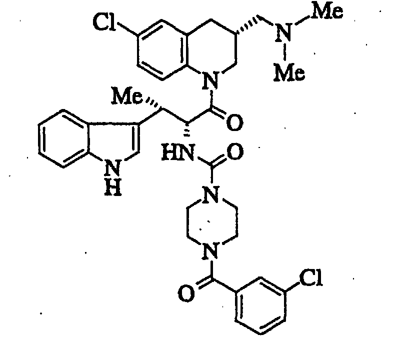

IR(KBr) 2932, 1634, 1489, 1256, 1013, 741 cm⁻¹

### Example 169

### 4-(2-chlorobenzoyl)-N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-1-piperazinecarboxamide

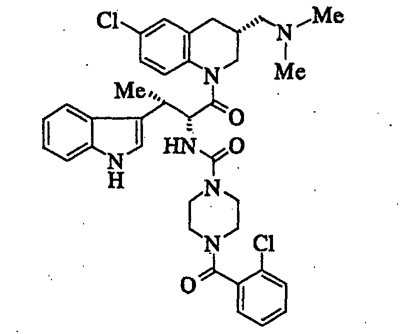

IR(KBr) 2934, 1632, 1487, 1251, 1229, 1007, 743 cm⁻¹

### Example 170

### N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-4-(trifluoroacetyl)-1,4-diazepane-1-carboxamide

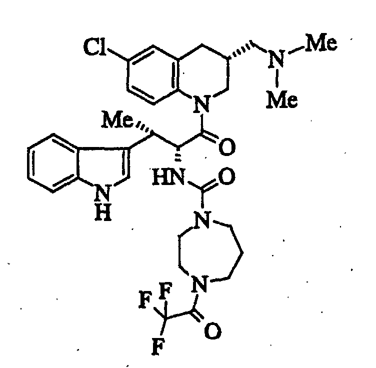

IR(KBr) 2942, 1628, 1489, 1209, 928, 743 cm⁻¹

### Example 171

### N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-1,4-diazepane-1-carboxamide

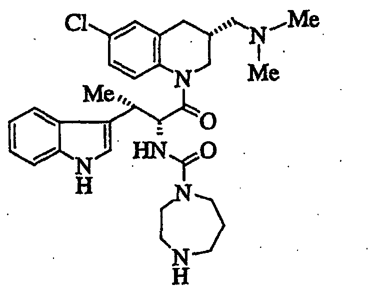

To a solution of N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-4-(trifluoroacetyl)-1,4-diazepane-1-carboxamide (220 mg) in methanol (6 ml) was added aqueous 10% potassium carbonate solution (3 ml), and the mixture was stirred at room temperature for 12 hrs. To the reaction solution was added water and the mixture was extracted with ethyl acetate. The ethyl acetate layer was washed with saturated brine, dried and concentrated under reduced pressure. The residue was purified by alumina column chromatography (developing solvent: ethyl acetate-ethyl acetate/ethanol = 10/1) to give the title compound (113 mg) as an amorphous powder.
IR(KBr) 2938, 1628, 1489, 1231, 743 cm⁻¹

### Example 172

### 4-benzoyl-N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-1,4-diazepane-1-carboxamide

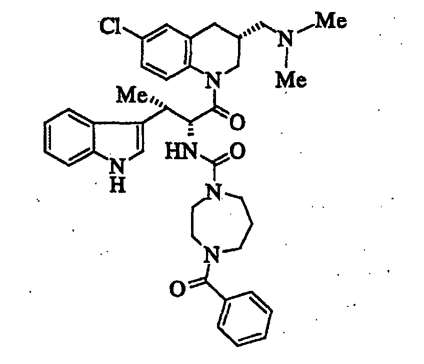

To a solution of N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-1,4-diazepane-1-carboxamide (79 mg) and triethylamine (17 mg) in tetrahydrofuran (1 ml) was added benzoyl chloride (22 mg) at 0°C. The reaction solution was stirred at 0°C for 1 hr., and then saturated aqueous sodium hydrogen carbonate was added thereto and extracted with ethyl acetate. The organic layer was washed with saturated brine, dried and concentrated. The residue was purified by alumina column chromatography (developing solvent: ethyl acetate-ethyl acetate/ethanol = 20/1) to give the title compound (88 mg) as an amorphous powder.
IR (KBr) 2936, 1634, 1489, 1229, 743 cm⁻¹

### Example 173

### N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-4-(phenylsulfonyl)-1,4-diazepane-1-carboxamide

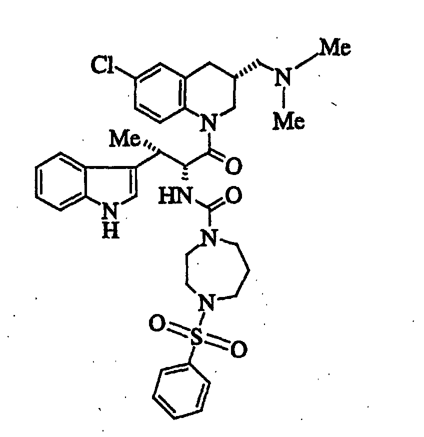

To a solution of N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-1,4-diazepane-1-carboxamide (20 mg) and triethylamine (4 mg) in tetrahydrofuran (0.5 ml) was added benzenesulfonyl chloride (8 mg) at 0°C. The reaction solution was stirred at 0°C for 1 hr., and then saturated aqueous sodium hydrogen carbonate was added thereto and extracted with ethyl acetate. The organic layer was washed with saturated brine, dried and concentrated. The residue was purified by alumina column chromatography (developing solvent: ethyl acetate) to give the title compound (11 mg) as an amorphous powder.
IR(KBr) 2942, 1632, 1489, 1161, 907, 731, 693 cm⁻¹

| **Formulation Example 1** | |
|---|---|
| (1) Compound obtained in Example 1 | 50.0 mg |
| (2) Lactose | 34.0 mg |
| (3) Cornstarch | 10.6 mg |
| (4) Cornstarch (paste) | 5.0 mg |
| (5) Magnesium stearate | 0.4 mg |
| (6) Carboxymethyl cellulose calcium | 20.0 mg |
| Total | 120.0 mg |

The above (1) to (6) were admixed in an ordinary manner, and tabletted using a tabletting machine, to obtain tablets.

### Experimental Example 1

The followings are some examples of the pharmacological actions of the compounds of the present invention, which should not be construed as being limiting to them. The gene manipulation using *E. coli* was conducted in accordance with the method described in the 1989 Edition of Molecular Cloning. (1) Cloning of human somatostatin receptor protein subtype 4 (hSSTR4) DNA

DNA oligomers S4-1 and S4-2 were synthesized based on the known human SSTR4 DNA sequence (Rohrer et al., Proc. Natl, Acad. Sci., USA 90, 4196-4200, 1993). The sequence of S4-1 is 5'-GGCTCGAGTCACCATGAGCGCCCCCTCG-3' (Sequence No. 1) and that of S4-2 is 5'-GGGCTCGAGCTCCTCAGAAGGTGGTGG-3' (Sequence No. 2).

Human chromosomal DNA (Clonetech Inc. Catalog No. CL6550-1) was used as the template. To 0.5 ng of said DNA was added 25 pmol of each of the above-mentioned DNA oligomers and the polymerase chain reaction was carried out using 2.5 units of PfuDNA polymerase (Strata gene). The composition of the reaction mixture was in accordance with the directions attached to said Pfu DNA polymerase.

The conditions of the reaction were as follows: One cycle consisting of the reactions at 94°C for 1 minute, at 66°C for 1 minute and at 75°C for 2 minutes, and 35 cycles were repeated. The reaction mixture was subjected to electrophoresis on 1% agarose gel to find that the DNA fragments of the intended size (about 1.2 kb) were specifically amplified. Said DNA fragments were recovered from the agarose gel in the usual manner and ligated to pUC118 cleaved at the Hinc II site to transform into the competent cells, *Escherichia coli* JM109. The transformant having plasmid containing said DNA fragments was selected out and the sequence of the inserted DNA fragments was confirmed by the automatic sequence analyzer employing fluorochroming, ALF DNA Sequencer (Pharmacia). As the results, the amino acid sequence expected from the nucleotide sequence was completely in agreement with the sequence described in the above-mentioned reports by Rohrer et al.

### (2) Construction of the expression plasmid of human somatostatin receptor protein subtype 4 (hSSTR4) DNA

pAKKO-111 was used as the expression vector in CHO (Chinese Hamster Ovary) cells. pAKKO-111 was constructed as follows: The 1.4 kb DNA fragment containing SR_{α} promoter and poly A appositional signal was obtained from pTB1417 described in JP-A-H5(1993)-076385 by the treatment with a restriction enzyme (Hind III) and a restriction enzyme (Cla I). On the other hand, the 4.5 kb DNA fragment containing dihydrofolic acid reductase gene (dhfr) was obtained from pTB348 [Naruo, K. et al., Biochem. Biophys. Res. Commun., 128, 256-264, 1985] by the treatment with a restriction enzyme (Cla I) and a restriction enzyme (Sal I). These DNA fragments were treated with T4 polymerase to make the terminal blunt-ended and ligated with T4 ligase to construct pAKKO-111 plasmid.

Then, 5 µg of the plasmid having human SSTR4 DNA fragment was digested with a restriction enzyme (XhoI) and subjected to electrophoresis on 1% agarose gel to recover the 1.2 kb DNA fragment encoding human SSTR4. Next, 1 µg of the above-mentioned expression vector pAKKO-111 (5.5 kb) was digested with Sal I to prepare the cloning site for insertion of human SSTR4 DNA fragment. Said expression vector fragment and the 1.2 kb DNA fragment were ligated with T4DNA ligase. The reaction mixture was introduced into *E*. *coli* JM109 by the calcium chloride method to obtain the expression plasmid pA1-11-hSSTR4 in which human SSTR4 DNA fragment was inserted in regular direction against the promoter from the transformants. This transformant is expressed as *Escherichia coli* JM109/pA-1-11-hSSTR4.

### (3) Transfection and expression of human somatostatin receptor protein subtype 4 (hSSTR4) DNA in CHO (dhfr⁻) cells

1 x 10⁶ CHO (dhfr⁻) cells were cultured for 24 hours in HAM F12 medium containing 10% bovine fetal serum on a laboratory dish of 8 cm in diameter. The cells were transfected by 10 µg of the human SSTR4 DNA expression plasmid, pA-1-11-hSSTR4 obtained above using the calcium phosphate method (Cell Phect Transfection Kit; Pharmacia). The medium was switched to Dulbecco's Modified Eagle Medium (DMEM) containing 10% dialyzed bovine fetal serum 24 hours after the transfection to select the colony-forming cells (i.e. dhfr⁺ cells) in this medium. Further, the selected cells were cloned from a single cell by the limiting dilution method and the somatostatin receptor protein expression activity of these cells was measured as follows: Human SSTR4 receptor expression cell strain was diluted with a buffer solution for assay [50 mM of Tris hydrochloride, 1 mM of EDTA, 5 mM of magnesium chloride, 0.1% of BSA, 0.2 mg/ml of bacitracin, 10 µg/ml of leupeptin, 1 µg/ml of pepstatin and 200 units/ml of aprotinin (pH 7.5)] to adjust the cell number to 2 x 10⁴/200 µl. 200 µl of the dilution was placed in a tube and to this was added 2 µl of 5 nM [¹²⁵I]-somatostatin-14 (2000 Ci/mmol, Amersham). The mixture was incubated at 25°C for 60 minutes. For measurement of non-specific binding (NSB), the tube to which 2 µl of somatostatin-14 (10⁻⁴ M) was added was also incubated. To the tube was added 1.5 ml of a buffer solution for washing [50 mM of Tris-hydrochloride, 1 mM of EDTA and 5 mM of magnesium chloride (pH 7.5)] and the mixture was filtered by GF/F glass fiber filter paper (Whatman) and washed further with 1.5 ml of the same buffer solution. The amount of [¹²⁵I] of the filter was measured by a γ-counter. Thus, a highly somatostatin-binding cell strain, hSSTR4-1-2, was selected.

### (4) Cloning of rat somatostatin receptor protein subtype 4 (rSSTR4) DNA

DNA oligomers S4-3 and S4-4 were synthesized based on the known rat SSTR4 DNA sequence (Bito. H et al., J. Biol. Chem., 269, 12722-12730, 1994).

The sequence of S4-3 is 5'-AAGCATGAACACGCCTGCAACTC-3' (Sequence No. 3) and that of S4-4 is 5'-GGTTTTCAGAAAGTAGTGGTCTT-3' (Sequence No. 4).

As the template, a chromosomal DNA prepared from Sprague-Dawley rats by using Easy-DNA™ KIT (Invitrogen) was used. To 0.5 ng of said DNA was added 25 pmol of each of the above-mentioned DNA oligomers and the polymerase chain reaction was carried out using TaKaRa LAPCR KIT (TaKaRa).

The conditions of the reaction were as follows: One cycle consisting of the reactions at 95°C for 30 seconds, at 65°C for 2 minutes and 30 seconds, and 30 cycles were repeated. The reaction mixture was subjected to electrophoresis on 1% agarose gel to find that the DNA fragments of the intended size (about 1.2 kb) were specifically amplified. Said DNA fragments were recovered from the agarose gel in the usual manner and ligated to a vector (pCR™ 2.1 (Trade name)) of ORIGINALTA CLONINGKIT (Invitrogen) to transform into the competent cells, *Escherichia coli* JM109. The transformant having plasmid containing said DNA fragments was selected out and the sequence of the inserted DNA fragments was confirmed by the automatic sequence analyzer employing fluorochroming, ALF DNA Sequencer (Pharmacia). As the results, the amino acid sequence expected from the nucleotide sequence was completely in agreement with the sequence described in the above-mentioned reports by Bito. H et al.

### (5) Construction of the expression plasmid of rat somatostatin receptor protein subtype 4 (rSSTR4) DNA

pAKKO-111 was used as the expression vector in CHO cells. 5 µg of the plasmid having rat SSTR4 DNA fragment obtained above was digested with a restriction enzyme (EcoRI), treated with T4 DNA polymerase, and subjected to electrophoresis on 1% agarose gel to recover the 1.2 kb DNA fragment encoding rat SSTR4. Next, 1 µg of the above-mentioned expression vector pAKKO-111 (5.5 kb) was digested with a restriction enzyme (ClaI), treated with T4 DNA polymerase and Alikaline Phosphatase, to prepare the cloning site for insertion of rat SSTR4 DNA fragment. Said expression vector fragment and the 1.2 kb DNA fragment were ligated with T4 DNA ligase. The reaction mixture was introduced into *E*. *coli* JM109 by the calcium chloride method to obtain the expression plasmid pA1-11-rSSTR4 in which rat SSTR4 DNA fragment was inserted in regular direction against the promoter from the transformants. This transformant is expressed as *Escherichia coli* JM109/pA-1-11-rSSTR4.

### (6) Transfection and expression of rat somatostatin receptor protein subtype 4 (rSSTR4) DNA in CHO (dhfr⁻) cells

1 x 10⁶ CHO (dhfr⁻) cells were cultured for 24 hours in α-MEM medium (containing ribonucleoside and deoxynucleoside) containing 10% bovine fetal serum on a laboratory dish of 8 cm in diameter. The cells were transfected by 10 µg of the rat SSTR4 DNA expression plasmid 1 pA-1-11-rSSTR4 obtained above using the calcium phosphate method (Cell Phect Transfection Kit; Pharmacia). The medium was switched to α-MEM medium (free of ribonucleoside and deoxynucleoside) containing 10% dialyzed bovine fetal serum 24 hours after the transfection to select the colony-forming cells (i.e. dhfr⁺ cells) in this medium. Further, the selected cells were cloned from a single cell by the limiting dilution method and the somatostatin receptor protein expression activity of these cells was measured by the binding method mentioned above. Thus, a highly somatostatin-binding cell strain, rSSTR4-20-25, was selected.

### (7) Preparation of CHO cell membrane fractions containing somatostatin receptor 4

Human and rat somatostatin receptor 4 expressing CHO cell strain, hSSTR4-1-2 or rSSTR4-20-25 (1 x 10⁹) was floated on a phosphate buffered saline supplemented with 5 mM EDTA (PBS-EDTA) and centrifuged. To the cell pellets was added 10 ml of a homogenate buffer for cells (10 mM NaHCO₃, 5 mM EDTA, pH 7.5), which was homogenated using a Politron homogenizer. The supernatant obtained by centrifugation at 400 x g for 15 minutes was further centrifuged at 100,000 x g for 1 hour to give a precipitate of the membrane fraction. The precipitates were suspended in 2 ml of a buffer solution for assay [25 mM of Tris-HCl, 1 mM of EDTA (Ethylenediaminetetraacetic Acid), 0.1% of BSA (Bovine Serum Albumin), 0.25 mM of PMSF (Phenylmethylsulfonyl Fluoride), 1 µg/ml pepstatin, 20 µg/ml leupeptin, 10 µg/ml Phosphoramidone, pH 7.5], which was centrifuged at 100,000 x g for 1 hour. The membrane fraction recovered as precipitates was suspended again in 20 ml of the buffer solution for assay, which was placed in tubes and stored at -80°C. The suspension was thawed and used at every use.

### Experimental Example 2

### (1) Cloning of human somatostatin receptor protein subtype 1 (SSTR1) DNA

DNA oligomers S1-1 and S1-2 were synthesized based on the known human SSTR1 cDNA sequence (Proc. Natl. Acad. Sci., USA vol. 89, p.251-255, 1992). The sequence of S1-1 is 5'-GGTCGACCTCAGCTAGGATGTTCCCCAATG-3' (Sequence No. 5) and that of S1-2 is 5'-GGTCGACCCGGGCTCAGAGCGTCGTGAT-3' (Sequence No. 6). Human chromosomal DNA (Clonetech Inc. Catalog No. CL 6550-1) was used as the template. To 0.5 ng of said DNA was added 25 pmol of each of the above-mentioned DNA oligomers and the polymerase chain reaction was carried out using 2.5 units of Pfu DNA polymerase (Stratagene). The composition of the reaction mixture was in accordance with the directions attached to said Pfu DNA polymerase. The conditions of the reaction were as follows: One cycle consisting of the reactions at 94°C for 1 minute, at 63°C for 1 minute and at 75°C for 2 minutes, and 35 cycles were repeated. The reaction mixture was subjected to electrophoresis on 1% agarose gel to find that the DNA fragments of the intended size (about 1.2 kb) were specifically amplified. Said DNA fragments were recovered from the agarose gel in the usual manner and ligated to pUC118 cleaved at the Hinc II site to transform into the competent cells, *Escherichia coli* JM109. The transformant having plasmid containing said DNA fragments was selected out and the sequence of the inserted DNA fragments was confirmed by the automatic sequence analyzer employing fluorochroming, ALF DNA Sequencer (Pharmacia). As the results, the amino acid sequence expected from the nucleotide sequence was completely in agreement with the sequence described in the above-mentioned literature.

### (2) Construction of the expression plasmid of human somatostatin receptor protein subtype 1 (SSTR1) DNA

pAKKO-111 was used as the expression vector in CHO (Chinese Hamster Ovary) cells. PAKKO-111 was constructed as follows: The 1.4 kb DNA fragment containing SRα promoter and poly A appositional signal was obtained from pTB1417 described in JP-A-H5 (1993)-076385 by treatment with Hind III and Cla I. On the other hand, the 4.5 kb DNA fragment containing dihydrofolic acid reductase (DHFR) gene was obtained from pTB348 [Biochem. Biophys. Res. Commun., 128, p.256-264, 1985] by treatment with Cla I and Sal I. These DNA fragments were treated with T4 polymerase to make the terminal blunt-ended and ligated with T4 ligase to construct pAKKO-111 plasmid. Then, 5 µg of the plasmid having human SSTR1 DNA fragment obtained under the above (1) was digested with the restriction enzyme Sal I and subjected to electrophoresis on 1% agarose gel to recover the 1.2 kb DNA fragment encoding human SSTR1. Next, 1 µg of the above-mentioned expression vector pAKKO-111 (5.5 kb) was digested with Sal I to prepare the cloning site for insertion of human SSTR1 DNA fragment. Said expression vector fragment and the 1.2 kb DNA fragment were ligated with T4 DNA ligase. The reaction mixture was introduced into *E*. *coli* JM109 by the calcium chloride method to obtain the expression plasmid pA1-11-SSTR1 in which human SSTR1 DNA fragment was inserted in regular direction against the promoter from the transformants. This transformant is expressed as *Escherichia coli* JM109/pA-1-11-SSTR1.

### (3) Transfection and expression of human somatostatin receptor protein subtype 1 (SSTR1) DNA in CHO (dhfr⁻) cells

1 x 10⁶ CHO (dhfr⁻) cells were cultured for 24 hours in HAM F12 medium containing 10% bovine fetal serum on a laboratory dish of 8 cm in diameter. The cells were transfected by 10 µg of the human SSTR1 cDNA expression plasmid 1 pA-1-11-SSTR1, obtained under the above (2) using the calcium phosphate method (Cell Phect Transfection Kit: Pharmacia). The medium was switched to DMEM medium containing 10% dialyzed bovine fetal serum 24 hours after the transfection to select the colony-forming cells (i.e. DHFR+ cells) in this medium. Further, the selected cells were cloned from a single cell by the limiting dilution method and the somatostatin protein activity was measured as follows: Human SSTR cDNA expression cell strain was diluted with a buffer solution for assay [50 mM of Tris hydrochloride, 1 mM of EDTA, 5 mM of magnesium chloride, 0.1% of BSA, 0.2 mg/ml of bacitracin, 10 µg/ml of leupeptin, 1 µg/ml of pepstatin and 200 units/ml of aprotinin (pH 7.5)] to adjust the cell number to 2 x 10⁴/200 µl. 200 µl of the dilution was placed in a tube and to this was added 2 µl of 5 nM [¹²⁵I]-somatostatin-14 (2000 Ci/mmol, Amersham). The mixture was incubated at 25°C for 60 minutes. For measurement of non-specific binding (NSB), the tube to which 2 µl of somatostatin-14 (10⁻⁴ M) was added was also incubated. To the tube was added 1.5 ml of a buffer solution for washing [50 mM of Tris hydrochloride, 1 mM of EDTA and 5 mM of magnesium chloride (pH 7.5)] and the mixture was filtered by GF/F glass fiber filter paper (Whatman) and washed further with 1.5 ml of the same buffer solution. The amount of [¹²⁵I] of the filter was measured by a γ-counter. Thus, a highly somatostatin-binding cell strain, SSTR1-8-3, was selected.

### (4) Cloning of human somatostatin receptor protein subtype 2 (SSTR2) DNA

DNA oligomers PT-1 and PT-2 were synthesized based on the known human SSTR2c DNA sequence (Proc. Natl. Acad. Sci., USA vol.89, p.251-255, 1992). The sequence of PT-1 is 5'-GGTCGACACCATGGACATGGCGGATGAG-3' (Sequence No. 7) and that of PT-2 is 5'-GGTCGACAGTTCAGATACTGGTTTGG-3' (Sequence No. 8). Human pituitary gland cDNA (Clonetech Inc. Catalog No. 7173-1) was used as the template. To 1 ng of said cDNA was added 25 pmol of each of the above-mentioned DNA oligomers and the polymerase chain reaction was carried out using 2.5 units of Taq DNA polymerase (TaKaRa Shuzo). The composition of the reaction mixture was in accordance with the directions attached to said Taq DNA polymerase. The conditions of the reaction were as follows: One cycle consisting of the reactions at 94°C for 30 seconds, at 52°C for 20 seconds and at 72°C for 60 seconds, and 30 cycles were repeated. The reaction mixture was subjected to electrophoresis on 1% agarose gel to find that the DNA fragments of the intended size (about 1.1 kb) was specifically amplified. Said DNA fragments were recovered from the agarose gel in the usual manner and ligated to pUC118 cleaved at the Hinc II site to transform into the competent cells, *Escherichia coli* JM109. Two strains (No. 5 and No. 7) of the transformant having plasmid containing said DNA fragments were selected out and the sequence of the inserted DNA fragments was confirmed by the automatic sequence analyzer employing fluorochroming, 373A DNA Sequencer (Applied Biosystem). As the results, point mutation was confirmed at one site between Sal I and Bst PI in the sequence of the 770 base fragment of No. 5 strain, and point mutation was also confirmed at one site between Bst PI and Sal I in the sequence of the 360 base fragment of No. 7 strain. Therefore, the fragments remaining after removing the Bst PI-Sal I fragment of No. 5 strain and the Bst PI-Sal I fragment of No. 7 strain were purified by electrophoresis on agarose to construct a plasmid in which these fragments were ligated by the ligation reaction. Confirmation of the insertion sequence of the DNA fragment of this plasmid revealed that it was completely in agreement with the sequence described in the above literature.

### (5) Construction of the expression plasmid of human somatostatin receptor protein subtype 2 (SSTR2) DNA

pAKKO-111 mentioned under the above (2) was used as the expression vector in CHO (Chinese Hamster Ovary), cells. 5 µg of the plasmid having human SSTR2 cDNA fragment obtained under the above (4) was digested with the restriction enzyme Sal I and subjected to electrophoresis on 1% agarose gel to recover the 1.1 kb DNA fragment encoding human SSTR2. Next, 1 µg of the above-mentioned expression vector pAKKO-111 (5.5 kb) was digested with Sal I to prepare the cloning site for insertion of human SSTR2 DNA fragment. Said expression vector fragment and the 1.1 kb DNA fragment were ligated with T4 DNA ligase. The reaction mixture was introduced into *E. coli* JM109 by the calcium chloride method to obtain the expression plasmid pAC01 in which human SSTR2 DNA fragment was inserted in regular direction against the promoter from the transformants. This transformant is expressed as *Escherichia coli* JM109/pAC-01.

### (6) Transfection and expression of human somatostatin receptor protein subtype 2 (SSTR2) DNA in CHO (dhfr⁻) cells

1 x 10⁶ CHO (dhfr⁻) cells were cultured for 24 hours in HAM F12 medium containing 10% bovine fetal serum on a laboratory dish of 8 cm in diameter. To the cells was transfected 10 µg of the human SSTR2 cDNA expression plasmid, pA-C01, obtained under the above (5) by the calcium phosphate method (Cell Phect Transfection Kit: Pharmacia). The medium was switched to DMEM medium containing 10% dialyzed bovine fetal serum 24 hours after the transfection to select the colony-forming cells (i.e. DHFR⁺ cells) in this medium. Further, the selected cells were cloned from a single cell by the limiting dilution method and a cell strain which highly expresses human SSTR2, SSTR2-HS5-9, was selected.

### (7) Cloning of human somatostatin receptor protein subtype 3 (SSTR3) DNA

DNA oligomers S3-1 and S3-2 were synthesized based on the known human SSTR3c DNA sequence (Mol. Endocrinol., vol.6, p.2136-2142, 1992). The sequence of S3-1 is 5'-GGTCGACCTCAACCATGGACATGCTTCATC-3' (Sequence No. 9) and that of S3-2 is 5'-GGTCGACTTTCCCCAGGCCCCTACAGGTA-3' (Sequence No. 10). Human chromosomal DNA (Clone Tech Inc. Catalog No. CL6550-1) was used as the template. To 0.5 ng of said DNA was added 25 pmol of each of the above-mentioned DNA oligomers and the polymerase chain reaction was carried out using 2.5 units of Pfu DNA polymerase (Strata gene). The composition of the reaction mixture was in accordance with the directions attached to said Pfu DNA polymerase. The conditions of the reaction were as follows: One cycle consisting of the reactions at 94°C for 1 minute, at 63°C for 1 minute and at 75°C for 2 minutes, and 35 cycles were repeated. The reaction mixture was subjected to electrophoresis on 1% agarose gel to find that the DNA fragments of the intended size (about 1.3 kb) was specifically amplified. As the results, the amino acid sequence expected from the nucleotide sequence was completely in agreement with the sequence described in the above-mentioned literature.

### (8) Construction of the expression plasmid of human somatostatin receptor protein subtype 3 (SSTR3) DNA

pAKKO-111 mentioned under the above (2) was used as the expression vector in CHO cells. 5 µg of the plasmid having human SSTR3 DNA fragment obtained under the above (7) was digested with the restriction enzyme Sal I and subjected to electrophoresis on 1% agarose gel to recover the 1.3 kb DNA fragment encoding human SSTR3. Next, 1 µg of the above-mentioned expression vector pAKKO-111 (5.5 kb) was digested with Sal I to prepare the cloning site for insertion of human SSTR3 DNA fragment. Said expression vector and the 1.3 kb DNA fragment were ligated with T4DNA ligase. The reaction mixture was introduced into *E. coli* JM109 by the calcium chloride method to obtain the expression plasmid pA1-11-SSTR3 in which human SSTR3 DNA fragment was inserted in regular direction against the promoter from the transformants. This transformant is expressed as *Escherichia coli* JM109/pA-1-11-SSTR3.

### (9) Transfection and expression of human somatostatin receptor protein subtype 3 (SSTR3) DNA in CHO (dhfr⁻) cells

1 x 10⁶ CHO (dhfr⁻) cells were cultured for 24 hours in HAM F12 medium containing 10% bovine fetal serum on a laboratory dish of 8 cm in diameter. The cells were transfected by 10 µg of the human SSTR3 DNA expression plasmid, pA-1-11-SSTR3, obtained under the above (5) using the calcium phosphate method. The medium was switched to DMEM medium containing 10% dialyzed bovine fetal serum 24 hours after the transfection to select the colony-forming cells (i.e. DHFR⁺ cells) in this medium. Further, the selected cells were cloned from a single cell by the limiting dilution method and the somatostatin receptor protein expression activity of these cells was measured by the binding assay mentioned under the above (3). Thus, a highly somatostatin-binding cell strain, SSTR3-15-19, was selected.

### (10) Cloning of human somatostatin receptor protein subtype (SSTR5) DNA

DNA oligomers S5-1 and S5-2 were synthesized based on the known human SSTR5c DNA sequence (Biochem Biophys. Res. Commun., vol.195, p.844-852, 1993). The sequence of S5-1 is 5'-GGTCGACCACCATGGAGCCCCTGTTCCC-3' (Sequence No. 11) and that of S5-2 is 5'-CCGTCGACACTCTCACAGCTTGCTGG-3' (Sequence No. 12). Human chromosomal DNA (Clonetech Inc. Catalog No. CL6550-1) was used as the template. To 0.5 ng of said DNA was added 25 pmol of each of the above-mentioned DNA oligomers and the polymerase chain reaction was carried out using 2.5 units of Pfu DNA polymerase (Stratagene). The composition of the reaction mixture was in accordance with the directions attached to PfuDNA polymerase. The conditions of the reaction were as follows: One cycle consisting of the reactions at 94°C for 1 minute, at 66°C for 1 minute and at 75°C for 2 minutes, and 35 cycles were repeated. The reaction mixture was subjected to electrophoresis on 1% agarose gel to find that the DNA fragments of the intended size (about 1.1 kb) were specifically amplified. Confirmation of the insertion sequence of said DNA fragment by the method mentioned under the above (1) revealed that the amino acid sequence expected from the nucleotide sequence was completely in agreement with the sequence described in the above-mentioned literature. (11) Construction of the expression plasmid of human somatostatin receptor protein subtype 5 (SSTR5) DNA.

pAKKO-111 mentioned under the above (2) was used as the expression vector in CHO cells. 5 µg of the plasmid having human SSTR5 DNA fragment obtained under the above (10) was digested with the restriction enzyme Sal I and subjected to electrophoresis on 1% agarose gel to recover the 1.1 kb DNA fragment encoding human SSTR5. Next, 1 µg of the above-mentioned expression vector pAKKO-111 (5.5 kb) was digested with Sal I to prepare the cloning site for insertion of human SSTR5 DNA fragment. Said expression vector fragment and the 1.1 kb DNA fragment were ligated with T4 DNA ligase. The reaction mixture was introduced into *E. coli* JM109 by the calcium chloride method to obtain the expression plasmid pA1-11-SSTR5 in which human SSTR5 DNA fragment was inserted in regular direction against the promoter from the transformants. This transformant is expressed as *Escherichia coli* JM109/pA-1-11-SSTR5.

### (12) Transfection and expression of human somatostatin receptor protein subtype 5 (SSTR5) DNA in CHO (dhfr⁻) cells

1 x 10⁶ CHO (dhfr⁻) cells were cultured for 24 hours in HAM F12 medium containing 10% bovine fetal serum on a laboratory dish of 8 cm in diameter. To the cells was transfected 10 µg of the human SSTR5 cDNA expression plasmid, pA-1-11-SSTR5, obtained under the above (11) by the calcium phosphate method. The medium was switched to DMEM medium containing 10% dialyzed bovine fetal serum 24 hours after the transfection to select the colony-forming cells (i.e. DHFR⁺ cells) in this medium. Further, the selected cells were cloned from a single cell by the limiting dilution method and the somatostatin receptor protein expression activity of these cells was measured by binding assay mentioned under the above (3). Thus, a highly somatostatin-biding cell strain, SSTR5-32-4, was selected.

### Experimental Example 3

### Measurement of the binding inhibition rate of ¹²⁵I-Somatostatin

The receptor binding inhibition rate (%) of the subject compound was calculated using each of the membrane fractions prepared in Experimental Examples 1 and 2.

The membrane fraction was diluted with a buffer solution for assay to adjust the concentration to 3 µg/ml. The diluate was placed in tubes each in quantity of 173 µl. To this were simultaneously added 2 µl of a solution of a subject compound in DMSO and 25 µl of a 200 pM radioisotope-labeled somatostatin-14 (¹²⁵I-somatostatin-14: Amersham). For measurement of the maximum binding, a reaction mixture added with 2 µl of DMSO and 25 µl of a 200 pM ¹²⁵I-somatostatin was prepared. For measurement of non-specific binding, a reaction mixture added with 2 µl of a 100 µM somatostatin solution in DMSO and 25 µl of a 200 pM ¹²⁵I-somatostatin solution was prepared at the same time. The mixtures were allowed to react at 25°C for 60 minutes. Then, the reaction mixture was filtered by aspiration using a Whatman glass filter (GF-B) treated with polyethylenimine. After filtration, the radioactivity of ¹²⁵I-somatostatin-14 remaining on the filter paper was measured by a γ-counter. The binding inhibition rate (%) of each subject compound was calculated by the following formula:
(TB-SB)/(TB-NSB) x 100
SB: radioactivity when a compound was added
TB: maximum binding radioactivity
NSB: non-specific binding radioactivity

The binding inhibition rates were measured by changing the concentrations of the subject compound, and the 50% inhibiting concentration of the subject compound (IC₅₀ value) was calculated by the Hill plots. The results are shown in [Table 1 ].

**[Table 1]**

| IC₅₀ (nM) | | | |
|---|---|---|---|
| Example No | SSTR2 | SSTR3 | SSTR5 |
| 6 | 0.2 | 30 | 60 |
| 42 | 0.1 | 6 | 40 |
| 141 | 0.05 | 3 | 10 |
| 153 | 0.1 | 10 | 40 |
| 157 | 0.05 | 1 | 5 |

This shows that the compound (I) of the present invention, salts thereof or prodrugs thereof have a binding inhibition effect on the human and rat somatostatin receptor.

### Experimental Example 4

### Glucagon secretion inhibitory activity test (rat)

A 0.5% methylcellulose suspension containing tne subject compound (3 mg/kg body weight) (compound administration group) or a 0.5% methylcellulose suspension (compound non-administration group) was orally administrated to SD rats (male, 7 weeks of age) after fasting overnight, and 120 minutes later, insulin (2 U/kg body weight, Novo Nordisk) was subcutaneously administreted. After 30 minutes from the insulin administration, the blood was drawn from the vein of eyegrounds of the rats using capillary, and blood plasma was separated by centrifugation. The concentration of glucagon in the obtained blood plasma was measured by radioimmunoassay using a Daiichi glucagon kit (Daiichi Isotope). As a non-treated group, the concentration of glucagon in the blood plasma of the rat of the compound non-administration group free of insulin administration was measured in the same manner as above.

The difference between the concentration of glucagon of the non-treated group and the concentration of glucagon of the compound non-administration group or the compound administration group was each calculated and the percentage of "the difference between the concentration of glucagon of the compound administration group and the concentration of glucagon of the non-treated group" relative to the "difference between the concentration of glucagon of the compound non-administration group and the concentration of glucagon of the non-treated group" as 100% was determined as "glucagon secretion (% of control)". The results are shown in Table 2.

**[Table 2]**

| Example No. | glucagon secretion (% of control) |
|---|---|
| 6 | 15.6 |
| 42 | 3.0 |

This shows that the compound (I) of the present invention has a glucagon secretion inhibitory activity in rats.

### Industrial Applicability

The compound of the present invention has an excellent somatostatin receptor binding inhibition action with low toxicity.

Therefore, the compound of the present invention is useful for disorders of an intracellular signal transduction system (e.g., diseases accompanied by excess sthenia or suppression, etc.); diseases accompanied by disorders of regulating cell proliferation; diseases accompanied by disorders of production and/or secretion of hormones, growth factors, or physiologically active substances, etc.; in a mammal.

### Free text in Sequence Listing

SEQ ID NO: 1 Oligonucleotide designed to act as primer for PCR
SEQ ID NO: 2 Oligonucleotide designed to act as primer for PCR
SEQ ID NO: 3 Oligonucleotide designed to act as primer for PCR
SEQ ID NO: 4 Oligonucleotide designed to act as primer for PCR
SEQ ID NO: 5 Oligonucleotide designed to act as primer for PCR
SEQ ID NO: 6 Oligonucleotide designed to act as primer for PCR
SEQ ID NO: 7 Oligonucleotide designed to act as primer for PCR
SEQ ID NO: 8 Oligonucleotide designed to act as primer for PCR
SEQ ID NO: 9 Oligonucleotide designed to act as primer for PCR
SEQ ID NO: 10 Oligonucleotide designed to act as primer for PCR
SEQ ID NO: 11 Oligonucleotide designed to act as primer for PCR
SEQ ID NO: 12 Oligonucleotide designed to act as primer for PCR

## Claims

1. A compound of the formula: 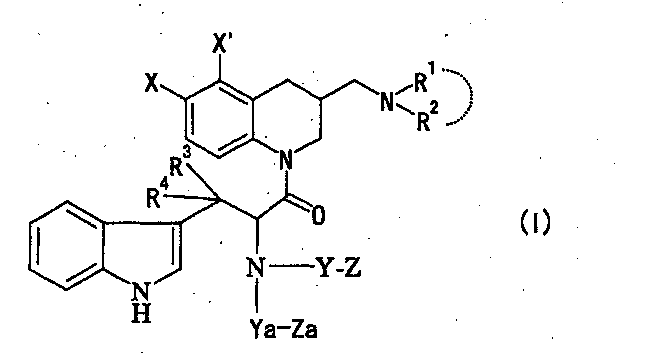 wherein
X and X' are the same or different and each represents a hydrogen atom, a halogen atom or an amino optionally having substituents;
R¹ and R² are the same or different and each represents a hydrogen atom or a C₁₋₆ alkyl optionally having substituents, or R¹ and R² form, together with the adjacent nitrogen atom, a nitrogen-containing heterocyclic ring optionally having substituents;
R³ represents a hydrocarbon group optionally having substituents or a heterocyclic group optionally having substituents;
R⁴ represents a hydrogen atom, a hydrocarbon group optionally having substituents or a heterocyclic group optionally having substituents, or
R³ and R⁴ may form, together with the adjacent carbon atom, a ring optionally having substituents;
Y and Ya are the same or different and each represents a bond or a spacer having a main chain of 1 to 8 atoms; and
Z and Za are the same or different and each represents a hydrogen atom, a halogen atom or a cyclic group optionally having substituents;
or a salt thereof or a prodrug thereof.

2. The compound according to claim 1, wherein X is a halogen atom and X' is a hydrogen atom.

3. The compound according to claim 1, wherein R¹ and R² are the same or different and each is a C₁₋₆ alkyl.

4. The compound according to claim 1, wherein R³ is a C₁₋₆ alkyl.

5. The compound according to claim 1, wherein R⁴ is a hydrogen atom.

6. The compound according to claim 1, wherein the spacers having a main chain of 1 to 8 atoms represented by Y and Ya are a divalent group comprising 1 to 5 groups selected from -O-, -S-, -CO-, -SO-, -SO₂-, -NR⁵- (R⁵ is a hydrogen atom,a C₁₋₆ alkyl optionally having substituents, a C₁₋₆ alkyl-carbonyl optionally having substituents or a C₁₋₆ alkylsulfonyl optionally having substituents) and a divalent C₁₋₆ non-cyclic hydrocarbon group optionally having substituents.

7. The compound according to claim 1, wherein Y is -CO-.

8. The compound according to claim 1, wherein Y is -CH₂-.

9. The compound according to claim 1, wherein Z is a cyclic group optionally having substituents.

10. The compound according to claim 9, wherein the cyclic group optionally having substituents is a 4- to 10-membered monocyclic non-aromatic heterocyclic group, which may have 1 to 3 substituents selected from an oxo, an optionally halogenated C₁₋₆ alkyl, a C₆₋₁₄ aryloxy optionally having substituents, a C₇₋₁₉ aralkyl optionally having substituents, a carbamoyl, an optionally halogenated C₁₋₆ alkyl-carbonyl, an optionally halogenated C₁₋₆ alkylsulfonyl, a C₆₋₁₄ aryl-carbonyl optionally having substituents, a C₆₋₁₄ aryl-sulfonyl optionally having substituents, a C₇₋₁₉ aralkyloxy-carbonyl optionally having substituents, a heterocyclic carbonyl optionally having substituents, a C₆₋₁₄ aryl optionally having substituents, an aromatic heterocyclic group optionally having substituents, a 5- to 7-membered non-aromatic heterocyclic group optionally having substituents and a C₇₋₁₉ aralkyloxy optionally having substituents.

11. The compound according to claim 1, wherein Ya is a bond and Za is a hydrogen atom.

12. The compound according to claim 1, which is
N-[(1R,2S)-1-[((3R)-6-chloro-3-[(dimethylamino)methyl]-3,4-dihydro-1(2H)-quinolinyl)carbonyl]-2-(1H-indol-3-yl)propyl]-1-[(1-methyl-1H-indol-2-yl)carbonyl]-4-piperidinecarboxamide,
N-[(1R,2S)-1-[((3R)-6-chloro-3-[(dimethylamino)methyl]-3,4-dihydro-1(2H)-quinolinyl)carbonyl]-2-(1H-indol-3-yl)propyl]-4-phenoxy-1-piperidinecarboxamide,
(-)-N-[1-[((3R)-6-chloro-3-[(dimethylamino)methyl]-3,4-dihydro-1(2H)-quinolinyl)carbonyl]-2-(1H-indol-3-yl)propyl]-4-(4-hydroxyphenoxy)-1-piperidinecarboxamide,
N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-4-(4-fluorophenoxy)-1-piperidinecarboxamide,
4-benzoyl-N-[(1R,2S)-1-[[(3R)-6-chloro-3-[(dimethylamino)methyl]-3,4-dihydro-1(2H)-quinolinyl]carbonyl]-2-(1H-indol-3-yl)propyl]-1-piperazinecarboxamide, or a salt thereof.

13. A pharmaceutical composition comprising the compound according to claim 1, a salt thereof or a prodrug thereof.

14. The composition according to claim 13, which is a somatostatin receptor binding inhibitor.

15. The composition according to claim 14, which is a somatostatin subtype 2 receptor binding inhibitor.

16. The composition according to claim 13, which is a somatostatin receptor agonist.

17. The composition according to claim 16, which is a somatostatin subtype 2 receptor agonist.

18. The composition according to claim 13, which is an agent for preventing or treating diabetes or diabetic complications.

19. Use of the compound according to claim 1, a salt thereof or a prodrug thereof for manufacturing a somatostatin receptor binding inhibitor.

20. A method for inhibiting binding of a somatostatin receptor in a mammal, which comprises administering an effective amount of the compound according to claim 1, a salt thereof or a prodrug thereof to the mammal.

21. Use of the compound according to claim 1, a salt thereof or a prodrug thereof for manufacturing an agent for preventing or treating diabetes or diabetic complications.

22. A method for preventing or treating diabetes or diabetic complications in a mammal, which comprises administering an effective amount of the compound according to claim 1, a salt thereof or a prodrug thereof to the mammal.


SEQUENCE LISTING
<110> Takeda Chemical Industries, Ltd.
<120> Amine Compounds
<130> 2971WOOP
<150> JP 2001-322897
<151> 2001-10-19
<160> 12
<210> 1
<211> 28
<212> DNA
<213> Artificial Sequence
<220>
<223> Oligonucleotide designed to act as primer for PCR.
<400> 1
ggctcgagtc accatgagcg ccccctcg     28
<210> 2
<211> 27
<212> DNA
<213> Artificial Sequence
<220>
<223> Oligonucleotide designed to act as primer for PCR.
<400> 2
gggctcgagc tcctcagaag gtggtgg     27
<210> 3
<211> 23
<212> DNA
<213> Artificial Sequence
<220>
<223> Oligonucleotide designed to act as primer for PCR.

<400> 3
aagcatgaac acgcctgcaa ctc 23
<210> 4
<211> 23
<212> DNA
<213> Artificial Sequence
<220>
<223> Oligonucleotide designed to act as primer for PCR.
<400> 4
ggttttcaga aagtagtggt ctt 23
<210> 5
<211> 30
<212> DNA
<213> Artificial Sequence
<220>
<223> Oligonucleotide designed to act as primer for PCR.
<400> 5
ggtcgacctc agctaggatg ttccccaatg 30
<210> 6
<211> 28
<212> DNA
<213> Artificial Sequence
<220>
<223> Oligonucleotide designed to act as primer for PCR.
<400> 6
ggtcgacccg ggctcagagc gtcgtgat 28
<210> 7
<211> 28
<212> DNA

<213> Artificial Sequence

<220>

<223> Oligonucleotide designed to act as primer for PCR.

<400> 7

ggtcgacacc atggacatgg cggatgag     28

<210> 8

<211> 26

<212> DNA

<213> Artificial Sequence

<220>

<223> Oligonucleotide designed to act as primer for PCR.

<400> 8

ggtcgacagt tcagatactg gtttgg     26

<210> 9

<211> 30

<212> DNA

<213> Artificial Sequence

<220>

<223> Oligonucleotide designed to act as primer for PCR.

<400> 9

ggtcgacctc aaccatggac atgcttcatc   30

<210> 10

<211> 29

<212> DNA

<213> Artificial Sequence

<220>

<223> Oligonucleotide designed to act as primer for PCR.

<400> 10

ggtcgacttt ccccaggccc ctacaggta    29

<210> 11

<211> 28

<212> DNA

<213> Artificial Sequence

<220>

<223> Oligonucleotide designed to act as primer for PCR.

<400> 11

ggtcgaccac catggagccc ctgttccc     28

<210> 12

<211> 26

<212> DNA

<213> Artificial Sequence

<220>

<223> Oligonucleotide designed to act as primer for PCR.

<400> 12

ccgtcgacac tctcacagct tgctgg     26